(19) Europäisches Patentamt
European Patent Office
Office européen des brevets

(11) EP 3 199 632 A1

(12) EUROPEAN PATENT APPLICATION

(43) Date of publication:
**02.08.2017 Bulletin 2017/31**

(51) Int Cl.:
***C12N 15/11*** *(2006.01)*

(21) Application number: **16152731.2**

(22) Date of filing: **26.01.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **ACIB GmbH**
  **8010 Graz (AT)**
- **Universität für Bodenkultur Wien**
  **1180 Wien (AT)**

(72) Inventors:
- **STEIGER, Matthias**
  **1120 Wien (AT)**
- **SAUER, Michael**
  **1040 Vienna (AT)**
- **MATTANOVICH, Diethard**
  **1180 Vienna (AT)**

(74) Representative: **Redl, Gerda et al**
**REDL Life Science Patent Attorneys**
**Donau-City-Straße 11**
**1220 Wien (AT)**

(54) **TEMPERATURE-INDUCIBLE CRISPR/CAS SYSTEM**

(57) A method of producing a mutant host cell with altered expression of a gene of interest (GOI) by a temperature-inducible CRISPR/Cas system, which comprises:
a) at a first working temperature, introducing into a host cell a CRISPR/Cas system employing
i) an inactive pre-guide RNA (pre-gRNA) which is a ribozyme-extended guide RNA comprising a guide RNA (gRNA) that recognizes a predetermined target site at the GOI, terminally extended by at least one self-processing Hammerhead (HH) ribozyme characterized by the presence of a thermosensitive hairpin and a cleavage site adjacent to the gRNA; and
ii) a RNA-guided Cas endonuclease which recognizes the target site upon hybridizing with the gRNA;

b) increasing the first working temperature to a second working temperature, thereby changing the structure of the thermosensitive hairpin and activating the pre-gRNA by self-catalyzed cleavage at the cleavage site to obtain the gRNA, which induces binding of the endonuclease to the target site, thereby altering expression of the GOI.

EP 3 199 632 A1

SEQUENCE LISTING

<110> ACIB GmbH
Universtitä¤t fÃ¼r Bodenkultur Wien

<120> TEMPERATURE-INDUCIBLE CRISPR/CAS SYSTEM

<130> AC012EP

<160> 25

<170> BiSSAP 1.3

<210> 1
<211> 37
<212> RNA
<213> Artificial Sequence

<220>
<223> HH ribozyme constant region

<400> 1
cugaugaguc cgugaggacg aaacgaguaa gcucguc 37

<210> 2
<211> 38
<212> RNA
<213> Artificial Sequence

<220>
<223> HH ribozyme constant region

<400> 2
cugaugaggc cgaaaggccg aaaaccguua agacgguc 38

<210> 3
<211> 59
<212> RNA
<213> Artificial Sequence

<220>
<223> HH ribozyme constant region

<400> 3
cugaugaggc cgaaaaccgu ugaacuuuug aauagugauu caggagguua aaauugcuc 59

<210> 4
<211> 67
<212> RNA
<213> Artificial Sequence

<220>
<223> HH ribozyme constant region

<400> 4
cugaugaguc cgugaggacg aaaaccguug aacuuuugaa uagugauuca ggagguuaaa 60
auugcuc 67

<210> 5
<211> 37
<212> RNA
<213> Artificial Sequence

<220>
<223> HH ribozyme constant region

<220>
<221> misc_feature
<222> 8
<223> /note="R=either A or G and complementary to Y at position 19"

<220>
<221> misc_feature
<222> 9
<223> /note="N=any nucleotide A, G, U or C and complementary to N at position 18"

<220>
<221> misc_feature
<222> 10
<223> /note="N=any nucleotide A, G, U or C and complementary to N at position 17"

<220>
<221> misc_feature
<222> 11
<223> /note="N=any nucleotide A, G, U or C and complementary to N at position 16"

<220>
<221> misc_feature
<222> 12
<223> /note="N=any nucleotide A, G, U or C"

<220>
<221> misc_feature
<222> 13
<223> /note="N=any nucleotide A, G, U or C"

<220>
<221> misc_feature
<222> 14
<223> /note="N=any nucleotide A, G, U or C"

<220>
<221> misc_feature
<222> 15
<223> /note="N=any nucleotide A, G, U or C, and can be optionally extended by up to 10 nucleotides"

<220>
<221> misc_feature
<222> 16
<223> /note="N=any nucleotide A, G, U or C and complementary to N at position 11"

<220>
<221> misc_feature
<222> 17
<223> /note="N=any nucleotide A, G, U or C and complementary to N at position 10"

```
<220>
<221> misc_feature
<222> 18
<223> /note="N=any nucleotide A, G, U or C and complementary to N at
      position 9"

<220>
<221> misc_feature
<222> 19
<223> /note="Y=either C or T and complementary to R at position 8"

<220>
<221> misc_feature
<222> 24
<223> /note="N=any nucleotide A, G, U or C and complementary to N at
      position 35"

<220>
<221> misc_feature
<222> 25
<223> /note="N=any nucleotide A, G, U or C and complementary to N at
      position 34"

<220>
<221> misc_feature
<222> 26
<223> /note="N=any nucleotide A, G, U or C and complementary to N at
      position 33"

<220>
<221> misc_feature
<222> 27
<223> /note="N=any nucleotide A, G, U or C and complementary to N at
      position 32"

<220>
<221> misc_feature
<222> 28
<223> /note="N=any nucleotide A, G, U or C"

<220>
<221> misc_feature
<222> 29
<223> /note="N=any nucleotide A, G, U or C"

<220>
<221> misc_feature
<222> 30
<223> /note="N=any nucleotide A, G, U or C"

<220>
<221> misc_feature
<222> 31
<223> /note="N=any nucleotide A, G, U or C, and can be optionally
      extended by up to 10 nucleotides"

<220>
<221> misc_feature
<222> 32
<223> /note="N=any nucleotide A, G, U or C and complementary to N at
      position 27"
```

<220>
<221> misc_feature
<222> 33
<223> /note="N=any nucleotide A, G, U or C and complementary to N at position 26"

<220>
<221> misc_feature
<222> 34
<223> /note="N=any nucleotide A, G, U or C and complementary to N at position 25"

<220>
<221> misc_feature
<222> 35
<223> /note="N=any nucleotide A, G, U or C and complementary to N at position 24"

<400> 5
cugaugarnn nnnnnnnyg aaannnnnnn nnnnnuc 37

<210> 6
<211> 143
<212> RNA
<213> Artificial Sequence

<220>
<223> pre-gRNA

<400> 6
gagccacuga ugaguccgug aggacgaaac gaguaagcuc gucuggcucg cuccauaagc 60

cagguuuuag agcuagaaau agcaaguuaa aauaaggcua guccguuauc aacuugaaaa 120

aguggcaccg agucggugcu uuu 143

<210> 7
<211> 143
<212> RNA
<213> Artificial Sequence

<220>
<223> pre-gRNA

<220>
<221> misc_feature
<222> 4
<223> /note="N=any nucleotide A, G, U or C and complementary to N at position 46"

<220>
<221> misc_feature
<222> 5
<223> /note="N=any nucleotide A, G, U or C and complementary to N at position 45"

<220>
<221> misc_feature
<222> 6
<223> /note="N=any nucleotide A, G, U or C and complementary to N at position 44"

<220>
<221> misc_feature
<222> 44
<223> /note="N=any nucleotide A, G, U or C and complementary to N at position 6"

<220>
<221> misc_feature
<222> 45
<223> /note="N=any nucleotide A, G, U or C and complementary to N at position 5"

<220>
<221> misc_feature
<222> 46
<223> /note="N=any nucleotide A, G, U or C and complementary to N at position 4"

<220>
<221> misc_feature
<222> 50
<223> /note="N=any nucleotide A, G, U or C"

<220>
<221> misc_feature
<222> 51
<223> /note="N=any nucleotide A, G, U or C"

<220>
<221> misc_feature
<222> 52
<223> /note="N=any nucleotide A, G, U or C"

<220>
<221> misc_feature
<222> 53
<223> /note="N=any nucleotide A, G, U or C"

<220>
<221> misc_feature
<222> 54
<223> /note="N=any nucleotide A, G, U or C"

<220>
<221> misc_feature
<222> 55
<223> /note="N=any nucleotide A, G, U or C"

<220>
<221> misc_feature
<222> 56
<223> /note="N=any nucleotide A, G, U or C"

<220>
<221> misc_feature
<222> 57
<223> /note="N=any nucleotide A, G, U or C"

<220>
<221> misc_feature
<222> 58
<223> /note="N=any nucleotide A, G, U or C"

<220>
<221> misc_feature
<222> 59
<223> /note="N=any nucleotide A, G, U or C"

<220>
<221> misc_feature
<222> 60
<223> /note="N=any nucleotide A, G, U or C"

<220>
<221> misc_feature
<222> 61
<223> /note="N=any nucleotide A, G, U or C"

<220>
<221> misc_feature
<222> 62
<223> /note="N=any nucleotide A, G, U or C"

<220>
<221> misc_feature
<222> 63
<223> /note="N=any nucleotide A, G, U or C"

<400> 7
gagnnncuga ugaguccgug aggacgaaac gaguaagcuc gucnnnctcn nnnnnnnnnn 60

nnnguuuuag agcuagaaau agcaaguuaa aauaaggcua guccguuauc aacuugaaaa 120

aguggcaccg agucggugcu uuu 143

<210> 8
<211> 143
<212> RNA
<213> Artificial Sequence

<220>
<223> pre-gRNA

<220>
<221> misc_feature
<222> 1
<223> /note="N=any nucleotide A, G, U or C and complementary to N at position 49"

<220>
<221> misc_feature
<222> 2
<223> /note="N=any nucleotide A, G, U or C and complementary to N at position 48"

<220>
<221> misc_feature
<222> 3
<223> /note="N=any nucleotide A, G, U or C and complementary to N at position 47"

<220>
<221> misc_feature
<222> 4

<223> /note="N=any nucleotide A, G, U or C and complementary to N at position 46"

<220>
<221> misc_feature
<222> 5
<223> /note="N=any nucleotide A, G, U or C and complementary to N at position 45"

<220>
<221> misc_feature
<222> 6
<223> /note="N=any nucleotide A, G, U or C and complementary to N at position 44"

<220>
<221> misc_feature
<222> 44
<223> /note="N=any nucleotide A, G, U or C and complementary to N at position 6"

<220>
<221> misc_feature
<222> 45
<223> /note="N=any nucleotide A, G, U or C and complementary to N at position 5"

<220>
<221> misc_feature
<222> 46
<223> /note="N=any nucleotide A, G, U or C and complementary to N at position 4"

<220>
<221> misc_feature
<222> 47
<223> /note="N=any nucleotide A, G, U or C and complementary to N at position 3"

<220>
<221> misc_feature
<222> 48
<223> /note="N=any nucleotide A, G, U or C and complementary to N at position 2"

<220>
<221> misc_feature
<222> 49
<223> /note="N=any nucleotide A, G, U or C and complementary to N at position 1"

<220>
<221> misc_feature
<222> 50
<223> /note="N=any nucleotide A, G, U or C"

<220>
<221> misc_feature
<222> 51
<223> /note="N=any nucleotide A, G, U or C"

<220>
<221> misc_feature

<222> 52
<223> /note="N=any nucleotide A, G, U or C"

<220>
<221> misc_feature
<222> 53
<223> /note="N=any nucleotide A, G, U or C"

<220>
<221> misc_feature
<222> 54
<223> /note="N=any nucleotide A, G, U or C"

<220>
<221> misc_feature
<222> 55
<223> /note="N=any nucleotide A, G, U or C"

<220>
<221> misc_feature
<222> 56
<223> /note="N=any nucleotide A, G, U or C"

<220>
<221> misc_feature
<222> 57
<223> /note="N=any nucleotide A, G, U or C"

<220>
<221> misc_feature
<222> 58
<223> /note="N=any nucleotide A, G, U or C"

<220>
<221> misc_feature
<222> 59
<223> /note="N=any nucleotide A, G, U or C"

<220>
<221> misc_feature
<222> 60
<223> /note="N=any nucleotide A, G, U or C"

<220>
<221> misc_feature
<222> 61
<223> /note="N=any nucleotide A, G, U or C"

<220>
<221> misc_feature
<222> 62
<223> /note="N=any nucleotide A, G, U or C"

<220>
<221> misc_feature
<222> 63
<223> /note="N=any nucleotide A, G, U or C"

<400> 8
nnnnnncuga ugaguccgug aggacgaaac gaguaagcuc gucnnnnnnn nnnnnnnnnn 60

nnnguuuuag agcuagaaau agcaaguuaa aauaaggcua guccguuauc aacuugaaaa 120

aguggcaccg agucggugcu uuu 143

<210> 9
<211> 173
<212> RNA
<213> Artificial Sequence

<220>
<223> pre-gRNA

<220>
<221> misc_feature
<222> 1
<223> /note="N=any nucleotide A, G, U or C and complementary to N at position 79"

<220>
<221> misc_feature
<222> 2
<223> /note="N=any nucleotide A, G, U or C and complementary to N at position 78"

<220>
<221> misc_feature
<222> 3
<223> /note="N=any nucleotide A, G, U or C and complementary to N at position 77"

<220>
<221> misc_feature
<222> 4
<223> /note="N=any nucleotide A, G, U or C and complementary to N at position 76"

<220>
<221> misc_feature
<222> 5
<223> /note="N=any nucleotide A, G, U or C and complementary to N at position 75"

<220>
<221> misc_feature
<222> 6
<223> /note="N=any nucleotide A, G, U or C and complementary to N at position 74"

<220>
<221> misc_feature
<222> 74
<223> /note="N=any nucleotide A, G, U or C and complementary to N at position 6"

<220>
<221> misc_feature
<222> 75
<223> /note="N=any nucleotide A, G, U or C and complementary to N at position 5"

<220>
<221> misc_feature
<222> 76
<223> /note="N=any nucleotide A, G, U or C and complementary to N at

position 4"

<220>
<221> misc_feature
<222> 77
<223> /note="N=any nucleotide A, G, U or C and complementary to N at position 3"

<220>
<221> misc_feature
<222> 78
<223> /note="N=any nucleotide A, G, U or C and complementary to N at position 2"

<220>
<221> misc_feature
<222> 79
<223> /note="N=any nucleotide A, G, U or C and complementary to N at position 1"

<220>
<221> misc_feature
<222> 80
<223> /note="N=any nucleotide A, G, U or C"

<220>
<221> misc_feature
<222> 81
<223> /note="N=any nucleotide A, G, U or C"

<220>
<221> misc_feature
<222> 82
<223> /note="N=any nucleotide A, G, U or C"

<220>
<221> misc_feature
<222> 83
<223> /note="N=any nucleotide A, G, U or C"

<220>
<221> misc_feature
<222> 84
<223> /note="N=any nucleotide A, G, U or C"

<220>
<221> misc_feature
<222> 85
<223> /note="N=any nucleotide A, G, U or C"

<220>
<221> misc_feature
<222> 86
<223> /note="N=any nucleotide A, G, U or C"

<220>
<221> misc_feature
<222> 87
<223> /note="N=any nucleotide A, G, U or C"

<220>
<221> misc_feature
<222> 88

<223> /note="N=any nucleotide A, G, U or C"

<220>
<221> misc_feature
<222> 89
<223> /note="N=any nucleotide A, G, U or C"

<220>
<221> misc_feature
<222> 90
<223> /note="N=any nucleotide A, G, U or C"

<220>
<221> misc_feature
<222> 91
<223> /note="N=any nucleotide A, G, U or C"

<220>
<221> misc_feature
<222> 92
<223> /note="N=any nucleotide A, G, U or C"

<220>
<221> misc_feature
<222> 93
<223> /note="N=any nucleotide A, G, U or C"

<400> 9
nnnnnncuga ugaguccgug aggacgaaaa ccguugaacu uuugaauagu gauucaggag 60

guuaaaauug cucnnnnnnn nnnnnnnnnn nnnguuuuag agcuagaaau agcaaguuaa 120

aauaaggcua guccguuauc aacuugaaaa aguggcaccg agucggugcu uuu 173

<210> 10
<211> 78
<212> RNA
<213> Artificial Sequence

<220>
<223> HDV ribozyme

<400> 10
ggccggcaug gucccagccu ccucgcuggc gccggcuggg caacaugcuu cggcauggcg 60

aaugggacag cuuuggac 78

<210> 11
<211> 23
<212> RNA
<213> Artificial Sequence

<220>
<223> target sequence

<400> 11
tggctcgctc cataagccag agg 23

<210> 12
<211> 37

<212> RNA
<213> Artificial Sequence

<220>
<223> HH ribozyme

<400> 12
ctgatgagtc cgtgaggacg aaacgagtaa gctcgtc 37

<210> 13
<211> 22
<212> RNA
<213> Artificial Sequence

<220>
<223> Primer

<400> 13
ccattctcat caaggattac gc 22

<210> 14
<211> 22
<212> RNA
<213> Artificial Sequence

<220>
<223> Primer

<400> 14
gaattgtgct gcacttttga ag 22

<210> 15
<211> 20
<212> RNA
<213> Artificial Sequence

<220>
<223> target sequence

<400> 15
tggctcgctc cataagccag 20

<210> 16
<211> 4140
<212> DNA
<213> Artificial Sequence

<220>
<223> Cas9 protein

<400> 16
atggacaaga agtactccat tgggctcgat atcggcacaa acagcgtcgg ttgggccgtc 60

attacggacg agtacaaggt gccgagcaaa aaattcaaag ttctgggcaa taccgatcgc 120

cacagcataa agaagaacct cattggcgcc ctcctgttcg actccgggga gacggccgaa 180

gccacgcggc tcaaaagaac agcacggcgc agatataccc gcagaaagaa tcggatctgc 240

tacctgcagg agatctttag taatgagatg gctaaggtgg atgactcttt cttccatagg 300

ctggaggagt cctttttggt ggaggaggat aaaaagcacg agcgccaccc aatctttggc 360

aatatcgtgg acgaggtggc gtaccatgaa aagtacccaa ccatatatca tctgaggaag 420

aagcttgtag acagtactga taaggctgac ttgcggttga tctatctcgc gctggcgcat 480

atgatcaaat ttcggggaca cttcctcatc gaggggggacc tgaacccaga caacagcgat 540

gtcgacaaac tctttatcca actggttcag acttacaatc agcttttcga agagaacccg 600

atcaacgcat ccggagttga cgccaaagca atcctgagcg ctaggctgtc caaatcccgg 660

cggctcgaaa acctcatcgc acagctccct ggggagaaga agaacggcct gtttggtaat 720

cttatcgccc tgtcactcgg gctgaccccc aactttaaat ctaacttcga cctggccgaa 780

gatgccaagc ttcaactgag caaagacacc tacgatgatg atctcgacaa tctgctggcc 840

cagatcggcg accagtacgc agaccttttt ttggcggcaa agaacctgtc agacgccatt 900

ctgctgagtg atattctgcg agtgaacacg gagatcacca aagctccgct gagcgctagt 960

atgatcaagc gctatgatga gcaccaccaa gacttgactt tgctgaaggc ccttgtcaga 1020

cagcaactgc ctgagaagta caaggaaatt ttcttcgatc agtctaaaaa tggctacgcc 1080

ggatacattg acggcggagc aagccaggag gaattttaca aatttattaa gcccatcttg 1140

gaaaaaatgg acggcaccga ggagctgctg gtaaagctta acagagaaga tctgttgcgc 1200

aaacagcgca ctttcgacaa tggaagcatc ccccaccaga ttcacctggg cgaactgcac 1260

gctatcctca ggcggcaaga ggatttctac ccctttttga aagataacag ggaaaagatt 1320

gagaaaatcc tcacatttcg gataccctac tatgtaggcc ccctcgcccg gggaaattcc 1380

agattcgcgt ggatgactcg caaatcagaa gagactatca ctccctggaa cttcgaggaa 1440

gtcgtggata agggggcctc tgcccagtcc ttcatcgaaa ggatgactaa ctttgataaa 1500

aatctgccta acgaaaaggt gcttcctaaa cactctctgc tgtacgagta cttcacagtt 1560

tataacgagc tcaccaaggt caaatacgtc acagaaggga tgagaaagcc agcattcctg 1620

tctggagagc agaagaaagc tatcgtggac ctcctcttca agacgaaccg gaaagttacc 1680

gtgaaacagc tcaaagagga ctatttcaaa aagattgaat gtttcgactc tgttgaaatc 1740

agcggagtgg aggatcgctt caacgcatcc ctgggaacgt atcacgatct cctgaaaatc 1800

attaaagaca aggacttcct ggacaatgag gagaacgagg acattcttga ggacattgtc 1860

ctcaccctta cgttgtttga agatagggag atgattgaag aacgcttgaa aacttacgct 1920

catctcttcg acgacaaagt catgaaacag ctcaagaggc gccgatatac aggatggggg 1980

cggctgtcaa gaaaactgat caatgggatc cgagacaagc agagtggaaa gacaatcctg 2040

gattttctta agtccgatgg atttgccaac cggaacttca tgcagttgat ccatgatgac 2100

tctctcacct ttaaggagga catccagaaa gcacaagttt ctggccaggg ggacagcctt 2160

cacgagcaca tcgctaatct tgcaggtagc ccagctatca aaaagggaat actgcagacc 2220
gttaaggtcg tggatgaact cgtcaaagta atgggaaggc ataagcccga gaatatcgtt 2280
atcgagatgg cccgagagaa ccaaactacc cagaagggac agaagaacag tagggaaagg 2340
atgaagagga ttgaagaggg tataaaagaa ctggggtccc aaatccttaa ggaacaccca 2400
gttgaaaaca cccagcttca gaatgagaag ctctacctgt actacctgca gaacggcagg 2460
gacatgtacg tggatcagga actggacatc aatcggctct ccgactacga cgtggatcat 2520
atcgtgcccc agtcttttct caaagatgat tctattgata ataaagtgtt gacaagatcc 2580
gataaaaata gagggaagag tgataacgtc ccctcagaag aagttgtcaa gaaaatgaaa 2640
aattattggc ggcagctgct gaacgccaaa ctgatcacac aacggaagtt cgataatctg 2700
actaaggctg aacgaggtgg cctgtctgag ttggataaag ccggcttcat caaaaggcag 2760
cttgttgaga cacgccagat caccaagcac gtggcccaaa ttctcgattc acgcatgaac 2820
accaagtacg atgaaaatga caaactgatt cgagaggtga aagttattac tctgaagtct 2880
aagctcgtct cagatttcag aaaggacttt cagttttata aggtgagaga gatcaacaat 2940
taccaccatg cgcatgatgc ctacctgaat gcagtggtag gcactgcact tatcaaaaaa 3000
tatcccaagc ttgaatctga atttgtttac ggagactata aagtgtacga tgttaggaaa 3060
atgatcgcaa agtctgagca ggaaataggc aaggccaccg ctaagtactt cttttacagc 3120
aatattatga atttttcaa gaccgagatt acactggcca atggagagat tcggaagcga 3180
ccacttatcg aaacaaacgg agaaacagga gaaatcgtgt gggacaaggg tagggatttc 3240
gcgacagtcc ggaaggtcct gtccatgccg caggtgaaca tcgttaaaaa gaccgaagta 3300
cagaccggag gcttctccaa ggaaagtatc ctcccgaaaa ggaacagcga caagctgatc 3360
gcacgcaaaa aagattggga ccccaagaaa tacggcggat tcgattctcc tacagtcgct 3420
tacagtgtac tggttgtggc caaagtggag aaagggaagt ctaaaaaact caaaagcgtc 3480
aaggaactgc tgggcatcac aatcatggag cgatcaagct tcgaaaaaaa ccccatcgac 3540
tttctcgagg cgaaaggata taaagaggtc aaaaaagacc tcatcattaa gcttcccaag 3600
tactctctct ttgagcttga aaacggccgg aaacgaatgc tcgctagtgc gggcgagctg 3660
cagaaaggta acgagctggc actgccctct aaatacgtta atttcttgta tctggccagc 3720
cactatgaaa agctcaaagg gtcccccgaa gataatgagc agaagcagct gttcgtggaa 3780
caacacaaac actaccttga tgagatcatc gagcaaataa gcgaattctc caaaagagtg 3840
atcctcgccg acgctaacct cgataaggtg ctttctgctt acaataagca cagggataag 3900
cccatcaggg agcaggcaga aaacattatc cacttgttta ctctgaccaa cttgggcgcg 3960
cctgcagcct tcaagtactt cgacaccacc atagacagaa agcggtacac ctctacaaag 4020

gaggtcctgg acgccacact gattcatcag tcaattacgg ggctctatga aacaagaatc 4080

gacctctctc agctcggtgg agacagcagg gctgacccca agaagaagag gaaggtgtga 4140

<210> 17
<211> 14261
<212> DNA
<213> Artificial Sequence

<220>
<223> Plasmid

<400> 17
cgctgtgagc aaaaggccag caaaaggcca ggaaccgtaa aaaggccgcg ttgctggcgt 60

ttttccatag gctccgcccc cctgacgagc atcacaaaaa tcgacgctca agtcagaggt 120

ggcgaaaccc gacaggacta taaagatacc aggcgtttcc ccctggaagc tccctcgtgc 180

gctctcctgt tccgaccctg ccgcttaccg gatacctgtc cgcctttctc ccttcgggaa 240

gcgtggcgct ttctcaatgc tcacgctgta ggtatctcag ttcggtgtag gtcgttcgct 300

ccaagctggg ctgtgtgcac gaaccccccg ttcagcccga ccgctgcgcc ttatccggta 360

actatcgtct tgagtccaac ccggtaagac acgacttatc gccactggca gcagccactg 420

gtaacaggat tagcagagcg aggtatgtag gcggtgctac agagttcttg aagtggtggc 480

ctaactacgg ctacactaga aggacagtat ttggtatctg cgctctgctg aagccagtta 540

ccttcggaaa aagagttggt agctcttgat ccggcaaaca aaccaccgct ggtagcggtg 600

gtttttttgt ttgcaagcag cagattacgc gcagaaaaaa aggatctcaa gaagatcctt 660

tgatcttttc tacggggtgg agcgcaaaaa accccgcccc tgacagggcg gggttttttc 720

gcgatcggag gtacagtggc catgaaatcc aatcatttcc ttctggccgc cctcgggcaa 780

gagatagtgc cgcagaggac tctcacagca tctacatctg cgaccgcaac agccaccaag 840

cgaggcgcac atgagcttgt cctcctccca tgccaaagtt tggccctctt cgtttctgtg 900

atgctgaagg aagtcaaact cgtcgatgat aggaccagat ggtttgtcaa gggtcaacgc 960

tttccatgcc ttctggcacc ggtagtaatg ctcttctgca agggagactt gacgtttcgg 1020

atccgcgggc cccgggacat gctggaaggg attttctggc tcaataccac gtctgtattt 1080

gacccttttcc agacagttaa tccgctgcag gagggcgaac tgtagctcct cgttctcctt 1140

gtagcgcttg atccagtctt tttggatgtt gcacttgctt ggcctatgct tctcatataa 1200

tcttgccctg tcatagagac gacgtctgag attgtagcgt tcgtctttga tcacccggag 1260

ccagataggc ctgagtatat ctgacattag atcaaagggt ctgtggatag tctccttcag 1320

catcagcgac gcatgtgact cgcatgtcgg agagagcttg tgggtggtca tctttgatgg 1380

cgtcctctgc tttcccttga ttttcgttga ttgtttttcg aaagttaagt ctggaagtca 1440

agagaatcct tctgccagac attatattta cgtatactga cgtagtagaa acagcgtcag 1500

gatgaggaca tggtgtgtgc tggaccacgg aatcatagtt catcagtata ttgggttgga 1560
caaataacgc tgagcatgta tatgtcttta cacactataa aagccagcga acgccaataa 1620
aatagggcat attgatgtga aaatatgaca ccagttaaaa gcagtgtatt gattttatct 1680
ctcttcacct cggacctata ctaccgtata caagactcaa cttacttcca gatatagtaa 1740
tatacaccct atggacgaac cagcacaata attacagcca aacaacacca cccaaatggc 1800
atattcctaa tcagcactaa gcacaaatac cactgtcatc acagcataat caataagaat 1860
cccagacaac cgactcactc tgactcacct tacacaaacc cccaagcaaa gcgcagccca 1920
gaacctcagc caacaatcgg gcaacgtacg gggaaagatt ggccgatcca tgatgtcagc 1980
agccctaacc caaagcggac tagcgcatac cgcccctctg actccgccat cccagggctc 2040
gagaagcttc cgtggcgtcg atataaattc agcgggcctt gaacatccct ccttacgaca 2100
cacctcacgc gatcgatttt gacactcaca caccgccacc ctcacatcct ccacccacac 2160
cacacccctt aatcaaccca ccatcaccgc tagaacgtct atctcatcac cgacttctca 2220
tccatcttca aacatggagc cactgatgag tccgtgagga cgaaacgagt aagctcgtct 2280
ggctcgctcc ataagccagg ttttagagct agaaatagca agttaaaata aggctagtcc 2340
gttatcaact tgaaaaagtg gcaccgagtc ggtgcttttg gccggcatgg tcccagcctc 2400
ctcgctggcg ccggctgggc aacatgcttc ggcatggcga atgggacagc tttggactgc 2460
ttgatccact taacgttact gaaatcatca aacagcttga cgaatctgga tataagatcg 2520
ttggtgtcga tgtcagctcc ggagttgaga caaatggtgt tcaggatctc gataagatac 2580
gttcatttgt ccaagcagca aagagtgcct tctagtgatt taatagctcc atgtcaacaa 2640
gaataaaacg cgtttcgggt ttacctcttc cagatacagc tcatctgcaa tgcattaatg 2700
cattggacct cgcaacccta gtacgccctt caggctccgg cgaagcagaa gaatagctta 2760
gcagagtcta ttttcatttt cgggagacga gatcaagcag atcaacggtc gtcaagagtc 2820
ctacgagact gaggaatccg ctcttggctc cacgcgacta tatatttgtc tctaattgta 2880
ctttgacatg ctcctcttct ttactctgat agcttgacta tgaaaattcc gtcaccagcc 2940
cctgggttcg caaagataat tgcactgttt cttccttgaa ctctcaagcc tacaggacac 3000
acattcatcg taggtataaa cctcgaaaat cattcctact aagatgggta tacaatagta 3060
accatgcatg gttgcctagt gaatgctccg taacacccaa tacgccggcc gaaacttttt 3120
tacaactctc ctatgagtcg tttacccaga atgcacaggt acacttgttt agaggtaatc 3180
cttctttcta gaagtcctcg tgtactgtgt aagcgcccac tccacatctc cactcgctcc 3240
ggggagaacc gaagaggatg ggaaccccga ccatggcctg cggccgcagc tatcagtcct 3300
ctcagctgac gcccctcacg aaccctcgca ctctcccgaa atgctcttcg gtgtccatgg 3360

cgcatcatct cagcagcatc ccatgtcaaa ccatggcttt gggcccacgg actctgtggc 3420
cctgccgcaa catcaccatc accatcgact cccgccccat gcagcgctgc gcgccccagg 3480
gcgtcatgga gtgaatgtgg aatctcctcc tttgccctcg ggcccgccga gtgtggtggg 3540
ccagcctgga atgcctgatc cagcccccag gcctcgagga ccaaaactga agtttactcc 3600
cgaagaggac gctctactgg tggagttgaa ggaaaacaag aacttgacgt ggaagcaaat 3660
tgcagacttc ttcccgggcc gaacgagcgg taccttgcaa gtccgatact gcaccaagct 3720
gaaggctaag gatgtagctt ggagtgacga aatggttcga tttgctcctg atgtatttct 3780
acgcctgtct cacacatgct aatgaaggga ataggtacaa aggctgcagc gggcaatgca 3840
cgagtacgag aacgatcggt ggcgcatcat tgcagggaag gttggaaatg gcttcacccc 3900
agctgcttgc cgcgagaaag ccatgcagct ccatgagtaa aagcgttggg gaattttcat 3960
atttatatct actgtcgcca gattcggccc tgcttggacc ctctgatctc cttactctcc 4020
atattggttc aaatgtcggg tcaccgatag ggctggtggt gcaggcttgt tgtaggcacg 4080
ggaggatgat cagcataact ctgagtcact atagggacgg gttgatgtaa ggtattaagt 4140
gatgtatgat aattcatttt agcccggggg aacatatggc gccggcattt gttcgttcgc 4200
aatgaaccga cactagcgtc cgctctcgca gtttagcacc ggctgatccc gggctgaacg 4260
cggccattgc tcggccgggg catgtgttcc ttatctacgg cagaccgcag atgaccactg 4320
gagcagatta tagaccctaa gccctaagcc ggacacccaa tcgagtaggt ctgcggacca 4380
ggtcactgcg ggcagccgga gaagctccgc aaccaatcaa tccccggcgc tgactaaggg 4440
caggcgacca cgggccgaag cggcttcaaa ctcacctcaa cctccaaact ccctcatctc 4500
caaacgtcct tgccttgtct gccgtcattg caacccaccc accaggacac atggacaaga 4560
agtactccat tgggctcgat atcggcacaa acagcgtcgg ttgggccgtc attacggacg 4620
agtacaaggt gccgagcaaa aaattcaaag ttctgggcaa taccgatcgc cacagcataa 4680
agaagaacct cattggcgcc ctcctgttcg actccgggga gacggccgaa gccacgcggc 4740
tcaaaagaac agcacggcgc agatataccc gcagaaagaa tcggatctgc tacctgcagg 4800
agatctttag taatgagatg gctaaggtgg atgactcttt cttccatagg ctggaggagt 4860
cctttttggt ggaggaggat aaaaagcacg agcgccaccc aatctttggc aatatcgtgg 4920
acgaggtggc gtaccatgaa aagtacccaa ccatatatca tctgaggaag aagcttgtag 4980
acagtactga taaggctgac ttgcggttga tctatctcgc gctggcgcat atgatcaaat 5040
ttcggggaca cttcctcatc gagggggacc tgaacccaga caacagcgat gtcgacaaac 5100
tctttatcca actggttcag acttacaatc agcttttcga agagaacccg atcaacgcat 5160
ccggagttga cgccaaagca atcctgagcg ctaggctgtc caaatcccgg cggctcgaaa 5220
acctcatcgc acagctccct ggggagaaga agaacggcct gtttggtaat cttatcgccc 5280

tgtcactcgg gctgaccccc aactttaaat ctaacttcga cctggccgaa gatgccaagc 5340

ttcaactgag caaagacacc tacgatgatg atctcgacaa tctgctggcc cagatcggcg 5400

accagtacgc agaccttttt ttggcggcaa agaacctgtc agacgccatt ctgctgagtg 5460

atattctgcg agtgaacacg gagatcacca aagctccgct gagcgctagt atgatcaagc 5520

gctatgatga gcaccaccaa gacttgactt tgctgaaggc ccttgtcaga cagcaactgc 5580

ctgagaagta caaggaaatt ttcttcgatc agtctaaaaa tggctacgcc ggatacattg 5640

acggcggagc aagccaggag gaattttaca aatttattaa gcccatcttg gaaaaaatgg 5700

acggcaccga ggagctgctg gtaaagctta acagagaaga tctgttgcgc aaacagcgca 5760

ctttcgacaa tggaagcatc ccccaccaga ttcacctggg cgaactgcac gctatcctca 5820

ggcggcaaga ggatttctac ccctttttga aagataacag ggaaaagatt gagaaaatcc 5880

tcacatttcg gataccctac tatgtaggcc ccctcgcccg gggaaattcc agattcgcgt 5940

ggatgactcg caaatcagaa gagactatca ctccctggaa cttcgaggaa gtcgtggata 6000

agggggcctc tgcccagtcc ttcatcgaaa ggatgactaa ctttgataaa aatctgccta 6060

acgaaaaggt gcttcctaaa cactctctgc tgtacgagta cttcacagtt tataacgagc 6120

tcaccaaggt caaatacgtc acagaaggga tgagaaagcc agcattcctg tctggagagc 6180

agaagaaagc tatcgtggac ctcctcttca agacgaaccg gaaagttacc gtgaaacagc 6240

tcaaagagga ctatttcaaa aagattgaat gtttcgactc tgttgaaatc agcggagtgg 6300

aggatcgctt caacgcatcc ctgggaacgt atcacgatct cctgaaaatc attaaagaca 6360

aggacttcct ggacaatgag gagaacgagg acattcttga ggacattgtc ctcaccctta 6420

cgttgtttga agatagggag atgattgaag aacgcttgaa aacttacgct catctcttcg 6480

acgacaaagt catgaaacag ctcaagaggc gccgatatac aggatggggg cggctgtcaa 6540

gaaaactgat caatgggatc cgagacaagc agagtggaaa gacaatcctg gattttctta 6600

agtccgatgg atttgccaac cggaacttca tgcagttgat ccatgatgac tctctcacct 6660

ttaaggagga catccagaaa gcacaagttt ctggccaggg ggacagcctt cacgagcaca 6720

tcgctaatct tgcaggtagc ccagctatca aaaagggaat actgcagacc gttaaggtcg 6780

tggatgaact cgtcaaagta atgggaaggc ataagcccga gaatatcgtt atcgagatgg 6840

cccgagagaa ccaaactacc cagaagggac agaagaacag tagggaaagg atgaagagga 6900

ttgaagaggg tataaaagaa ctggggtccc aaatccttaa ggaacaccca gttgaaaaca 6960

cccagcttca gaatgagaag ctctacctgt actacctgca gaacggcagg gacatgtacg 7020

tggatcagga actggacatc aatcggctct ccgactacga cgtggatcat atcgtgcccc 7080

agtcttttct caaagatgat tctattgata ataaagtgtt gacaagatcc gataaaaata 7140

gagggaagag tgataacgtc ccctcagaag aagttgtcaa gaaaatgaaa aattattggc 7200
ggcagctgct gaacgccaaa ctgatcacac aacggaagtt cgataatctg actaaggctg 7260
aacgaggtgg cctgtctgag ttggataaag ccggcttcat caaaaggcag cttgttgaga 7320
cacgccagat caccaagcac gtggcccaaa ttctcgattc acgcatgaac accaagtacg 7380
atgaaaatga caaactgatt cgagaggtga aagttattac tctgaagtct aagctcgtct 7440
cagatttcag aaaggacttt cagttttata aggtgagaga gatcaacaat taccaccatg 7500
cgcatgatgc ctacctgaat gcagtggtag gcactgcact tatcaaaaaa tatcccaagc 7560
ttgaatctga atttgtttac ggagactata aagtgtacga tgttaggaaa atgatcgcaa 7620
agtctgagca ggaaataggc aaggccaccg ctaagtactt cttttacagc aatattatga 7680
atttttcaa gaccgagatt acactggcca atggagagat tcggaagcga ccacttatcg 7740
aaacaaacgg agaaacagga gaaatcgtgt gggacaaggg tagggatttc gcgacagtcc 7800
ggaaggtcct gtccatgccg caggtgaaca tcgttaaaaa gaccgaagta cagaccggag 7860
gcttctccaa ggaaagtatc ctcccgaaaa ggaacagcga caagctgatc gcacgcaaaa 7920
aagattggga ccccaagaaa tacggcggat tcgattctcc tacagtcgct tacagtgtac 7980
tggttgtggc caaagtggag aaagggaagt ctaaaaaact caaaagcgtc aaggaactgc 8040
tgggcatcac aatcatggag cgatcaagct tcgaaaaaaa ccccatcgac tttctcgagg 8100
cgaaaggata taaagaggtc aaaaaagacc tcatcattaa gcttcccaag tactctctct 8160
ttgagcttga aaacggccgg aaacgaatgc tcgctagtgc gggcgagctg cagaaaggta 8220
acgagctggc actgccctct aaatacgtta atttcttgta tctggccagc cactatgaaa 8280
agctcaaagg gtcccccgaa gataatgagc agaagcagct gttcgtggaa caacacaaac 8340
actaccttga tgagatcatc gagcaaataa gcgaattctc caaaagagtg atcctcgccg 8400
acgctaacct cgataaggtg ctttctgctt acaataagca cagggataag cccatcaggg 8460
agcaggcaga aaacattatc cacttgttta ctctgaccaa cttgggcgcg cctgcagcct 8520
tcaagtactt cgacaccacc atagacagaa agcggtacac ctctacaaag gaggtcctgg 8580
acgccacact gattcatcag tcaattacgg ggctctatga aacaagaatc gacctctctc 8640
agctcggtgg agacagcagg gctgacccca agaagaagag gaaggtgtga gcttcgtccg 8700
acggcggccc acgggtccca ggcctcggag atccgtcccc cttttccttt gtcgatatca 8760
tgtaattagt tatgtcacgc ttacattcac gccctccccc cacatccgct ctaaccgaaa 8820
aggaaggagt tagacaacct gaagtctagg tccctattta ttttttata gttatgttag 8880
tattaagaac gttatttata tttcaaattt ttcttttttt tctgtacaga cgcgtgtacg 8940
catgtaacat tatactgaaa accttgcttg agaaggtttt gggacgctcg aaggctttaa 9000
tttgcaagct ggcgctaatt catgaataac ggtgagacta gcggccggtc cccttatccc 9060

agctgttcca cgttggcctg cccctcagtt agcgctcaac tcaatgcccc tcactggcga 9120
ggcgagggca aggatggagg ggcagcatcg cctgagttgg agcaaagcgg ccgccatggg 9180
agcagcgaac caacggaggg atgccgtgct ttgtcgtggc tgctgtggcc aatccgggcc 9240
cttggttggc tcacagagcg ttgctgtgag tccatgagct attattgcta ggtacagtat 9300
agagagagga gagagagaga gagagagaga ggggaaaaaa ggtgaggttg aagtgagaaa 9360
aaaaaaaaaa aaaatccaac cactgacggc tgccggctct gccacccccc tccctccacc 9420
ccagaccacc tgcacactca gcgcgcagca tcacctaatc ttggctcgcc ttcccgcagc 9480
tcaggttgtt tttttttct ctctccctcg tcgaagccgc ccttgttccc ttatttattt 9540
ccctctccat ccttgtctgc ctttggtcca tctgcccctt tgtctgcatc tcttttgcac 9600
gcatcgcctt atcgtcgtct ctttttcac tcacgggagc ttgacgatga cctgactcgt 9660
gagcctcacc tgctgatttc tctccccccc tcccgaccgg cttgactttt gtttctcctc 9720
cagtacctta tcgcgaagcc ggaagaacct cttaacctct agatgaaaaa gcctgaactc 9780
accgcgacgt ctgtcgagaa gttcctgatc gaaaagttcg acagcgtctc cgacctgatg 9840
cagctctcgg agggcgaaga atctcgtgct ttcagcttcg atgtaggagg gcgtggatat 9900
gtcctgcggg taaatagctg cgccgatggt ttctacaaag atcgttatgt ttatcggcac 9960
tttgcatcgg ccgcgctccc gattccggaa gtgcttgaca ttggggaatt cagcgagagc 10020
ctgacctatt gcatctcccg ccgtgcacag ggtgtcacgt tgcaagacct gcctgaaacc 10080
gaactgcccg ctgttctgca gccggtcgcg gaggccatgg atgcgatcgc tgcggccgat 10140
ctcagccaga cgagcgggtt cggcccattc ggaccgcaag gaatcggtca atacactaca 10200
tggcgtgatt tcatatgcgc gattgctgat ccccatgtgt atcactggca aactgtgatg 10260
gacgacaccg tcagtgcgtc cgtcgcgcag gctctcgatg agctgatgct ttgggccgag 10320
gactgccccg aagtccggca cctcgtgcac gcggatttcg gctccaacaa tgtcctgacg 10380
gacaatggcc gcataacagc ggtcattgac tggagcgagg cgatgttcgg ggattcccaa 10440
tacgaggtcg ccaacatctt cttctggagg ccgtggttgg cttgtatgga gcagcagacg 10500
cgctacttcg agcggaggca cccggagctt gcaggatcgc cgcggctccg ggcgtatatg 10560
ctccgcattg gtcttgacca actctatcag agcttggttg acggcaattt cgatgatgca 10620
gcttgggcgc agggtcgatg cgacgcaatc gtccgatccg gagccgggac tgtcgggcgt 10680
acacaaatcg cccgcagaag cgcggccgtc tggaccgatg gctgtgtaga agtactcgcc 10740
gatagtggaa accgacgccc cagcactcgt ccgagggcaa aggaatagat gcatggcttt 10800
cgtgaccggg cttcaaacaa tgatgtgcga tggtgtggtt cccggttggc ggagtctttg 10860
tctactttgg ttgtctgtcg caggtcggta gaccgcaaat gagcaactga tggattgttg 10920

ccagcgatac tataattcac atggatggtc tttgtcgatc agtagctagt gagagagaga 10980
gaacatctat ccacaatgtc gagtgtctat tagacatact ccgagaataa agtcaactgt 11040
gtctgtgatc taaagatcga ttcggcagtc gagtagcgta taacaactcc gagtaccagc 11100
gaaagcacgt cgtgacagga gcagggcttt gccaactgcg caaccttgct tgaatgagga 11160
tacacggggt gcaacatggc tgtactgatc catcgcaacc aaaatttctg tttatagatc 11220
aagctggtag attccaatta ctccacctct tgcgcttctc catgacatgt aagtgcacgt 11280
ggaaaccata cccaaattgc ctacagctgc ggagcatgag cctatggcga tcagtctggt 11340
catgttaacc agcctgtgct ctgacgttaa tgcagaatag cttatgctgc agacgcgtta 11400
cgtatcggat ccagaattcg tgatatctga attcgtcgac aagcttattt tttgtatact 11460
gttttgtgat agcacgaagt ttttccacgg tatcttgtta aaaatatata tttgtggcgg 11520
gcttacctac atcaaattaa taagagacta attataaact aaacacacaa gcaagctact 11580
ttagggtaaa agtttataaa tgcttttgac gtataaacgt tgcttgtatt tattattaca 11640
attaaaggtg gatagaaaac ctagagacta gttagaaact aatctcaggt ttgcgttaaa 11700
ctaaatcaga gcccgagagg ttaacagaac ctagaagggg actagatatc cgggtaggga 11760
aacaaaaaaa aaaaacaaga cagccacata ttagggagac tagttagaag ctagttccag 11820
gactaggaaa ataaaagaca atgataccac agtctagttg acaactagat agattctaga 11880
ttgaggccaa agtctctgag atccaggtta gttgcaacta atactagtta gtatctagtc 11940
tcctataact ctgaagctag aataacttac tactattatc ctcaccactg ttcagctgcg 12000
caaacggagt gattgcaagg tgttcagaga ctagttattg actagtcagt gactagcaat 12060
aactaacaag gtattaacct accatgtctg ccatcaccct gcacttcctc gggctcagca 12120
gccttttcct cctcattttc atgctcattt tccttgttta agactgtgac tagtcaaaga 12180
ctagtccaga accacaaagg agaaatgtct taccactttc ttcattgctt gtctcttttg 12240
cattatccat gtctgcaact agttagagtc tagttagtga ctagtccgac gaggacttgc 12300
ttgtctccgg attgttggag gaactctcca gggcctcaag atccacaaca gagccttcta 12360
gatgactggt caataactag ttggtctttg tctgagtctg actgacttac gaggttgcat 12420
actcgctccc tttgcctcgt caatcgatga gaaaaagcgc caaaactcgc aatatggctt 12480
tgaaccacac ggtgctgaga ctagttagaa tctagtccca aactagcttg gatagcttac 12540
ctttgccctt tgcgttgcga caggtcttgc agggtatggt tcctttctca ccagctgatt 12600
tagctgcctt gctaccctca cggcggatct gcataaagag tggctagagg ttataaatta 12660
gcactgatcc taggtacggg gctgaatgta acttgccttt cctttctcat cgcgcggcaa 12720
gacaggcttg ctcaaattcc taccagtcac aggggtatgc acggcgtacg gaccacttga 12780
actagtcaca gattagttag caactagtct gcattgaatg gctgtactta cgggccctcg 12840

ccattgtcct gatcatttcc agcttcaccc tcgttgctgc aaagtagtta gtgactagtc 12900

aaggactagt tgaaatggga gaagaaactc acgaattctc gacaccctta gtattgtggt 12960

ccttggactt ggtgctgcta tatattagct aatacactag ttagactcac agaaacttac 13020

gcagctcgct tgcgcttctt ggtaggagtc ggggttggga gaacagtgcc ttcaaacaag 13080

ccttcatacc atgctacttg actagtcagg gactagtcac caagtaatct agataggact 13140

tgcctttggc ctccatcagt tccttcatag tgggaggtcc attgtgcaat gtaaactcca 13200

tgccgtggga gttcttgtcc ttcaagtgct tgaccaatat gtttctgttg gcagagggaa 13260

cctgtcaact agttaataac tagtcagaaa ctagtatagc agtagactca ctgtacgctt 13320

gaggcatccc ttcactcggc agtagacttc atatggatgg atatcaggca cgccattgtc 13380

gtcctgtgga ctagtcagta actaggctta aagctagtcg ggtcggctta ctatcttgaa 13440

atccggcagc gtaagctccc cgtccttaac tgcctcgaga tagtgacagt actctgggga 13500

ctttcggaga tcgttatcgc gaatgctcgg catactaatc gttgactagt cttggactag 13560

tcccgagcaa aaaggattgg aggaggagga ggaaggtgag agtgagacaa agagcgaaat 13620

aagagcttca aaggctatct ctaagcagta tgaaggttaa gtatctagtt cttgactaga 13680

tttaaaagag atttcgacta gttatgtacc tggagtttgg atataggaat gtgttgtggt 13740

aacgaaatgt aagggggagg aaagaaaaag tcggtcaaga ggtaactcta agtcggccat 13800

tccttttgg gaggcgctaa ccataaacgg catggtcgac ttagagttag ctcagggaat 13860

ttagggagtt atctgcgacc accgaggaac ggcggaatgc caaagaatcc cgatggagct 13920

ctagctggcg gttgacaacc ccaccttttg gcgtttctgc ggcgttgcag gcgggactgg 13980

atacttcgta gaaccagaaa ggcaaggcag aacgcgctca gcaagagtgt tggaagtgat 14040

agcatgatgt gccttgttaa ctaggtcaaa atctgcagta tgcttgatgt tatccaaagt 14100

gtgagagagg aaggtccaaa catacacgat tgggagaggg cctaggtata agagtttttg 14160

agtagaacgc atgtgagccc agccatctcg aggagattaa acacgggccg gcatttgatg 14220

gctatgttag taccccaatg gaaagcctga gagtccagtg g 14261

<210> 18
<211> 145
<212> RNA
<213> Artificial Sequence

<220>
<223> pre-gRNA

<400> 18
gaaccauccu gaugaguccg ugaggacgaa acgaguaagc ucgucgaugg uuccguucua 60

cgggcguuuu agagcuagaa auagcaaguu aaaauaaggc uaguccguua ucaacuugaa 120

aaaguggcac cgagucggug cuuuu 145

<210> 19
<211> 1368
<212> PRT
<213> Streptococcus pyogenes serotype M1

<220>
<223> xxx

<400> 19
```
Met Asp Lys Lys Tyr Ser Ile Gly Leu Asp Ile Gly Thr Asn Ser Val
1               5                   10                  15
Gly Trp Ala Val Ile Thr Asp Glu Tyr Lys Val Pro Ser Lys Lys Phe
            20                  25                  30
Lys Val Leu Gly Asn Thr Asp Arg His Ser Ile Lys Lys Asn Leu Ile
        35                  40                  45
Gly Ala Leu Leu Phe Asp Ser Gly Glu Thr Ala Glu Ala Thr Arg Leu
    50                  55                  60
Lys Arg Thr Ala Arg Arg Arg Tyr Thr Arg Arg Lys Asn Arg Ile Cys
65                  70                  75                  80
Tyr Leu Gln Glu Ile Phe Ser Asn Glu Met Ala Lys Val Asp Asp Ser
                85                  90                  95
Phe Phe His Arg Leu Glu Glu Ser Phe Leu Val Glu Glu Asp Lys Lys
            100                 105                 110
His Glu Arg His Pro Ile Phe Gly Asn Ile Val Asp Glu Val Ala Tyr
        115                 120                 125
His Glu Lys Tyr Pro Thr Ile Tyr His Leu Arg Lys Lys Leu Val Asp
    130                 135                 140
Ser Thr Asp Lys Ala Asp Leu Arg Leu Ile Tyr Leu Ala Leu Ala His
145                 150                 155                 160
Met Ile Lys Phe Arg Gly His Phe Leu Ile Glu Gly Asp Leu Asn Pro
                165                 170                 175
Asp Asn Ser Asp Val Asp Lys Leu Phe Ile Gln Leu Val Gln Thr Tyr
            180                 185                 190
Asn Gln Leu Phe Glu Glu Asn Pro Ile Asn Ala Ser Gly Val Asp Ala
        195                 200                 205
Lys Ala Ile Leu Ser Ala Arg Leu Ser Lys Ser Arg Arg Leu Glu Asn
    210                 215                 220
Leu Ile Ala Gln Leu Pro Gly Glu Lys Lys Asn Gly Leu Phe Gly Asn
225                 230                 235                 240
Leu Ile Ala Leu Ser Leu Gly Leu Thr Pro Asn Phe Lys Ser Asn Phe
                245                 250                 255
Asp Leu Ala Glu Asp Ala Lys Leu Gln Leu Ser Lys Asp Thr Tyr Asp
            260                 265                 270
Asp Asp Leu Asp Asn Leu Leu Ala Gln Ile Gly Asp Gln Tyr Ala Asp
        275                 280                 285
Leu Phe Leu Ala Ala Lys Asn Leu Ser Asp Ala Ile Leu Leu Ser Asp
    290                 295                 300
Ile Leu Arg Val Asn Thr Glu Ile Thr Lys Ala Pro Leu Ser Ala Ser
305                 310                 315                 320
Met Ile Lys Arg Tyr Asp Glu His His Gln Asp Leu Thr Leu Leu Lys
                325                 330                 335
Ala Leu Val Arg Gln Gln Leu Pro Glu Lys Tyr Lys Glu Ile Phe Phe
            340                 345                 350
Asp Gln Ser Lys Asn Gly Tyr Ala Gly Tyr Ile Asp Gly Gly Ala Ser
        355                 360                 365
Gln Glu Glu Phe Tyr Lys Phe Ile Lys Pro Ile Leu Glu Lys Met Asp
    370                 375                 380
Gly Thr Glu Glu Leu Leu Val Lys Leu Asn Arg Glu Asp Leu Leu Arg
385                 390                 395                 400
Lys Gln Arg Thr Phe Asp Asn Gly Ser Ile Pro His Gln Ile His Leu
```

405 410 415
Gly Glu Leu His Ala Ile Leu Arg Arg Gln Glu Asp Phe Tyr Pro Phe
420 425 430
Leu Lys Asp Asn Arg Glu Lys Ile Glu Lys Ile Leu Thr Phe Arg Ile
435 440 445
Pro Tyr Tyr Val Gly Pro Leu Ala Arg Gly Asn Ser Arg Phe Ala Trp
450 455 460
Met Thr Arg Lys Ser Glu Glu Thr Ile Thr Pro Trp Asn Phe Glu Glu
465 470 475 480
Val Val Asp Lys Gly Ala Ser Ala Gln Ser Phe Ile Glu Arg Met Thr
485 490 495
Asn Phe Asp Lys Asn Leu Pro Asn Glu Lys Val Leu Pro Lys His Ser
500 505 510
Leu Leu Tyr Glu Tyr Phe Thr Val Tyr Asn Glu Leu Thr Lys Val Lys
515 520 525
Tyr Val Thr Glu Gly Met Arg Lys Pro Ala Phe Leu Ser Gly Glu Gln
530 535 540
Lys Lys Ala Ile Val Asp Leu Leu Phe Lys Thr Asn Arg Lys Val Thr
545 550 555 560
Val Lys Gln Leu Lys Glu Asp Tyr Phe Lys Lys Ile Glu Cys Phe Asp
565 570 575
Ser Val Glu Ile Ser Gly Val Glu Asp Arg Phe Asn Ala Ser Leu Gly
580 585 590
Thr Tyr His Asp Leu Leu Lys Ile Ile Lys Asp Lys Asp Phe Leu Asp
595 600 605
Asn Glu Glu Asn Glu Asp Ile Leu Glu Asp Ile Val Leu Thr Leu Thr
610 615 620
Leu Phe Glu Asp Arg Glu Met Ile Glu Glu Arg Leu Lys Thr Tyr Ala
625 630 635 640
His Leu Phe Asp Asp Lys Val Met Lys Gln Leu Lys Arg Arg Arg Tyr
645 650 655
Thr Gly Trp Gly Arg Leu Ser Arg Lys Leu Ile Asn Gly Ile Arg Asp
660 665 670
Lys Gln Ser Gly Lys Thr Ile Leu Asp Phe Leu Lys Ser Asp Gly Phe
675 680 685
Ala Asn Arg Asn Phe Met Gln Leu Ile His Asp Asp Ser Leu Thr Phe
690 695 700
Lys Glu Asp Ile Gln Lys Ala Gln Val Ser Gly Gln Gly Asp Ser Leu
705 710 715 720
His Glu His Ile Ala Asn Leu Ala Gly Ser Pro Ala Ile Lys Lys Gly
725 730 735
Ile Leu Gln Thr Val Lys Val Val Asp Glu Leu Val Lys Val Met Gly
740 745 750
Arg His Lys Pro Glu Asn Ile Val Ile Glu Met Ala Arg Glu Asn Gln
755 760 765
Thr Thr Gln Lys Gly Gln Lys Asn Ser Arg Glu Arg Met Lys Arg Ile
770 775 780
Glu Glu Gly Ile Lys Glu Leu Gly Ser Gln Ile Leu Lys Glu His Pro
785 790 795 800
Val Glu Asn Thr Gln Leu Gln Asn Glu Lys Leu Tyr Leu Tyr Tyr Leu
805 810 815
Gln Asn Gly Arg Asp Met Tyr Val Asp Gln Glu Leu Asp Ile Asn Arg
820 825 830
Leu Ser Asp Tyr Asp Val Asp His Ile Val Pro Gln Ser Phe Leu Lys
835 840 845
Asp Asp Ser Ile Asp Asn Lys Val Leu Thr Arg Ser Asp Lys Asn Arg
850 855 860
Gly Lys Ser Asp Asn Val Pro Ser Glu Glu Val Val Lys Lys Met Lys
865 870 875 880
Asn Tyr Trp Arg Gln Leu Leu Asn Ala Lys Leu Ile Thr Gln Arg Lys
885 890 895
Phe Asp Asn Leu Thr Lys Ala Glu Arg Gly Gly Leu Ser Glu Leu Asp
900 905 910

```
Lys Ala Gly Phe Ile Lys Arg Gln Leu Val Glu Thr Arg Gln Ile Thr
        915                 920                 925
Lys His Val Ala Gln Ile Leu Asp Ser Arg Met Asn Thr Lys Tyr Asp
    930                 935                 940
Glu Asn Asp Lys Leu Ile Arg Glu Val Lys Val Ile Thr Leu Lys Ser
945                 950                 955                 960
Lys Leu Val Ser Asp Phe Arg Lys Asp Phe Gln Phe Tyr Lys Val Arg
                965                 970                 975
Glu Ile Asn Asn Tyr His His Ala His Asp Ala Tyr Leu Asn Ala Val
            980                 985                 990
Val Gly Thr Ala Leu Ile Lys Lys Tyr Pro Lys Leu Glu Ser Glu Phe
        995                 1000                1005
Val Tyr Gly Asp Tyr Lys Val Tyr Asp Val Arg Lys Met Ile Ala Lys
    1010                1015                1020
Ser Glu Gln Glu Ile Gly Lys Ala Thr Ala Lys Tyr Phe Phe Tyr Ser
1025                1030                1035                1040
Asn Ile Met Asn Phe Phe Lys Thr Glu Ile Thr Leu Ala Asn Gly Glu
                1045                1050                1055
Ile Arg Lys Arg Pro Leu Ile Glu Thr Asn Gly Glu Thr Gly Glu Ile
            1060                1065                1070
Val Trp Asp Lys Gly Arg Asp Phe Ala Thr Val Arg Lys Val Leu Ser
        1075                1080                1085
Met Pro Gln Val Asn Ile Val Lys Lys Thr Glu Val Gln Thr Gly Gly
    1090                1095                1100
Phe Ser Lys Glu Ser Ile Leu Pro Lys Arg Asn Ser Asp Lys Leu Ile
1105                1110                1115                1120
Ala Arg Lys Lys Asp Trp Asp Pro Lys Lys Tyr Gly Gly Phe Asp Ser
                1125                1130                1135
Pro Thr Val Ala Tyr Ser Val Leu Val Val Ala Lys Val Glu Lys Gly
            1140                1145                1150
Lys Ser Lys Lys Leu Lys Ser Val Lys Glu Leu Leu Gly Ile Thr Ile
        1155                1160                1165
Met Glu Arg Ser Ser Phe Glu Lys Asn Pro Ile Asp Phe Leu Glu Ala
    1170                1175                1180
Lys Gly Tyr Lys Glu Val Lys Lys Asp Leu Ile Ile Lys Leu Pro Lys
1185                1190                1195                1200
Tyr Ser Leu Phe Glu Leu Glu Asn Gly Arg Lys Arg Met Leu Ala Ser
                1205                1210                1215
Ala Gly Glu Leu Gln Lys Gly Asn Glu Leu Ala Leu Pro Ser Lys Tyr
            1220                1225                1230
Val Asn Phe Leu Tyr Leu Ala Ser His Tyr Glu Lys Leu Lys Gly Ser
        1235                1240                1245
Pro Glu Asp Asn Glu Gln Lys Gln Leu Phe Val Glu Gln His Lys His
    1250                1255                1260
Tyr Leu Asp Glu Ile Ile Glu Gln Ile Ser Glu Phe Ser Lys Arg Val
1265                1270                1275                1280
Ile Leu Ala Asp Ala Asn Leu Asp Lys Val Leu Ser Ala Tyr Asn Lys
                1285                1290                1295
His Arg Asp Lys Pro Ile Arg Glu Gln Ala Glu Asn Ile Ile His Leu
            1300                1305                1310
Phe Thr Leu Thr Asn Leu Gly Ala Pro Ala Ala Phe Lys Tyr Phe Asp
        1315                1320                1325
Thr Thr Ile Asp Arg Lys Arg Tyr Thr Ser Thr Lys Glu Val Leu Asp
    1330                1335                1340
Ala Thr Leu Ile His Gln Ser Ile Thr Gly Leu Tyr Glu Thr Arg Ile
1345                1350                1355                1360
Asp Leu Ser Gln Leu Gly Gly Asp
                1365
```

<210> 20
<211> 1307
<212> PRT
<213> Acidaminococcus sp. BV3L6

<220>
<223> xxx

<400> 20
Met Thr Gln Phe Glu Gly Phe Thr Asn Leu Tyr Gln Val Ser Lys Thr
1               5                   10                  15
Leu Arg Phe Glu Leu Ile Pro Gln Gly Lys Thr Leu Lys His Ile Gln
            20                  25                  30
Glu Gln Gly Phe Ile Glu Glu Asp Lys Ala Arg Asn Asp His Tyr Lys
        35                  40                  45
Glu Leu Lys Pro Ile Ile Asp Arg Ile Tyr Lys Thr Tyr Ala Asp Gln
    50                  55                  60
Cys Leu Gln Leu Val Gln Leu Asp Trp Glu Asn Leu Ser Ala Ala Ile
65                  70                  75                  80
Asp Ser Tyr Arg Lys Glu Lys Thr Glu Glu Thr Arg Asn Ala Leu Ile
                85                  90                  95
Glu Glu Gln Ala Thr Tyr Arg Asn Ala Ile His Asp Tyr Phe Ile Gly
            100                 105                 110
Arg Thr Asp Asn Leu Thr Asp Ala Ile Asn Lys Arg His Ala Glu Ile
        115                 120                 125
Tyr Lys Gly Leu Phe Lys Ala Glu Leu Phe Asn Gly Lys Val Leu Lys
    130                 135                 140
Gln Leu Gly Thr Val Thr Thr Thr Glu His Glu Asn Ala Leu Leu Arg
145                 150                 155                 160
Ser Phe Asp Lys Phe Thr Thr Tyr Phe Ser Gly Phe Tyr Glu Asn Arg
                165                 170                 175
Lys Asn Val Phe Ser Ala Glu Asp Ile Ser Thr Ala Ile Pro His Arg
            180                 185                 190
Ile Val Gln Asp Asn Phe Pro Lys Phe Lys Glu Asn Cys His Ile Phe
        195                 200                 205
Thr Arg Leu Ile Thr Ala Val Pro Ser Leu Arg Glu His Phe Glu Asn
    210                 215                 220
Val Lys Lys Ala Ile Gly Ile Phe Val Ser Thr Ser Ile Glu Glu Val
225                 230                 235                 240
Phe Ser Phe Pro Phe Tyr Asn Gln Leu Leu Thr Gln Thr Gln Ile Asp
                245                 250                 255
Leu Tyr Asn Gln Leu Leu Gly Gly Ile Ser Arg Glu Ala Gly Thr Glu
            260                 265                 270
Lys Ile Lys Gly Leu Asn Glu Val Leu Asn Leu Ala Ile Gln Lys Asn
        275                 280                 285
Asp Glu Thr Ala His Ile Ile Ala Ser Leu Pro His Arg Phe Ile Pro
    290                 295                 300
Leu Phe Lys Gln Ile Leu Ser Asp Arg Asn Thr Leu Ser Phe Ile Leu
305                 310                 315                 320
Glu Glu Phe Lys Ser Asp Glu Glu Val Ile Gln Ser Phe Cys Lys Tyr
                325                 330                 335
Lys Thr Leu Leu Arg Asn Glu Asn Val Leu Glu Thr Ala Glu Ala Leu
            340                 345                 350
Phe Asn Glu Leu Asn Ser Ile Asp Leu Thr His Ile Phe Ile Ser His
        355                 360                 365
Lys Lys Leu Glu Thr Ile Ser Ser Ala Leu Cys Asp His Trp Asp Thr
    370                 375                 380
Leu Arg Asn Ala Leu Tyr Glu Arg Arg Ile Ser Glu Leu Thr Gly Lys
385                 390                 395                 400
Ile Thr Lys Ser Ala Lys Glu Lys Val Gln Arg Ser Leu Lys His Glu
                405                 410                 415
Asp Ile Asn Leu Gln Glu Ile Ile Ser Ala Ala Gly Lys Glu Leu Ser
            420                 425                 430
Glu Ala Phe Lys Gln Lys Thr Ser Glu Ile Leu Ser His Ala His Ala
        435                 440                 445
Ala Leu Asp Gln Pro Leu Pro Thr Thr Leu Lys Lys Gln Glu Glu Lys
    450                 455                 460

Glu Ile Leu Lys Ser Gln Leu Asp Ser Leu Leu Gly Leu Tyr His Leu
465 470 475 480
Leu Asp Trp Phe Ala Val Asp Glu Ser Asn Glu Val Asp Pro Glu Phe
485 490 495
Ser Ala Arg Leu Thr Gly Ile Lys Leu Glu Met Glu Pro Ser Leu Ser
500 505 510
Phe Tyr Asn Lys Ala Arg Asn Tyr Ala Thr Lys Lys Pro Tyr Ser Val
515 520 525
Glu Lys Phe Lys Leu Asn Phe Gln Met Pro Thr Leu Ala Ser Gly Trp
530 535 540
Asp Val Asn Lys Glu Lys Asn Asn Gly Ala Ile Leu Phe Val Lys Asn
545 550 555 560
Gly Leu Tyr Tyr Leu Gly Ile Met Pro Lys Gln Lys Gly Arg Tyr Lys
565 570 575
Ala Leu Ser Phe Glu Pro Thr Glu Lys Thr Ser Glu Gly Phe Asp Lys
580 585 590
Met Tyr Tyr Asp Tyr Phe Pro Asp Ala Ala Lys Met Ile Pro Lys Cys
595 600 605
Ser Thr Gln Leu Lys Ala Val Thr Ala His Phe Gln Thr His Thr Thr
610 615 620
Pro Ile Leu Leu Ser Asn Asn Phe Ile Glu Pro Leu Glu Ile Thr Lys
625 630 635 640
Glu Ile Tyr Asp Leu Asn Asn Pro Glu Lys Glu Pro Lys Lys Phe Gln
645 650 655
Thr Ala Tyr Ala Lys Lys Thr Gly Asp Gln Lys Gly Tyr Arg Glu Ala
660 665 670
Leu Cys Lys Trp Ile Asp Phe Thr Arg Asp Phe Leu Ser Lys Tyr Thr
675 680 685
Lys Thr Thr Ser Ile Asp Leu Ser Ser Leu Arg Pro Ser Ser Gln Tyr
690 695 700
Lys Asp Leu Gly Glu Tyr Tyr Ala Glu Leu Asn Pro Leu Leu Tyr His
705 710 715 720
Ile Ser Phe Gln Arg Ile Ala Glu Lys Glu Ile Met Asp Ala Val Glu
725 730 735
Thr Gly Lys Leu Tyr Leu Phe Gln Ile Tyr Asn Lys Asp Phe Ala Lys
740 745 750
Gly His His Gly Lys Pro Asn Leu His Thr Leu Tyr Trp Thr Gly Leu
755 760 765
Phe Ser Pro Glu Asn Leu Ala Lys Thr Ser Ile Lys Leu Asn Gly Gln
770 775 780
Ala Glu Leu Phe Tyr Arg Pro Lys Ser Arg Met Lys Arg Met Ala His
785 790 795 800
Arg Leu Gly Glu Lys Met Leu Asn Lys Lys Leu Lys Asp Gln Lys Thr
805 810 815
Pro Ile Pro Asp Thr Leu Tyr Gln Glu Leu Tyr Asp Tyr Val Asn His
820 825 830
Arg Leu Ser His Asp Leu Ser Asp Glu Ala Arg Ala Leu Leu Pro Asn
835 840 845
Val Ile Thr Lys Glu Val Ser His Glu Ile Ile Lys Asp Arg Arg Phe
850 855 860
Thr Ser Asp Lys Phe Phe Phe His Val Pro Ile Thr Leu Asn Tyr Gln
865 870 875 880
Ala Ala Asn Ser Pro Ser Lys Phe Asn Gln Arg Val Asn Ala Tyr Leu
885 890 895
Lys Glu His Pro Glu Thr Pro Ile Ile Gly Ile Asp Arg Gly Glu Arg
900 905 910
Asn Leu Ile Tyr Ile Thr Val Ile Asp Ser Thr Gly Lys Ile Leu Glu
915 920 925
Gln Arg Ser Leu Asn Thr Ile Gln Gln Phe Asp Tyr Gln Lys Lys Leu
930 935 940
Asp Asn Arg Glu Lys Glu Arg Val Ala Ala Arg Gln Ala Trp Ser Val
945 950 955 960
Val Gly Thr Ile Lys Asp Leu Lys Gln Gly Tyr Leu Ser Gln Val Ile

```
                965                 970                 975
His Glu Ile Val Asp Leu Met Ile His Tyr Gln Ala Val Val Val Leu
            980                 985                 990
Glu Asn Leu Asn Phe Gly Phe Lys Ser Lys Arg Thr Gly Ile Ala Glu
        995                 1000                1005
Lys Ala Val Tyr Gln Gln Phe Glu Lys Met Leu Ile Asp Lys Leu Asn
    1010                1015                1020
Cys Leu Val Leu Lys Asp Tyr Pro Ala Glu Lys Val Gly Gly Val Leu
1025                1030                1035                1040
Asn Pro Tyr Gln Leu Thr Asp Gln Phe Thr Ser Phe Ala Lys Met Gly
                1045                1050                1055
Thr Gln Ser Gly Phe Leu Phe Tyr Val Pro Ala Pro Tyr Thr Ser Lys
            1060                1065                1070
Ile Asp Pro Leu Thr Gly Phe Val Asp Pro Phe Val Trp Lys Thr Ile
        1075                1080                1085
Lys Asn His Glu Ser Arg Lys His Phe Leu Glu Gly Phe Asp Phe Leu
    1090                1095                1100
His Tyr Asp Val Lys Thr Gly Asp Phe Ile Leu His Phe Lys Met Asn
1105                1110                1115                1120
Arg Asn Leu Ser Phe Gln Arg Gly Leu Pro Gly Phe Met Pro Ala Trp
                1125                1130                1135
Asp Ile Val Phe Glu Lys Asn Glu Thr Gln Phe Asp Ala Lys Gly Thr
            1140                1145                1150
Pro Phe Ile Ala Gly Lys Arg Ile Val Pro Val Ile Glu Asn His Arg
        1155                1160                1165
Phe Thr Gly Arg Tyr Arg Asp Leu Tyr Pro Ala Asn Glu Leu Ile Ala
    1170                1175                1180
Leu Leu Glu Glu Lys Gly Ile Val Phe Arg Asp Gly Ser Asn Ile Leu
1185                1190                1195                1200
Pro Lys Leu Leu Glu Asn Asp Asp Ser His Ala Ile Asp Thr Met Val
                1205                1210                1215
Ala Leu Ile Arg Ser Val Leu Gln Met Arg Asn Ser Asn Ala Ala Thr
            1220                1225                1230
Gly Glu Asp Tyr Ile Asn Ser Pro Val Arg Asp Leu Asn Gly Val Cys
        1235                1240                1245
Phe Asp Ser Arg Phe Gln Asn Pro Glu Trp Pro Met Asp Ala Asp Ala
    1250                1255                1260
Asn Gly Ala Tyr His Ile Ala Leu Lys Gly Gln Leu Leu Leu Asn His
1265                1270                1275                1280
Leu Lys Glu Ser Lys Asp Leu Lys Leu Gln Asn Gly Ile Ser Asn Gln
                1285                1290                1295
Asp Trp Leu Ala Tyr Ile Gln Glu Leu Arg Asn
            1300                1305
```

<210> 21
<211> 984
<212> PRT
<213> Campylobacter jejuni subsp. jejuni

<220>
<223> xxx

<400> 21

```
Met Ala Arg Ile Leu Ala Phe Asp Ile Gly Ile Ser Ser Ile Gly Trp
1               5                   10                  15
Ala Phe Ser Glu Asn Asp Glu Leu Lys Asp Cys Gly Val Arg Ile Phe
            20                  25                  30
Thr Lys Val Glu Asn Pro Lys Thr Gly Glu Ser Leu Ala Leu Pro Arg
        35                  40                  45
Arg Leu Ala Arg Ser Ala Arg Lys Arg Leu Ala Arg Arg Lys Ala Arg
    50                  55                  60
Leu Asn His Leu Lys His Leu Ile Ala Asn Glu Phe Lys Leu Asn Tyr
65                  70                  75                  80
```

```
Glu Asp Tyr Gln Ser Phe Asp Glu Ser Leu Ala Lys Ala Tyr Lys Gly
                85                  90                  95
Ser Leu Ile Ser Pro Tyr Glu Leu Arg Phe Arg Ala Leu Asn Glu Leu
            100                 105                 110
Leu Ser Lys Gln Asp Phe Ala Arg Val Ile Leu His Ile Ala Lys Arg
        115                 120                 125
Arg Gly Tyr Asp Asp Ile Lys Asn Ser Asp Asp Lys Glu Lys Gly Ala
    130                 135                 140
Ile Leu Lys Ala Ile Lys Gln Asn Glu Glu Lys Leu Ala Asn Tyr Gln
145                 150                 155                 160
Ser Val Gly Glu Tyr Leu Tyr Lys Glu Tyr Phe Gln Lys Phe Lys Glu
                165                 170                 175
Asn Ser Lys Glu Phe Thr Asn Val Arg Asn Lys Lys Glu Ser Tyr Glu
            180                 185                 190
Arg Cys Ile Ala Gln Ser Phe Leu Lys Asp Glu Leu Lys Leu Ile Phe
        195                 200                 205
Lys Lys Gln Arg Glu Phe Gly Phe Ser Phe Ser Lys Lys Phe Glu Glu
    210                 215                 220
Glu Val Leu Ser Val Ala Phe Tyr Lys Arg Ala Leu Lys Asp Phe Ser
225                 230                 235                 240
His Leu Val Gly Asn Cys Ser Phe Phe Thr Asp Glu Lys Arg Ala Pro
                245                 250                 255
Lys Asn Ser Pro Leu Ala Phe Met Phe Val Ala Leu Thr Arg Ile Ile
            260                 265                 270
Asn Leu Leu Asn Asn Leu Lys Asn Thr Glu Gly Ile Leu Tyr Thr Lys
        275                 280                 285
Asp Asp Leu Asn Ala Leu Leu Asn Glu Val Leu Lys Asn Gly Thr Leu
    290                 295                 300
Thr Tyr Lys Gln Thr Lys Lys Leu Leu Gly Leu Ser Asp Asp Tyr Glu
305                 310                 315                 320
Phe Lys Gly Glu Lys Gly Thr Tyr Phe Ile Glu Phe Lys Lys Tyr Lys
                325                 330                 335
Glu Phe Ile Lys Ala Leu Gly Glu His Asn Leu Ser Gln Asp Asp Leu
            340                 345                 350
Asn Glu Ile Ala Lys Asp Ile Thr Leu Ile Lys Asp Glu Ile Lys Leu
        355                 360                 365
Lys Lys Ala Leu Ala Lys Tyr Asp Leu Asn Gln Asn Gln Ile Asp Ser
    370                 375                 380
Leu Ser Lys Leu Glu Phe Lys Asp His Leu Asn Ile Ser Phe Lys Ala
385                 390                 395                 400
Leu Lys Leu Val Thr Pro Leu Met Leu Glu Gly Lys Lys Tyr Asp Glu
                405                 410                 415
Ala Cys Asn Glu Leu Asn Leu Lys Val Ala Ile Asn Glu Asp Lys Lys
            420                 425                 430
Asp Phe Leu Pro Ala Phe Asn Glu Thr Tyr Tyr Lys Asp Glu Val Thr
        435                 440                 445
Asn Pro Val Val Leu Arg Ala Ile Lys Glu Tyr Arg Lys Val Leu Asn
    450                 455                 460
Ala Leu Leu Lys Lys Tyr Gly Lys Val His Lys Ile Asn Ile Glu Leu
465                 470                 475                 480
Ala Arg Glu Val Gly Lys Asn His Ser Gln Arg Ala Lys Ile Glu Lys
                485                 490                 495
Glu Gln Asn Glu Asn Tyr Lys Ala Lys Lys Asp Ala Glu Leu Glu Cys
            500                 505                 510
Glu Lys Leu Gly Leu Lys Ile Asn Ser Lys Asn Ile Leu Lys Leu Arg
        515                 520                 525
Leu Phe Lys Glu Gln Lys Glu Phe Cys Ala Tyr Ser Gly Glu Lys Ile
    530                 535                 540
Lys Ile Ser Asp Leu Gln Asp Glu Lys Met Leu Glu Ile Asp His Ile
545                 550                 555                 560
Tyr Pro Tyr Ser Arg Ser Phe Asp Asp Ser Tyr Met Asn Lys Val Leu
                565                 570                 575
Val Phe Thr Lys Gln Asn Gln Glu Lys Leu Asn Gln Thr Pro Phe Glu
```

```
                580                 585                 590
Ala Phe Gly Asn Asp Ser Ala Lys Trp Gln Lys Ile Glu Val Leu Ala
            595                 600                 605
Lys Asn Leu Pro Thr Lys Lys Gln Lys Arg Ile Leu Asp Lys Asn Tyr
    610                 615                 620
Lys Asp Lys Glu Gln Lys Asn Phe Lys Asp Arg Asn Leu Asn Asp Thr
625                 630                 635                 640
Arg Tyr Ile Ala Arg Leu Val Leu Asn Tyr Thr Lys Asp Tyr Leu Asp
                645                 650                 655
Phe Leu Pro Leu Ser Asp Asp Glu Asn Thr Lys Leu Asn Asp Thr Gln
            660                 665                 670
Lys Gly Ser Lys Val His Val Glu Ala Lys Ser Gly Met Leu Thr Ser
        675                 680                 685
Ala Leu Arg His Thr Trp Gly Phe Ser Ala Lys Asp Arg Asn Asn His
    690                 695                 700
Leu His His Ala Ile Asp Ala Val Ile Ile Ala Tyr Ala Asn Asn Ser
705                 710                 715                 720
Ile Val Lys Ala Phe Ser Asp Phe Lys Lys Glu Gln Glu Ser Asn Ser
                725                 730                 735
Ala Glu Leu Tyr Ala Lys Lys Ile Ser Glu Leu Asp Tyr Lys Asn Lys
            740                 745                 750
Arg Lys Phe Phe Glu Pro Phe Ser Gly Phe Arg Gln Lys Val Leu Asp
        755                 760                 765
Lys Ile Asp Glu Ile Phe Val Ser Lys Pro Glu Arg Lys Lys Pro Ser
    770                 775                 780
Gly Ala Leu His Glu Glu Thr Phe Arg Lys Glu Glu Glu Phe Tyr Gln
785                 790                 795                 800
Ser Tyr Gly Gly Lys Glu Gly Val Leu Lys Ala Leu Glu Leu Gly Lys
                805                 810                 815
Ile Arg Lys Val Asn Gly Lys Ile Val Lys Asn Gly Asp Met Phe Arg
            820                 825                 830
Val Asp Ile Phe Lys His Lys Lys Thr Asn Lys Phe Tyr Ala Val Pro
        835                 840                 845
Ile Tyr Thr Met Asp Phe Ala Leu Lys Val Leu Pro Asn Lys Ala Val
    850                 855                 860
Ala Arg Ser Lys Lys Gly Glu Ile Lys Asp Trp Ile Leu Met Asp Glu
865                 870                 875                 880
Asn Tyr Glu Phe Cys Phe Ser Leu Tyr Lys Asp Ser Leu Ile Leu Ile
                885                 890                 895
Gln Thr Lys Asp Met Gln Glu Pro Glu Phe Val Tyr Tyr Asn Ala Phe
            900                 905                 910
Thr Ser Ser Thr Val Ser Leu Ile Val Ser Lys His Asp Asn Lys Phe
        915                 920                 925
Glu Thr Leu Ser Lys Asn Gln Lys Ile Leu Phe Lys Asn Ala Asn Glu
    930                 935                 940
Lys Glu Val Ile Ala Lys Ser Ile Gly Ile Gln Asn Leu Lys Val Phe
945                 950                 955                 960
Glu Lys Tyr Ile Val Ser Ala Leu Gly Glu Val Thr Lys Ala Glu Phe
                965                 970                 975
Arg Gln Arg Glu Asp Phe Lys Lys
            980
```

<210> 22
<211> 1629
<212> PRT
<213> Francisella tularensis

<220>
<223> xxx

<400> 22

```
Met Asn Phe Lys Ile Leu Pro Ile Ala Ile Asp Leu Gly Val Lys Asn
1               5                   10                  15
```

```
Thr Gly Val Phe Ser Ala Phe Tyr Gln Lys Gly Thr Ser Leu Glu Arg
            20                  25                  30
Leu Asp Asn Lys Asn Gly Lys Val Tyr Glu Leu Ser Lys Asp Ser Tyr
        35                  40                  45
Thr Leu Leu Met Asn Asn Arg Thr Ala Arg Arg His Gln Arg Arg Gly
    50                  55                  60
Ile Asp Arg Lys Gln Leu Val Lys Arg Leu Phe Lys Leu Ile Trp Thr
65                  70                  75                  80
Glu Gln Leu Asn Leu Glu Trp Asp Lys Asp Thr Gln Gln Ala Ile Ser
                85                  90                  95
Phe Leu Phe Asn Arg Arg Gly Phe Ser Phe Ile Thr Asp Gly Tyr Ser
            100                 105                 110
Pro Glu Tyr Leu Asn Ile Val Pro Glu Gln Val Lys Ala Ile Leu Met
        115                 120                 125
Asp Ile Phe Asp Asp Tyr Asn Gly Glu Asp Asp Leu Asp Ser Tyr Leu
    130                 135                 140
Lys Leu Ala Thr Glu Gln Glu Ser Lys Ile Ser Glu Ile Tyr Asn Lys
145                 150                 155                 160
Leu Met Gln Lys Ile Leu Glu Phe Lys Leu Met Lys Leu Cys Thr Asp
                165                 170                 175
Ile Lys Asp Asp Lys Val Ser Thr Lys Thr Leu Lys Glu Ile Thr Ser
            180                 185                 190
Tyr Glu Phe Glu Leu Leu Ala Asp Tyr Leu Ala Asn Tyr Ser Glu Ser
        195                 200                 205
Leu Lys Thr Gln Lys Phe Ser Tyr Thr Asp Lys Gln Gly Asn Leu Lys
    210                 215                 220
Glu Leu Ser Tyr Tyr His His Asp Lys Tyr Asn Ile Gln Glu Phe Leu
225                 230                 235                 240
Lys Arg His Ala Thr Ile Asn Asp Arg Ile Leu Asp Thr Leu Leu Thr
                245                 250                 255
Asp Asp Leu Asp Ile Trp Asn Phe Asn Phe Glu Lys Phe Asp Phe Asp
            260                 265                 270
Lys Asn Glu Glu Lys Leu Gln Asn Gln Glu Asp Lys Asp His Ile Gln
        275                 280                 285
Ala His Leu His His Phe Val Phe Ala Val Asn Lys Ile Lys Ser Glu
    290                 295                 300
Met Ala Ser Gly Gly Arg His Arg Ser Gln Tyr Phe Gln Glu Ile Thr
305                 310                 315                 320
Asn Val Leu Asp Glu Asn Asn His Gln Glu Gly Tyr Leu Lys Asn Phe
                325                 330                 335
Cys Glu Asn Leu His Asn Lys Lys Tyr Ser Asn Leu Ser Val Lys Asn
            340                 345                 350
Leu Val Asn Leu Ile Gly Asn Leu Ser Asn Leu Glu Leu Lys Pro Leu
        355                 360                 365
Arg Lys Tyr Phe Asn Asp Lys Ile His Ala Lys Ala Asp His Trp Asp
    370                 375                 380
Glu Gln Lys Phe Thr Glu Thr Tyr Cys His Trp Ile Leu Gly Glu Trp
385                 390                 395                 400
Arg Val Gly Val Lys Asp Gln Asp Lys Lys Asp Gly Ala Lys Tyr Ser
                405                 410                 415
Tyr Lys Asp Leu Cys Asn Glu Leu Lys Gln Lys Val Thr Lys Ala Gly
            420                 425                 430
Leu Val Asp Phe Leu Leu Glu Leu Asp Pro Cys Arg Thr Ile Pro Pro
        435                 440                 445
Tyr Leu Asp Asn Asn Asn Arg Lys Pro Pro Lys Cys Gln Ser Leu Ile
    450                 455                 460
Leu Asn Pro Lys Phe Leu Asp Asn Gln Tyr Pro Asn Trp Gln Gln Tyr
465                 470                 475                 480
Leu Gln Glu Leu Lys Lys Leu Gln Ser Ile Gln Asn Tyr Leu Asp Ser
                485                 490                 495
Phe Glu Thr Asp Leu Lys Val Leu Lys Ser Ser Lys Asp Gln Pro Tyr
            500                 505                 510
Phe Val Glu Tyr Lys Ser Ser Asn Gln Gln Ile Ala Ser Gly Gln Arg
```

```
        515                 520                 525
Asp Tyr Lys Asp Leu Asp Ala Arg Ile Leu Gln Phe Ile Phe Asp Arg
    530                 535                 540
Val Lys Ala Ser Asp Glu Leu Leu Leu Asn Glu Ile Tyr Phe Gln Ala
545                 550                 555                 560
Lys Lys Leu Lys Gln Lys Ala Ser Ser Glu Leu Glu Lys Leu Glu Ser
                565                 570                 575
Ser Lys Lys Leu Asp Glu Val Ile Ala Asn Ser Gln Leu Ser Gln Ile
            580                 585                 590
Leu Lys Ser Gln His Thr Asn Gly Ile Phe Glu Gln Gly Thr Phe Leu
        595                 600                 605
His Leu Val Cys Lys Tyr Tyr Lys Gln Arg Gln Arg Ala Arg Asp Ser
    610                 615                 620
Arg Leu Tyr Ile Met Pro Glu Tyr Arg Tyr Asp Lys Lys Leu His Lys
625                 630                 635                 640
Tyr Asn Asn Thr Gly Arg Phe Asp Asp Asp Asn Gln Leu Leu Thr Tyr
                645                 650                 655
Cys Asn His Lys Pro Arg Gln Lys Arg Tyr Gln Leu Leu Asn Asp Leu
            660                 665                 670
Ala Gly Val Leu Gln Val Ser Pro Asn Phe Leu Lys Asp Lys Ile Gly
        675                 680                 685
Ser Asp Asp Asp Leu Phe Ile Ser Lys Trp Leu Val Glu His Ile Arg
    690                 695                 700
Gly Phe Lys Lys Ala Cys Glu Asp Ser Leu Lys Ile Gln Lys Asp Asn
705                 710                 715                 720
Arg Gly Leu Leu Asn His Lys Ile Asn Ile Ala Arg Asn Thr Lys Gly
                725                 730                 735
Lys Cys Glu Lys Glu Ile Phe Asn Leu Ile Cys Lys Ile Glu Gly Ser
            740                 745                 750
Glu Asp Lys Lys Gly Asn Tyr Lys His Gly Leu Ala Tyr Glu Leu Gly
        755                 760                 765
Val Leu Leu Phe Gly Glu Pro Asn Glu Ala Ser Lys Pro Glu Phe Asp
    770                 775                 780
Arg Lys Ile Lys Lys Phe Asn Ser Ile Tyr Ser Phe Ala Gln Ile Gln
785                 790                 795                 800
Gln Ile Ala Phe Ala Glu Arg Lys Gly Asn Ala Asn Thr Cys Ala Val
                805                 810                 815
Cys Ser Ala Asp Asn Ala His Arg Met Gln Gln Ile Lys Ile Thr Glu
            820                 825                 830
Pro Val Glu Asp Asn Lys Asp Lys Ile Ile Leu Ser Ala Lys Ala Gln
        835                 840                 845
Arg Leu Pro Ala Ile Pro Thr Arg Ile Val Asp Gly Ala Val Lys Lys
    850                 855                 860
Met Ala Thr Ile Leu Ala Lys Asn Ile Val Asp Asp Asn Trp Gln Asn
865                 870                 875                 880
Ile Lys Gln Val Leu Ser Ala Lys His Gln Leu His Ile Pro Ile Ile
                885                 890                 895
Thr Glu Ser Asn Ala Phe Glu Phe Glu Pro Ala Leu Ala Asp Val Lys
            900                 905                 910
Gly Lys Ser Leu Lys Asp Arg Arg Lys Lys Ala Leu Glu Arg Ile Ser
        915                 920                 925
Pro Glu Asn Ile Phe Lys Asp Lys Asn Asn Arg Ile Lys Glu Phe Ala
    930                 935                 940
Lys Gly Ile Ser Ala Tyr Ser Gly Ala Asn Leu Thr Asp Gly Asp Phe
945                 950                 955                 960
Asp Gly Ala Lys Glu Glu Leu Asp His Ile Ile Pro Arg Ser His Lys
                965                 970                 975
Lys Tyr Gly Thr Leu Asn Asp Glu Ala Asn Leu Ile Cys Val Thr Arg
            980                 985                 990
Gly Asp Asn Lys Asn Lys Gly Asn Arg Ile Phe Cys Leu Arg Asp Leu
        995                 1000                1005
Ala Asp Asn Tyr Lys Leu Lys Gln Phe Glu Thr Thr Asp Asp Leu Glu
    1010                1015                1020
```

Ile Glu Lys Lys Ile Ala Asp Thr Ile Trp Asp Ala Asn Lys Lys Asp
1025 1030 1035 1040
Phe Lys Phe Gly Asn Tyr Arg Ser Phe Ile Asn Leu Thr Pro Gln Glu
1045 1050 1055
Gln Lys Ala Phe Arg His Ala Leu Phe Leu Ala Asp Glu Asn Pro Ile
1060 1065 1070
Lys Gln Ala Val Ile Arg Ala Ile Asn Asn Arg Asn Arg Thr Phe Val
1075 1080 1085
Asn Gly Thr Gln Arg Tyr Phe Ala Glu Val Leu Ala Asn Asn Ile Tyr
1090 1095 1100
Leu Arg Ala Lys Lys Glu Asn Leu Asn Thr Asp Lys Ile Ser Phe Asp
1105 1110 1115 1120
Tyr Phe Gly Ile Pro Thr Ile Gly Asn Gly Arg Gly Ile Ala Glu Ile
1125 1130 1135
Arg Gln Leu Tyr Glu Lys Val Asp Ser Asp Ile Gln Ala Tyr Ala Lys
1140 1145 1150
Gly Asp Lys Pro Gln Ala Ser Tyr Ser His Leu Ile Asp Ala Met Leu
1155 1160 1165
Ala Phe Cys Ile Ala Ala Asp Glu His Arg Asn Asp Gly Ser Ile Gly
1170 1175 1180
Leu Glu Ile Asp Lys Asn Tyr Ser Leu Tyr Pro Leu Asp Lys Asn Thr
1185 1190 1195 1200
Gly Glu Val Phe Thr Lys Asp Ile Phe Ser Gln Ile Lys Ile Thr Asp
1205 1210 1215
Asn Glu Phe Ser Asp Lys Lys Leu Val Arg Lys Lys Ala Ile Glu Gly
1220 1225 1230
Phe Asn Thr His Arg Gln Met Thr Arg Asp Gly Ile Tyr Ala Glu Asn
1235 1240 1245
Tyr Leu Pro Ile Leu Ile His Lys Glu Leu Asn Glu Val Arg Lys Gly
1250 1255 1260
Tyr Thr Trp Lys Asn Ser Glu Glu Ile Lys Ile Phe Lys Gly Lys Lys
1265 1270 1275 1280
Tyr Asp Ile Gln Gln Leu Asn Asn Leu Val Tyr Cys Leu Lys Phe Val
1285 1290 1295
Asp Lys Pro Ile Ser Ile Asp Ile Gln Ile Ser Thr Leu Glu Glu Leu
1300 1305 1310
Arg Asn Ile Leu Thr Thr Asn Asn Ile Ala Ala Thr Ala Glu Tyr Tyr
1315 1320 1325
Tyr Ile Asn Leu Lys Thr Gln Lys Leu His Glu Tyr Tyr Ile Glu Asn
1330 1335 1340
Tyr Asn Thr Ala Leu Gly Tyr Lys Lys Tyr Ser Lys Glu Met Glu Phe
1345 1350 1355 1360
Leu Arg Ser Leu Ala Tyr Arg Ser Glu Arg Val Lys Ile Lys Ser Ile
1365 1370 1375
Asp Asp Val Lys Gln Val Leu Asp Lys Asp Ser Asn Phe Ile Ile Gly
1380 1385 1390
Lys Ile Thr Leu Pro Phe Lys Lys Glu Trp Gln Arg Leu Tyr Arg Glu
1395 1400 1405
Trp Gln Asn Thr Thr Ile Lys Asp Asp Tyr Glu Phe Leu Lys Ser Phe
1410 1415 1420
Phe Asn Val Lys Ser Ile Thr Lys Leu His Lys Lys Val Arg Lys Asp
1425 1430 1435 1440
Phe Ser Leu Pro Ile Ser Thr Asn Glu Gly Lys Phe Leu Val Lys Arg
1445 1450 1455
Lys Thr Trp Asp Asn Asn Phe Ile Tyr Gln Ile Leu Asn Asp Ser Asp
1460 1465 1470
Ser Arg Ala Asp Gly Thr Lys Pro Phe Ile Pro Ala Phe Asp Ile Ser
1475 1480 1485
Lys Asn Glu Ile Val Glu Ala Ile Ile Asp Ser Phe Thr Ser Lys Asn
1490 1495 1500
Ile Phe Trp Leu Pro Lys Asn Ile Glu Leu Gln Lys Val Asp Asn Lys
1505 1510 1515 1520
Asn Ile Phe Ala Ile Asp Thr Ser Lys Trp Phe Glu Val Glu Thr Pro

1525 1530 1535
Ser Asp Leu Arg Asp Ile Gly Ile Ala Thr Ile Gln Tyr Lys Ile Asp
1540 1545 1550
Asn Asn Ser Arg Pro Lys Val Arg Val Lys Leu Asp Tyr Val Ile Asp
1555 1560 1565
Asp Asp Ser Lys Ile Asn Tyr Phe Met Asn His Ser Leu Leu Lys Ser
1570 1575 1580
Arg Tyr Pro Asp Lys Val Leu Glu Ile Leu Lys Gln Ser Thr Ile Ile
1585 1590 1595 1600
Glu Phe Glu Ser Ser Gly Phe Asn Lys Thr Ile Lys Glu Met Leu Gly
1605 1610 1615
Met Lys Leu Ala Gly Ile Tyr Asn Glu Thr Ser Asn Asn
1620 1625

<210> 23
<211> 1368
<212> PRT
<213> Artificial Sequence

<220>
<223> cas9 endonuclease

<400> 23
Met Asp Lys Lys Tyr Ser Ile Gly Leu Ala Ile Gly Thr Asn Ser Val
1 5 10 15
Gly Trp Ala Val Ile Thr Asp Glu Tyr Lys Val Pro Ser Lys Lys Phe
20 25 30
Lys Val Leu Gly Asn Thr Asp Arg His Ser Ile Lys Lys Asn Leu Ile
35 40 45
Gly Ala Leu Leu Phe Asp Ser Gly Glu Thr Ala Glu Ala Thr Arg Leu
50 55 60
Lys Arg Thr Ala Arg Arg Arg Tyr Thr Arg Arg Lys Asn Arg Ile Cys
65 70 75 80
Tyr Leu Gln Glu Ile Phe Ser Asn Glu Met Ala Lys Val Asp Asp Ser
85 90 95
Phe Phe His Arg Leu Glu Glu Ser Phe Leu Val Glu Glu Asp Lys Lys
100 105 110
His Glu Arg His Pro Ile Phe Gly Asn Ile Val Asp Glu Val Ala Tyr
115 120 125
His Glu Lys Tyr Pro Thr Ile Tyr His Leu Arg Lys Lys Leu Val Asp
130 135 140
Ser Thr Asp Lys Ala Asp Leu Arg Leu Ile Tyr Leu Ala Leu Ala His
145 150 155 160
Met Ile Lys Phe Arg Gly His Phe Leu Ile Glu Gly Asp Leu Asn Pro
165 170 175
Asp Asn Ser Asp Val Asp Lys Leu Phe Ile Gln Leu Val Gln Thr Tyr
180 185 190
Asn Gln Leu Phe Glu Glu Asn Pro Ile Asn Ala Ser Gly Val Asp Ala
195 200 205
Lys Ala Ile Leu Ser Ala Arg Leu Ser Lys Ser Arg Arg Leu Glu Asn
210 215 220
Leu Ile Ala Gln Leu Pro Gly Glu Lys Lys Asn Gly Leu Phe Gly Asn
225 230 235 240
Leu Ile Ala Leu Ser Leu Gly Leu Thr Pro Asn Phe Lys Ser Asn Phe
245 250 255
Asp Leu Ala Glu Asp Ala Lys Leu Gln Leu Ser Lys Asp Thr Tyr Asp
260 265 270
Asp Asp Leu Asp Asn Leu Leu Ala Gln Ile Gly Asp Gln Tyr Ala Asp
275 280 285
Leu Phe Leu Ala Ala Lys Asn Leu Ser Asp Ala Ile Leu Leu Ser Asp
290 295 300
Ile Leu Arg Val Asn Thr Glu Ile Thr Lys Ala Pro Leu Ser Ala Ser
305 310 315 320

```
Met Ile Lys Arg Tyr Asp Glu His His Gln Asp Leu Thr Leu Leu Lys
                325                 330                 335
Ala Leu Val Arg Gln Gln Leu Pro Glu Lys Tyr Lys Glu Ile Phe Phe
            340                 345                 350
Asp Gln Ser Lys Asn Gly Tyr Ala Gly Tyr Ile Asp Gly Gly Ala Ser
        355                 360                 365
Gln Glu Glu Phe Tyr Lys Phe Ile Lys Pro Ile Leu Glu Lys Met Asp
    370                 375                 380
Gly Thr Glu Glu Leu Leu Val Lys Leu Asn Arg Glu Asp Leu Leu Arg
385                 390                 395                 400
Lys Gln Arg Thr Phe Asp Asn Gly Ser Ile Pro His Gln Ile His Leu
                405                 410                 415
Gly Glu Leu His Ala Ile Leu Arg Arg Gln Glu Asp Phe Tyr Pro Phe
            420                 425                 430
Leu Lys Asp Asn Arg Glu Lys Ile Glu Lys Ile Leu Thr Phe Arg Ile
        435                 440                 445
Pro Tyr Tyr Val Gly Pro Leu Ala Arg Gly Asn Ser Arg Phe Ala Trp
    450                 455                 460
Met Thr Arg Lys Ser Glu Glu Thr Ile Thr Pro Trp Asn Phe Glu Glu
465                 470                 475                 480
Val Val Asp Lys Gly Ala Ser Ala Gln Ser Phe Ile Glu Arg Met Thr
                485                 490                 495
Asn Phe Asp Lys Asn Leu Pro Asn Glu Lys Val Leu Pro Lys His Ser
            500                 505                 510
Leu Leu Tyr Glu Tyr Phe Thr Val Tyr Asn Glu Leu Thr Lys Val Lys
        515                 520                 525
Tyr Val Thr Glu Gly Met Arg Lys Pro Ala Phe Leu Ser Gly Glu Gln
    530                 535                 540
Lys Lys Ala Ile Val Asp Leu Leu Phe Lys Thr Asn Arg Lys Val Thr
545                 550                 555                 560
Val Lys Gln Leu Lys Glu Asp Tyr Phe Lys Lys Ile Glu Cys Phe Asp
                565                 570                 575
Ser Val Glu Ile Ser Gly Val Glu Asp Arg Phe Asn Ala Ser Leu Gly
            580                 585                 590
Thr Tyr His Asp Leu Leu Lys Ile Ile Lys Asp Lys Asp Phe Leu Asp
        595                 600                 605
Asn Glu Glu Asn Glu Asp Ile Leu Glu Asp Ile Val Leu Thr Leu Thr
    610                 615                 620
Leu Phe Glu Asp Arg Glu Met Ile Glu Glu Arg Leu Lys Thr Tyr Ala
625                 630                 635                 640
His Leu Phe Asp Asp Lys Val Met Lys Gln Leu Lys Arg Arg Arg Tyr
                645                 650                 655
Thr Gly Trp Gly Arg Leu Ser Arg Lys Leu Ile Asn Gly Ile Arg Asp
            660                 665                 670
Lys Gln Ser Gly Lys Thr Ile Leu Asp Phe Leu Lys Ser Asp Gly Phe
        675                 680                 685
Ala Asn Arg Asn Phe Met Gln Leu Ile His Asp Asp Ser Leu Thr Phe
    690                 695                 700
Lys Glu Asp Ile Gln Lys Ala Gln Val Ser Gly Gln Gly Asp Ser Leu
705                 710                 715                 720
His Glu His Ile Ala Asn Leu Ala Gly Ser Pro Ala Ile Lys Lys Gly
                725                 730                 735
Ile Leu Gln Thr Val Lys Val Val Asp Glu Leu Val Lys Val Met Gly
            740                 745                 750
Arg His Lys Pro Glu Asn Ile Val Ile Glu Met Ala Arg Glu Asn Gln
        755                 760                 765
Thr Thr Gln Lys Gly Gln Lys Asn Ser Arg Glu Arg Met Lys Arg Ile
    770                 775                 780
Glu Glu Gly Ile Lys Glu Leu Gly Ser Gln Ile Leu Lys Glu His Pro
785                 790                 795                 800
Val Glu Asn Thr Gln Leu Gln Asn Glu Lys Leu Tyr Leu Tyr Tyr Leu
                805                 810                 815
Gln Asn Gly Arg Asp Met Tyr Val Asp Gln Glu Leu Asp Ile Asn Arg
```

```
            820                 825                 830
Leu Ser Asp Tyr Asp Val Asp Ala Ile Val Pro Gln Ser Phe Leu Lys
        835                 840                 845
Asp Asp Ser Ile Asp Asn Lys Val Leu Thr Arg Ser Asp Lys Asn Arg
    850                 855                 860
Gly Lys Ser Asp Asn Val Pro Ser Glu Glu Val Val Lys Lys Met Lys
865                 870                 875                 880
Asn Tyr Trp Arg Gln Leu Leu Asn Ala Lys Leu Ile Thr Gln Arg Lys
                885                 890                 895
Phe Asp Asn Leu Thr Lys Ala Glu Arg Gly Gly Leu Ser Glu Leu Asp
            900                 905                 910
Lys Ala Gly Phe Ile Lys Arg Gln Leu Val Glu Thr Arg Gln Ile Thr
        915                 920                 925
Lys His Val Ala Gln Ile Leu Asp Ser Arg Met Asn Thr Lys Tyr Asp
    930                 935                 940
Glu Asn Asp Lys Leu Ile Arg Glu Val Lys Val Ile Thr Leu Lys Ser
945                 950                 955                 960
Lys Leu Val Ser Asp Phe Arg Lys Asp Phe Gln Phe Tyr Lys Val Arg
                965                 970                 975
Glu Ile Asn Asn Tyr His His Ala His Asp Ala Tyr Leu Asn Ala Val
            980                 985                 990
Val Gly Thr Ala Leu Ile Lys Lys Tyr Pro Lys Leu Glu Ser Glu Phe
        995                 1000                1005
Val Tyr Gly Asp Tyr Lys Val Tyr Asp Val Arg Lys Met Ile Ala Lys
    1010                1015                1020
Ser Glu Gln Glu Ile Gly Lys Ala Thr Ala Lys Tyr Phe Phe Tyr Ser
1025                1030                1035                1040
Asn Ile Met Asn Phe Phe Lys Thr Glu Ile Thr Leu Ala Asn Gly Glu
                1045                1050                1055
Ile Arg Lys Arg Pro Leu Ile Glu Thr Asn Gly Glu Thr Gly Glu Ile
            1060                1065                1070
Val Trp Asp Lys Gly Arg Asp Phe Ala Thr Val Arg Lys Val Leu Ser
        1075                1080                1085
Met Pro Gln Val Asn Ile Val Lys Lys Thr Glu Val Gln Thr Gly Gly
    1090                1095                1100
Phe Ser Lys Glu Ser Ile Leu Pro Lys Arg Asn Ser Asp Lys Leu Ile
1105                1110                1115                1120
Ala Arg Lys Lys Asp Trp Asp Pro Lys Lys Tyr Gly Gly Phe Asp Ser
                1125                1130                1135
Pro Thr Val Ala Tyr Ser Val Leu Val Val Ala Lys Val Glu Lys Gly
            1140                1145                1150
Lys Ser Lys Lys Leu Lys Ser Val Lys Glu Leu Leu Gly Ile Thr Ile
        1155                1160                1165
Met Glu Arg Ser Ser Phe Glu Lys Asn Pro Ile Asp Phe Leu Glu Ala
    1170                1175                1180
Lys Gly Tyr Lys Glu Val Lys Lys Asp Leu Ile Ile Lys Leu Pro Lys
1185                1190                1195                1200
Tyr Ser Leu Phe Glu Leu Glu Asn Gly Arg Lys Arg Met Leu Ala Ser
                1205                1210                1215
Ala Gly Glu Leu Gln Lys Gly Asn Glu Leu Ala Leu Pro Ser Lys Tyr
            1220                1225                1230
Val Asn Phe Leu Tyr Leu Ala Ser His Tyr Glu Lys Leu Lys Gly Ser
        1235                1240                1245
Pro Glu Asp Asn Glu Gln Lys Gln Leu Phe Val Glu Gln His Lys His
    1250                1255                1260
Tyr Leu Asp Glu Ile Ile Glu Gln Ile Ser Glu Phe Ser Lys Arg Val
1265                1270                1275                1280
Ile Leu Ala Asp Ala Asn Leu Asp Lys Val Leu Ser Ala Tyr Asn Lys
                1285                1290                1295
His Arg Asp Lys Pro Ile Arg Glu Gln Ala Glu Asn Ile Ile His Leu
            1300                1305                1310
Phe Thr Leu Thr Asn Leu Gly Ala Pro Ala Ala Phe Lys Tyr Phe Asp
        1315                1320                1325
```

```
Thr Thr Ile Asp Arg Lys Arg Tyr Thr Ser Thr Lys Glu Val Leu Asp
    1330                1335                1340
Ala Thr Leu Ile His Gln Ser Ile Thr Gly Leu Tyr Glu Thr Arg Ile
1345                1350                1355                1360
Asp Leu Ser Gln Leu Gly Gly Asp
                1365
```

<210> 24
<211> 221
<212> RNA
<213> Artificial Sequence

<220>
<223> pre-gRNA

<220>
<221> misc_feature
<222> 1
<223> /note="N=any nucleotide A, G, U or C and complementary to N at position 49"

<220>
<221> misc_feature
<222> 2
<223> /note="N=any nucleotide A, G, U or C and complementary to N at position 48"

<220>
<221> misc_feature
<222> 3
<223> /note="N=any nucleotide A, G, U or C and complementary to N at position 47"

<220>
<221> misc_feature
<222> 4
<223> /note="N=any nucleotide A, G, U or C and complementary to N at position 46"

<220>
<221> misc_feature
<222> 5
<223> /note="N=any nucleotide A, G, U or C and complementary to N at position 45"

<220>
<221> misc_feature
<222> 6
<223> /note="N=any nucleotide A, G, U or C and complementary to N at position 44"

<220>
<221> misc_feature
<222> 44
<223> /note="N=any nucleotide A, G, U or C and complementary to N at position 6"

<220>
<221> misc_feature
<222> 45
<223> /note="N=any nucleotide A, G, U or C and complementary to N at position 5"

<220>
<221> misc_feature
<222> 46
<223> /note="N=any nucleotide A, G, U or C and complementary to N at position 4"

<220>
<221> misc_feature
<222> 47
<223> /note="N=any nucleotide A, G, U or C and complementary to N at position 3"

<220>
<221> misc_feature
<222> 48
<223> /note="N=any nucleotide A, G, U or C and complementary to N at position 2"

<220>
<221> misc_feature
<222> 49
<223> /note="N=any nucleotide A, G, U or C and complementary to N at position 1"

<220>
<221> misc_feature
<222> 50
<223> /note="N=any nucleotide A, G, U or C"

<220>
<221> misc_feature
<222> 51
<223> /note="N=any nucleotide A, G, U or C"

<220>
<221> misc_feature
<222> 52
<223> /note="N=any nucleotide A, G, U or C"

<220>
<221> misc_feature
<222> 53
<223> /note="N=any nucleotide A, G, U or C"

<220>
<221> misc_feature
<222> 54
<223> /note="N=any nucleotide A, G, U or C"

<220>
<221> misc_feature
<222> 55
<223> /note="N=any nucleotide A, G, U or C"

<220>
<221> misc_feature
<222> 56
<223> /note="N=any nucleotide A, G, U or C"

<220>
<221> misc_feature
<222> 57
<223> /note="N=any nucleotide A, G, U or C"

<220>
<221> misc_feature
<222> 58
<223> /note="N=any nucleotide A, G, U or C"

<220>
<221> misc_feature
<222> 59
<223> /note="N=any nucleotide A, G, U or C"

<220>
<221> misc_feature
<222> 60
<223> /note="N=any nucleotide A, G, U or C"

<220>
<221> misc_feature
<222> 61
<223> /note="N=any nucleotide A, G, U or C"

<220>
<221> misc_feature
<222> 62
<223> /note="N=any nucleotide A, G, U or C"

<220>
<221> misc_feature
<222> 63
<223> /note="N=any nucleotide A, G, U or C"

<400> 24
nnnnnncuga ugaguccgug aggacgaaac gaguaagcuc gucnnnnnnn nnnnnnnnnn 60

nnnguuuuag agcuagaaau agcaaguuaa aauaaggcua guccguuauc aacuugaaaa 120

aguggcaccg agucggugcu uuuggccggc augguc ccag ccuccucgcu ggcgccggcu 180

gggcaacaug cuucggcaug gcgaauggga cagcuuugga c 221

<210> 25
<211> 178
<212> RNA
<213> Artificial Sequence

<220>
<223> gRNA

<220>
<221> misc_feature
<222> 1
<223> /note="N=any nucleotide A, G, U or C and complementary to N at position 44 in SEQ ID 24"

<220>
<221> misc_feature
<222> 2
<223> /note="N=any nucleotide A, G, U or C and complementary to N at position 45 in SEQ ID 24"

<220>
<221> misc_feature

<222> 3
<223> /note="N=any nucleotide A, G, U or C and complementary to N at position 46 in SEQ ID 24"

<220>
<221> misc_feature
<222> 4
<223> /note="N=any nucleotide A, G, U or C and complementary to N at position 47 in SEQ ID 24"

<220>
<221> misc_feature
<222> 5
<223> /note="N=any nucleotide A, G, U or C and complementary to N at position 48 in SEQ ID 24"

<220>
<221> misc_feature
<222> 6
<223> /note="N=any nucleotide A, G, U or C and complementary to N at position 49 in SEQ ID 24"

<220>
<221> misc_feature
<222> 7
<223> /note="N=any nucleotide A, G, U or C and complementary to N at position 50 in SEQ ID 24"

<220>
<221> misc_feature
<222> 8
<223> /note="N=any nucleotide A, G, U or C and complementary to N at position 51 in SEQ ID 24"

<220>
<221> misc_feature
<222> 9
<223> /note="N=any nucleotide A, G, U or C and complementary to N at position 52 in SEQ ID 24"

<220>
<221> misc_feature
<222> 10
<223> /note="N=any nucleotide A, G, U or C and complementary to N at position 53 in SEQ ID 24"

<220>
<221> misc_feature
<222> 11
<223> /note="N=any nucleotide A, G, U or C and complementary to N at position 54 in SEQ ID 24"

<220>
<221> misc_feature
<222> 12
<223> /note="N=any nucleotide A, G, U or C and complementary to N at position 55 in SEQ ID 24"

<220>
<221> misc_feature
<222> 13
<223> /note="N=any nucleotide A, G, U or C and complementary to N at position 56 in SEQ ID 24"

```
<220>
<221> misc_feature
<222> 14
<223> /note="N=any nucleotide A, G, U or C and complementary to N at
      position 57 in SEQ ID 24"

<220>
<221> misc_feature
<222> 15
<223> /note="N=any nucleotide A, G, U or C and complementary to N at
      position 58 in SEQ ID 24"

<220>
<221> misc_feature
<222> 16
<223> /note="N=any nucleotide A, G, U or C and complementary to N at
      position 59 in SEQ ID 24"

<220>
<221> misc_feature
<222> 17
<223> /note="N=any nucleotide A, G, U or C and complementary to N at
      position 60 in SEQ ID 24"

<220>
<221> misc_feature
<222> 18
<223> /note="N=any nucleotide A, G, U or C and complementary to N at
      position 61 in SEQ ID 24"

<220>
<221> misc_feature
<222> 19
<223> /note="N=any nucleotide A, G, U or C and complementary to N at
      position 62 in SEQ ID 24"

<220>
<221> misc_feature
<222> 20
<223> /note="N=any nucleotide A, G, U or C and complementary to N at
      position 63 in SEQ ID 24"

<400> 25
nnnnnnnnnn nnnnnnnnnn guuuuagagc uagaaauagc aaguuaaaau aaggcuaguc     60

cguuaucaac uugaaaaagu ggcaccgagu cggugcuuuu ggccggcaug gucccagccu    120

ccucgcuggc gccggcuggg caacaugcuu cggcauggcg aaugggacag cuuuggac      178
```

**Description**

FIELD OF THE INVENTION

The present application refers to a method using the CRISPR/Cas system in a temperature controllable manner, in particular a method of producing a mutant host cell with altered expression of a gene of interest (GOI) by a temperature-inducible CRISPR/Cas system, and a host cell transformed with the temperature-inducible CRISPR/Cas system.

BACKGROUND

In order to control cellular functions it is necessary to have suitable tools available which react on external stimuli or signals. Such signals can be of chemical origin like e.g. galactose, glucose, insulin or physical origin like light or temperature. The advantage of physical signals is that they can easily be turned on and off in a biological process, whereas this is more difficult for chemical signals e.g. to remove a chemical substance from a solution.

One parameter which can conveniently be changed in a biological system is temperature. The suitable temperature range for biological systems is limited depending on the optimal growth temperature, which is for mesophilic organisms typically in the range between 20°C and 45°C. Temperature controllable elements are available for this narrow temperature range.

A RNA thermometer called thermozyme which is based on a hammerhead ribozyme (HHR, or HH ribozyme) that cleaves itself to generate a liberated ribosome binding site, thereby permitting expression of a downstream gene, is described by Saragliadis et al. (RNA Biology 2013, 106: 1009-1016). A thermosensitive fourU hairpin was attached to the ribozyme in place of stem III of a parental HHR derived from *Schistosoma mansoni* (SC-HHR), to modulate gene expression by temperature-controlled base pairing and melting.

CRISPR/Cas9 (clustered regularly interspaced short palindromic repeat) is an efficient gene editing tool, which can be used in a plethora of different organisms to generate double -strand breaks subsequently introducing mutations at this locus in a targeted manner (Jinek M et al. 2012. Science 337: 816-21). Two main components are needed for the gene editing process: an endonuclease like Cas9 from *Streptococcus pyogenes* and a guide RNA (gRNA), containing the target specific sequence. Other endonucleases like CPF1 is a Cas enzyme which can be used instead of Cas9 (System CC et al. 2015 Cell 163: 1-13.).

The CRISPR/Cas technology has been used e.g. to engineer gene knockouts in mammalian cells (Ran F Ann et al. Nature Protocols 2013, 8(11):2281-2308).

WO2015195621A1 describes the HI promoter for use in the expression of a CRISPR guide RNA with altered specificity of the 5' nucleotide, as well as use of the HI promoter sequence as a bidirectional promoter to express Cas9 nuclease and the gRNA simultaneously. Compositions and methods are also described for the expression and regulation of gRNA expression *in vivo* through the use of RNA ribozymes and regulatable aptazymes. In particular an aptamer-regulated ribozyme is described which comprises a cis-acting hammerhead ribozyme comprising a ligand binding aptamer; and a nucleotide sequence encoding the guide RNA, wherein the 5' end of the nucleotide sequence is directly coupled to the hammerhead ribozyme, wherein binding of the ligand to the aptamer produces a conformational change in the ribozyme such that the ribozyme undergoes self-cleavage between the 5' end of the nucleotide sequence and the ribozyme, wherein the gRNA is released. One particular ligand inducing the conformational change is theophylline.

WO2015153940A1 describes an engineered construct comprising a promoter operably linked to a nucleic acid that comprises a guide RNA which may be flanked by nucleotide sequences encoding ribozymes, e.g. a hammerhead ribozyme or a Hepatitis delta virus ribozyme.

One challenge to generate a functional system is to have a functional guide RNA, which has a defined 5' and 3' end. This problem was solved with the introduction of self-splicing ribozymes, which are fused to both ends of the DNA template to enable a self-splicing of the transcribed RNA. (Gao Y et al. 2014. J. Integr. Plant Biol. 56: 343-9). Any Polymerase II promoter can be used to transcribe this DNA to generate a fully functional guide RNA, which will be recognized by the Cas9 endonuclease.

The creation of conditional gene deletions is challenging and is currently based on Cre/lox systems (Arakawa H et al. 2001. BMC Biotechnol. 1: 7). Such systems can be chemically induced by e.g. tetracycline.

SUMMARY OF THE INVENTION

It is the object of the present invention to provide for an improved method of producing a host cell line with mutations at a predefined locus using the CRISPR/Cas9 system in a controlled manner, in particular a method of producing an inducible mutation of interest (MOI).

The object is solved by the subject matter as claimed.

According to the invention, there is provided a method of producing a mutant host cell with altered expression of a gene of interest (GOI) by a temperature-inducible CRISPR/Cas system, which comprises:

a) at a first working temperature, introducing into a host cell a CRISPR/Cas system employing

i) an inactive pre-guide RNA (pre-gRNA) which is a ribozyme-extended guide RNA comprising a guide RNA (gRNA) that recognizes a predetermined target site at the GOI, terminally extended by at least one self-processing Hammerhead (HH) ribozyme characterized by the presence of a thermosensitive hairpin and a cleavage site adjacent to the gRNA; and

ii) a RNA-guided Cas endonuclease which recognizes the target site upon hybridizing with the gRNA;

b) increasing the first working temperature to a second working temperature, thereby changing the structure of the thermosensitive hairpin and activating the pre-gRNA by self-catalyzed cleavage at the cleavage site to obtain the gRNA, which induces binding of the endonuclease to the target site, thereby altering expression of the GOI.

As described herein, the gRNA is specifically hybridizing with the genomic target site under stringent conditions and consequently the Cas endonuclease recognizes the target site immediately inducing the DNA break and MOI. Therefore, the present invention in particular provides the temperature-induced gRNA which triggers the MOI at the target site, by *in situ* activating the respective pre-gRNA.

Specifically, the DNA break is proximal to a protospacer adjacent motif (PAM) sequence. Specifically, the DNA break is a double strand break or a paired single strand break, proximal to a PAM, preferably 3 bp upstream of the PAM. Exemplary PAM sequences are selected from the group consisting of sequence: NGG, sequence: NGAN, sequence: NGNG, sequence: NGAG, sequence: NGCG or sequence: TTN, wherein N is any nucleotide (A, C, G, or U), or a complementary sequence of any of the foregoing. The paired single strand break is herein also referred to as a specific embodiment of a "double strand" break. The paired nicking (single strand break) is specifically proximal to two PAMs, one PAM for each single strand break.

The complementary DNA sequences are typically recognized for the DNA break of the complementary strand. It is preferred that a suitable PAM sequence is selected which is recognized by the specific endonuclease and the specific CRISPR system.

Specifically, the MOI results from a mutational pattern characteristic for a RNA-guided endonuclease that lies proximal to a PAM. Such mutation(s) are typically located within 20 bp upstream and downstream of the DNA break, specifically within 10bp or 15 bp upstream and downstream of the DNA break.

Unless specified herein otherwise, the term "at" with respect to a genetic location, such as the location of a genetic target site, or the location of a MOI "at the GOI" or a MOI "at the genetic target site", shall always refer to a position within the genetic site or in close proximity to such genetic target site, meaning within about 20 bp, or 10 bp, or 5 bp upstream or downstream the genetic site.

Specifically, the GOI is in close proximity to the target site. Specifically, the MOI is within or in close proximity to the GOI.

According to a specific embodiment, the endonuclease catalyzes a DNA break at the target site (e.g., within the hybridizing region or in close proximity thereto) upon hybridizing with the gRNA and the GOI expression is altered by cellular repair mechanisms induced by a DNA break, thereby introducing a mutation of interest (MOI) at the GOI (e.g., within the GOI or within a regulatory element operably linked to the GOI and regulating the expression of the GOI), preferably a MOI comprising at least one frameshift mutation, insertion, substitution, and/or deletion of one or more nucleotides impairing the open reading frame (ORF) of the GOI. Specifically, the MOI includes one or more point mutations, or mutation of DNA elements or sequences that disrupt gene function or gene expression, e.g. an insertion of foreign DNA elements or sequences that are non-naturally present (knock-in) or a deletion of sequences that are naturally present (knock-out), e.g. deletions of entire genes, exons or regulatory elements.

Specifically, in order to delete a genomic region that is naturally present, a double strand DNA break may be induced as described herein, employing two gRNA molecules hybridizing on both sides lateral to the genomic (chromosomal) region to be excised, e.g. proximal or adjacent to the 5' and 3' end of the genomic region. Upon such DNA break the cellular repair would provide for the joining of the free ends, thereby excising the genomic region.

Specifically, the endonuclease is an inactive Cas9 (dCas9 or endonuclease deficient Cas9) endonuclease which binds to the target site upon hybridizing with the gRNA, thereby repressing the GOI expression. An exemplary inactive, inhibitory Cas9 endonuclease is characterized by mutations in the RuvC and HNH-nuclease domain (e.g. point mutations D10A and H840A or R70A in SpCas9 Uniprot: Q99ZW2) SEQ ID 23.

According to a further specific aspect, at least two different target sites are targeted by different crRNAs or gRNAs, employing the same or different functional pairs of tracrRNA and endonuclease or functional pairs of the constant part of gRNA and endonuclease.

According to a specific aspect, at least a first and a second pre-gRNA are used recognizing at least a first and a different second target site at the GOI, wherein said at least first and second pre-gRNAs are activated by increasing the first working temperature to the second working temperature.

Specifically, the one or more target sites are within or in close proximity to the same GOI.

Specifically, the HH ribozyme has thermo-inducible nuclease activity, such that primary transcripts of the pre-gRNA undergo self-catalyzed cleavage to precisely release the gRNA. Specifically, the HH ribozyme is directly linked to the variable region of the gRNA recognizing the predetermined target site, without linking nucleobases. Thus, the use of additional transcription initiating nucleobases such as "G" or "A" at the 5'-terminus of the gRNA can be avoided.

Specifically, the HH ribozyme comprises a 5'-terminal nucleotide sequence complementary to and hybridizing with the 5'-terminal part of the gRNA, e.g. forming a region of intrastrand pairing of at least 4, 5, 6, 7, or 8 bp, thereby masking the variable region of the gRNA. Upon cleavage of the HH ribozyme, the 5'-terminal part of the gRNA is released, and capable of hybridizing to the genomic target site.

Specifically, the hairpin consists of a nucleic acid region with intrastrand complementarity and pairing, which is herein referred to as "stem" region, preferably comprising 3-12 pairs of consecutive complementary bases in reverse sequence, , and a variable loop region of 4-10 nt length which is herein referred to as "loop" region, and which is located between the regions of internal complementarity. Specifically, a pair of complementary bases is any of a G-C or A-U base pair.

According to a specific aspect, the HH ribozyme comprises

a) a HH ribozyme constant region;
b) a variable 3'-terminal nucleotide sequence of at least 3 nucleotides; and
c) a complementary 5'-terminal nucleotide sequence hybridizing to the 3'-terminal nucleotide sequence, thereby obtaining a thermosensitive stem I of at least 3 complementary bp.

According to the invention, there is further provided a thermosensitive HH ribozyme, which comprises or consists of

a) a HH ribozyme constant region, preferably consisting of the nt sequence identified as SEQ ID 5; which is terminally extended by
b) at least a variable 3'-terminal nucleotide sequence of at least 3 nucleotides; and
c) a complementary 5'-terminal nucleotide sequence hybridizing to the 3'-terminal nucleotide sequence, thereby obtaining a thermosensitive stem I,

wherein the stem I is characterized by at least any of

i. the hybridizing part of the variable 3'-terminal nucleotide sequence of b) consists of at least 6 nucleotides and comprises at least four G or C residues;
ii. the hybridizing part of the variable 3'-terminal nucleotide sequence of b) comprises or consists of at least 6 nucleotides having the sequence NNNCUC, wherein N is any of A, G, C, or U; or
iii. a length of at least 7 bp, or less than 6 bp.

According to the invention, there is further provided a pre-gRNA, which comprises the thermosensitive HH ribozyme. Specifcally, the novel pre-gRNA comprises

a) a HH ribozyme constant region; which is terminally extended by
b) at least a variable 3'-terminal nucleotide sequence of at least 3 nucleotides; and
c) a complementary 5'-terminal nucleotide sequence hybridizing to the 3'-terminal nucleotide sequence, thereby obtaining a thermosensitive stem I,

wherein the stem I is characterized by at least any of

i. the hybridizing part of the variable 3'-terminal nucleotide sequence of b) consists of at least 6 nucleotides and comprises at least four G or C residues;
ii. the hybridizing part of the variable 3'-terminal nucleotide sequence of b) comprises or consists of at least 6 nucleotides having the sequence NNNCUC, wherein N is any of A, G, C, or U; or
iii. a length of at least 7 bp, or less than 6 bp.

Specifically, the thermosensitive stem I consists of the 3'-terminal nucleotide sequence and the complementary 5'-terminal nucleotide sequence hybridizing to each other at the first working temperature, or under stringent conditions.

Specifically, the thermosensitive stem is characterized by the 3'-terminal nucleotide sequence and the complementary 5'-terminal nucleotide sequence hybridizing to each other at the first working temperature, and not hybridizing (herin also referred to as "melting") to each other at the second working temperature.

Specifically, the stem I is longer than 6 bp, such that the melting temperature is at least 20°C.

Specifically, the stem I hybridizing part consists of at least 7 bp, to increase thermostability, preferably at least 7 bp, or at least 8 bp, or at least 9 bp, or at least 10 bp, e.g. up to 12 bp or 11 bp

Specifically, the stem I hybridizing part consists of at least 3 bp up to 5 bp, to reduce thermostability, specifically up to 4 bp, or 3 bp.

Specifically, the HH ribozyme constant region of component a) comprises or consists of the RNA identified by SEQ ID 5.

Specifically, the complementary sequence of component c) comprises at least 3, 4, 5, 6, 7, 8, 9, 10, 11, or 12 nt which hybridize to the variable 3-terminal nucleotide sequence, thereby obtaining a stem I of at least 3 bp, or at least 4, 5, 6, 7, 8, 9, 10, 11, or 12 bp.

Specifically, the component b) comprises the hybridizing variable nucleotide sequence which is further 3'-terminally extended by a non-hybridizing variable sequence of one or more nucleotides, preferably at least 8, 9, 10, 11, 12, 13, 14, 15, 16, or 17 nt.

Specifically, the variable nucleotide sequence of component b) including the hybridizing and non-hybridizing part has a length of 15-25 nt, preferably at least 15, 16, 17, 18, 19, or 20; and up to 25, 24, 23, 22, 21, or 20. Specifically, the variable nucleotide sequence of component b) has a length of 20 nt, which can be specifically designed as a nucleotide sequence complementary to the target site.

Specifically, any of the variable sequences is characterized by a sequence of one or more consecutive nucleotides, wherein each of the nt is any of A, C, G, or U.

Specifically, the complementary sequences of component b) and c) hybridize to each within the host cell, herein also referred to as *"in vivo* stringent conditions".

The pre-gRNA may comprise the HH ribozyme linked to one or both terminal positions of the gRNA. Specifically, the pre-gRNA comprising two HH ribozymes characterized by the gRNA flanked by two HH ribozymes, which may comprise the same or different hairpins. For example, the pre-gRNA may comprise a first HH ribozyme comprising a first thermosensitive hairpin structure which differs from the thermosensitive hairpin structure of a second HH ribozyme, e.g. wherein the difference is in the length of the region of intrastrand pairing. As a consequence, the first and second HH ribozymes are cleaved from the pre-gRNA at different working temperatures.

Specifically, the 3'-terminal nucleotide sequence of the HH ribozyme forms the 5'-terminus of the gRNA upon cleaving the pre-gRNA; and the 5'-terminal nucleotide sequence of the HH ribozyme is hybridizing thereto.

Specifically, the HH ribozyme constant region (herein also referred to as "backbone") comprises a nucleotide sequence identified as SEQ ID 5, preferably any of the nucleotide sequences identified as SEQ ID 1, SEQ ID 2, SEQ ID 4, or SEQ ID 5, or a functional variant of any of the foregoing with at least 70 % sequence identity, or at least 80%, or at least 90%, or at least 95% sequence identity.

According to a specific aspect, the pre-gRNA comprises any of the nucleotide sequences identified as SEQ ID 6, SEQ ID 7, SEQ ID 8, or SEQ ID 9, or a functional variant of any of the foregoing with at least 70% sequence identity, or at least 80%, or at least 90%, or at least 95% sequence identity.

In addition to the HH ribozyme terminus, the pre-gRNA may further comprise another defined ribozyme terminus opposite to the HH ribozyme terminus.

Specifically, the pre-gRNA further comprises a hepatitis delta virus type (HDV) ribozyme as a further terminal extension of the gRNA sequence opposite to the extension by the HH ribozyme. Such pre-gRNA specifically comprises a first ribozyme terminus of a HH ribozyme, and a second ribozyme terminus of a HDV ribozyme, specifically wherein the first terminal part consists of the HH ribozyme, and the second terminal part consists of the HDV ribozyme.

Specifically, the HDV ribozyme comprises the nucleotide sequence identified as SEQ ID 10, or a functional variant thereof with at least 70 % sequence identity, or at least 80%, or at least 90%, or at least 95% sequence identity.

According to a specific embodiment, the first working temperature is any below 30 °C, and the second working temperature is any above 30°C.

More specifically, the first working temperature is below 30 °C and at least 4°C, or at least 10°C, or at least 15°C or at least 20°C, or at least room temperature.

More specifically, the second working temperature it above 30°C and less than 45°C, preferably less than 40°C, preferably less than 35°C.

According to a specific aspect, the host cell is cultivated in a fed-batch culture, wherein the host cell is first cultivated at the first working temperature during the growth phase, followed by increasing the first working temperature to the second working temperature thereby activating the pre-gRNA and altering the expression of the GOI in the production phase.

Specifically, the host cell is capable of cellular repair mechanism induced by DNA break, e.g. non-homologous end joining or homology-directed repair, or other mechanisms, such as including base-excision repair, mismatch repair or single strand annealing.

Specifically, the host cell is any of a bacterial, filamentous fungi, yeast, mammalian, or avian host cell. Specifically preferred is any eukaryotic cell suitable for recombinant expression of a POI.

More specifically, the bacterial host cell is of a genus selected from the group consisting of genus Escherichia (e.g. *Escherichia coli*), genus *Lactobacillus* (e.g. *Lactobacillus plantarum), Bacillus (e.g. Bacillus subtilis),* preferably any of the species *Escherichia coli* MG1655, *Escherichia coli* Crooks, *Escherichia coli* W, *Escherichia coli* K12.

More specifically, the filamentous fungi host cell is of a genus selected from the group consisting of genus *Aspergillus* (e.g. *Aspergillus niger, Aspergillus terreus, Aspergillus nidulans), Trichoderma* (e.g. *Trichoderma reesei),* Neurospora (e.g. *Neurospora crassa), Ustilago (Ustilago maydis), Fusarium (e.g. Fusarium graminearum),* preferably any of the species *Aspergillus niger* ATCC1015, *Aspergillus niger CBS* 513.88, *Trichoderma reesei* QM6a, Rut C-30.

More specifically, the yeast host cell is of a genus selected from the group consisting of Saccharomyces (e.g. *Saccharomyces cerevisiae),* Candida *(e.g. Candida albicans, Candida lignohabitans* [syn. *Sugiyamaella lignohabitans*]), *Yarrowia* (*e.g. Yarrowia lipolytica), Schizosaccharomyces (e.g. Schizosaccharomyces pombe),* preferably any of the species *Pichia pastoris* CBS 7435, DSMZ 70382, GS115, CBS 2612, *Candida lignohabitans* CBS 10342. Examples of preferred yeast cells used as host cells as described herein include but are not limited to, the *Saccharomyces* genus (e.g. *Saccharomyces cerevisiae*), the *Pichia* genus (e.g. *P. pastoris, or P. methanolica*), the *Komagataella* genus (*K. pastoris, K. pseudopastoris* or *K. phaffii*), the *Sugiyamaella genus* (e.g. S. *lignohabitans), Hansenula polymorpha, Yarrowia lipolytica, Schefferomyces stipitis* or *Kluyveromyces lactis.*

More specifically, the mammalian host cell is any of the species human, monkey, hamster, mouse, or swine. Examples of preferred mammalian cells are BHK, CHO (CHO-DG44, CHO-DUXB11, CHO-DUKX, CHO-K1, CHOK1 SV, CHO-S), HeLa, HEK293, MDCK, NIH3T3, NS0, PER.C6, SP2/0 and VERO cells.

More specifically, the avian host cell is any of the species chicken, or duck.

Specifically, the host cell may be provided in an *in vitro* or *ex vivo* host cell culture.

According to a specific embodiment, the host cell may be provided in a transgenic animal, e.g. a mammalian animal, yet, excluding human beings.

According to a specific aspect, the host cell is engineered to express the Cas9 endonuclease and/or the gRNA, or one or more components of the gRNA. Specifically, a host cell line is engineered to express both, the Cas9 enzyme and the gRNA, or at least one component of the gRNA.

According to a specific embodiment, the CRISPR/Cas system is introduced into the host cell by one or more plasmids transforming the cell into a cell producing the pre-gRNA and the endonuclease. The plasmid may comprise a nucleic acid encoding the gRNA or one or more of its componets operably linked to regulatory elements, suitable for the expression of the gRNA or one or more of its components by the cells.

The guide RNA may be provided as a binary complex of its components, the target specific crRNA (CRISPR RNA, specifically recognizing the target site) and the tracrRNA (trans-activating crRNA), and optional further linker sequences, each provided as separate components that associate *ex vivo* or within a cell. Alternatively, tracrRNA and crRNA may also be provided as separate components. Preferably, the guide RNA is provided as a chimeric or recombination product which comprises the components tracrRNA and crRNA linked to each other, e.g. by a linkage where the crRNA is linked to the 5' end of the tracrRNA directly, with or without a linker sequence. The crRNA typically comprises a constant part, which is the 3' part that provides for the association or linkage with the tracrRNA. The crRNA further comprises a variable part, designed to hybridize with a specific target site, which is typically incorporated in the 5' part or 5' end of the crRNA and gRNA, respectively.

According to a specific aspect, a component consisting of the RNA-guided endonuclease in conjunction with the tracrRNA may be used. Such component is preferably used in combination with the target-specific RNA (crRNA).

The gRNA and the RNA-guided endonuclease may be conveniently provided as a ternary complex of the endonuclease with the tracrRNA and the crRNA, each provided as separate components that associate *ex vivo* or within a cell. Preferably, there is provided a binary complex of the endonuclease with the gRNA, each provided as separate components that associate *ex vivo* or within a cell. In such complex with the endonuclease, the gRNA preferably comprises the tracrRNA and the crRNA linked to each other, thus is a chimeric RNA product.

Preferably functional pairs of tracrRNA or gRNA paired with an RNA-guided endonuclease are used, e.g. a functional pair of the constant part of the gRNA and the endonuclease, specifically functional pairs in a complex or as separate components. Specifically, the functional pairs are of a suitable type II CRISPR systems, such as a CRISPR system of bacterial origin.

Functional pairs of the tracrRNA or gRNA and the matching endonuclease are preferably used with one or more different crRNA components, e.g. with a series of crRNA oligonucleotides that target different genomic target sites.

According to a specific aspect, the endonuclease is selected from the group consisting of Cas9 enzymes originating from any of *Streptococcus pyogenes, Streptococcus thermophiles, Neisseria Meningitis* or *Treponema Denticola,* or the Cpf1 enzyme originating from *Acidaminococcus sp.* or *Lachnospiraceae bacterium,* and functional variants of any of the foregoing, including Cas9 nickases or artificial enzymes.

Preferred Cas9 enzymes comprise or consist of a polypeptide identified by any of SEQ ID 19, SEQ ID 20, SEQ ID 21, SEQ ID 22, or a functional variant thereof with at least 70% sequence identity, or at least 80%, or at least 90%, or at least 95% sequence identity.

Functional variants of a parent Cas enzyme may e.g. be analogs, such as wild-type sequences obtained from other species, e.g. other bacterial species of the same genus or family as the parent endonuclease, or mutated wild-type sequences of analogs. When an analog of the endonuclease is used, specifically the analogous tracrRNA or gRNA sequence of the same species or the same family may be used to form a functional pair, e.g. which components are natively paired.

According to a specific aspect, the GOI is selected from the group consisting of genes encoding regulatory proteins of the host cell, preferably metabolic enzymes, or proteins involved in cell cycle arrest, preferably any of the regulatory proteins listed in Table 1, or analogues thereof naturally-occurring in the host cell.

The invention further provides for a method of producing a protein of interest (POI) in a host cell transformed with the temperature-inducible CRISPR/Cas system as described herein, wherein the host cell comprises a gene encoding the POI regulated by the GOI expression product, comprising

a) growing the host cell under growth conditions at the first working temperature thereby obtaining a cell culture;
b) elevating said first working temperature in the cell culture to the second working temperature, thereby altering expression of the GOI and initiating the production of the POI; and
c) isolating the POI from the cell culture.

Specifically, the POI is a heterologous protein, preferably selected from therapeutic proteins, including antibodies or fragments thereof, enzymes and peptides, protein antibiotics, toxin fusion proteins, carbohydrate - protein conjugates, structural proteins, regulatory proteins, vaccines and vaccine like proteins or particles, process enzymes, growth factors, hormones and cytokines, or a metabolite of a POI.

Preferably a genetically stable host cell is used, which comprises the same type of MOI or the stable phenotype characterized by the altered expression of the GOI over at least 10 generations, or at least 20 generations.

Specifically, the host cell is provided in a stable host cell culture.

The invention further provides for a host cell transformed with the temperature-inducible CRISPR/Cas system as described herein, wherein the host cell comprises a gene encoding a heterologous POI regulated by the GOI expression product.

## FIGURES

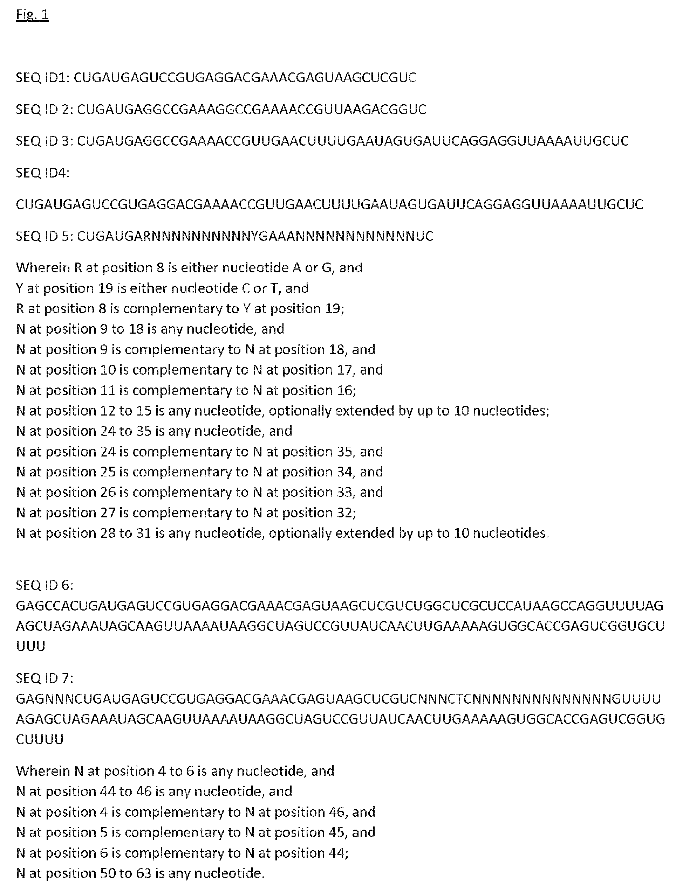

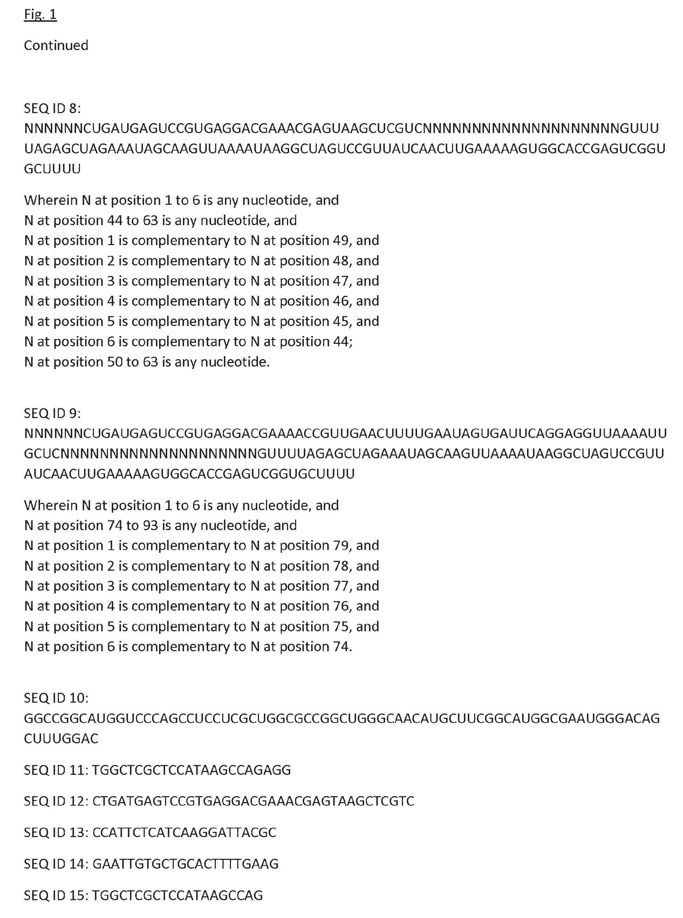

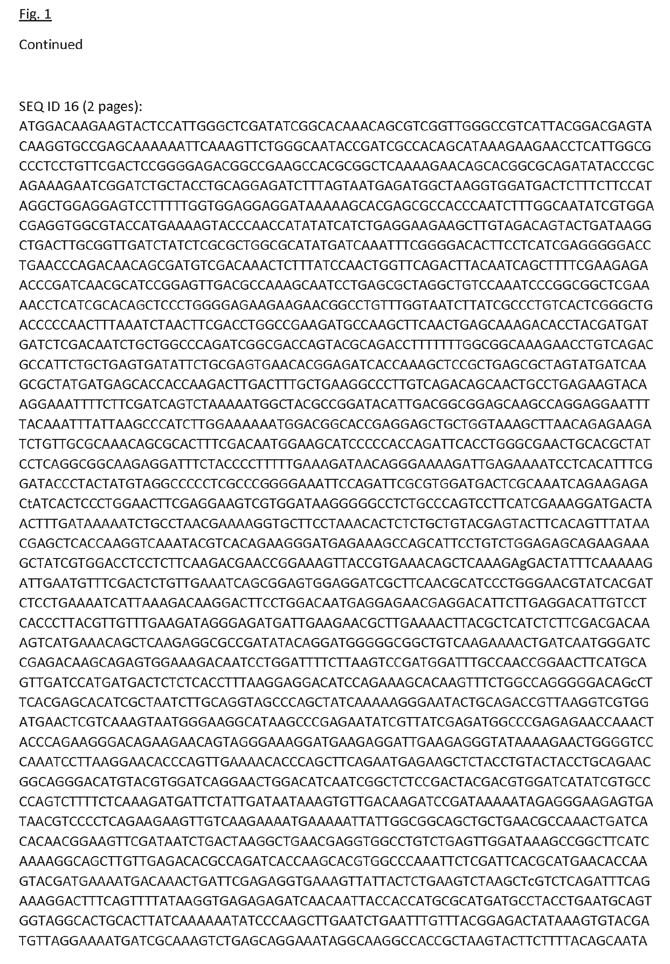

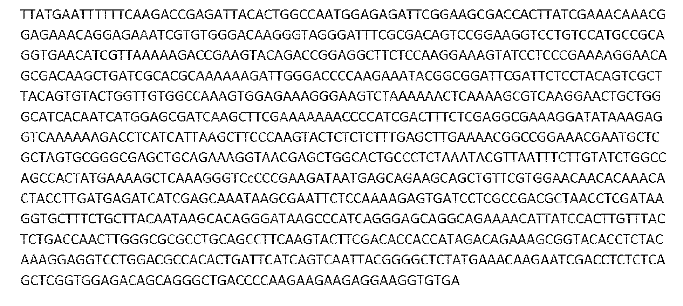

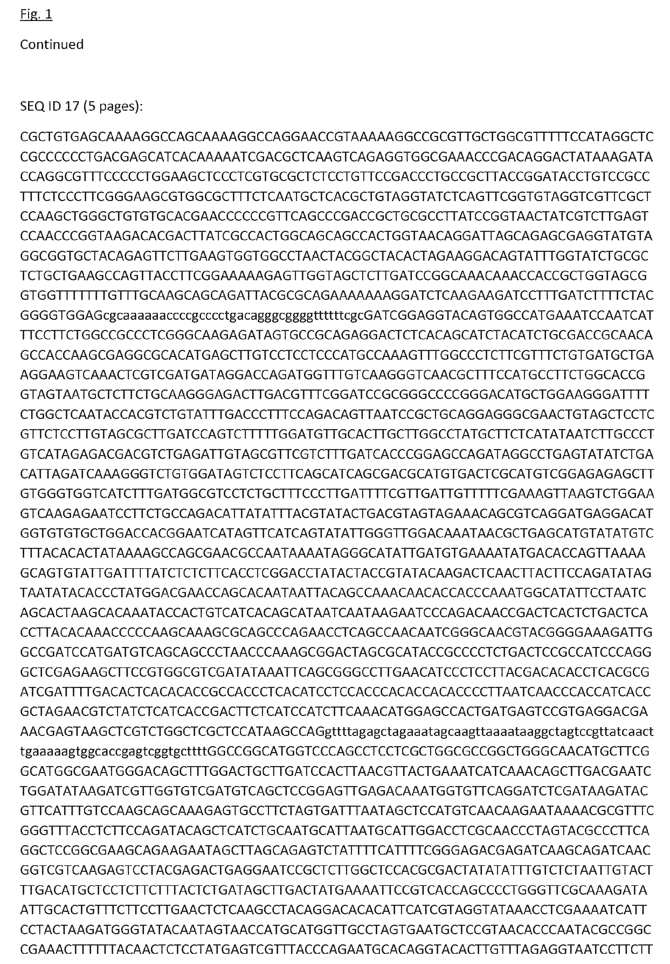

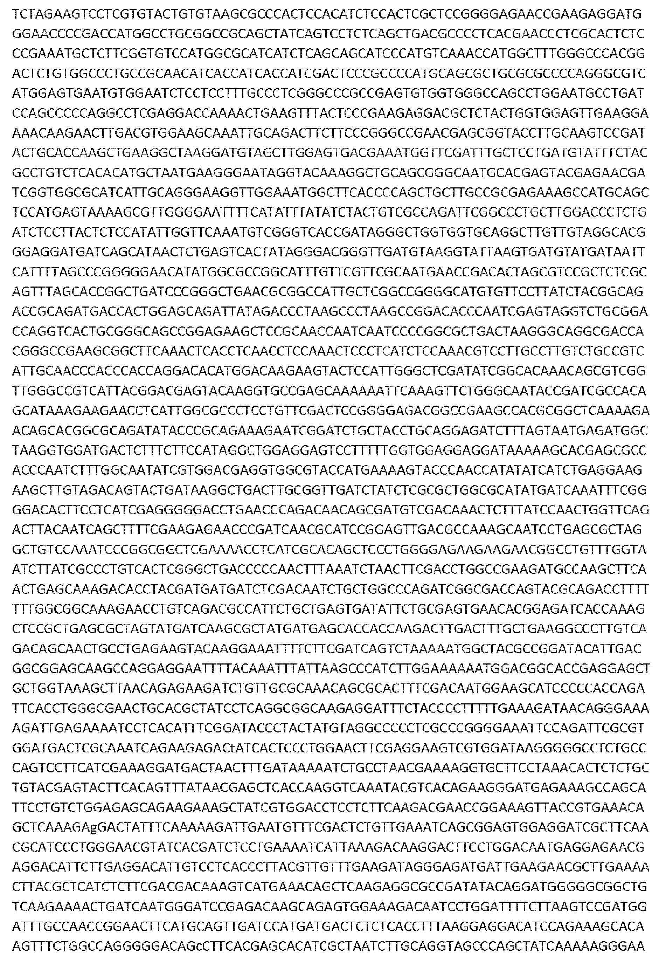

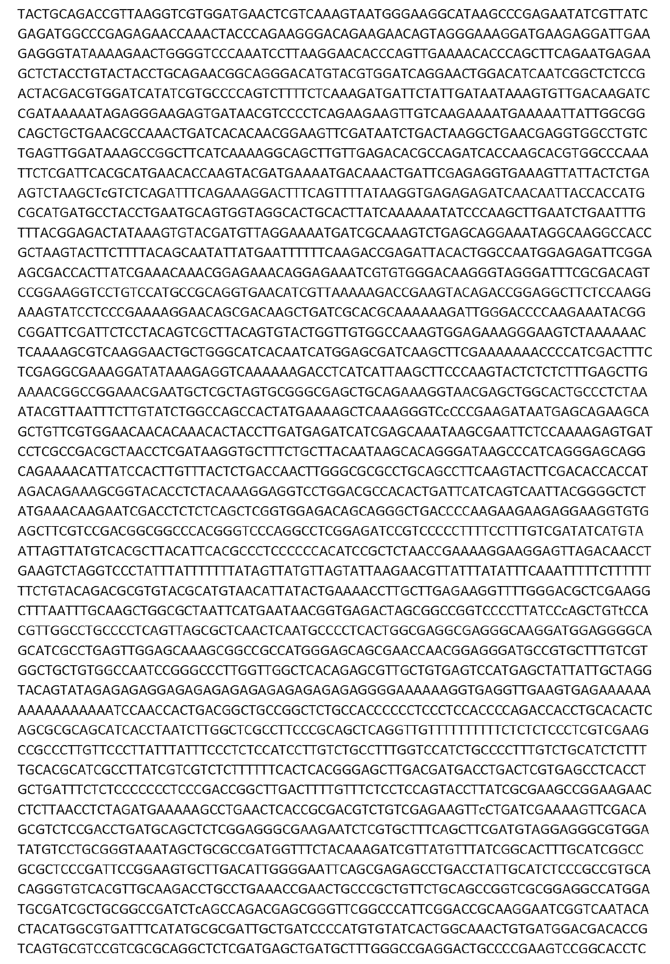

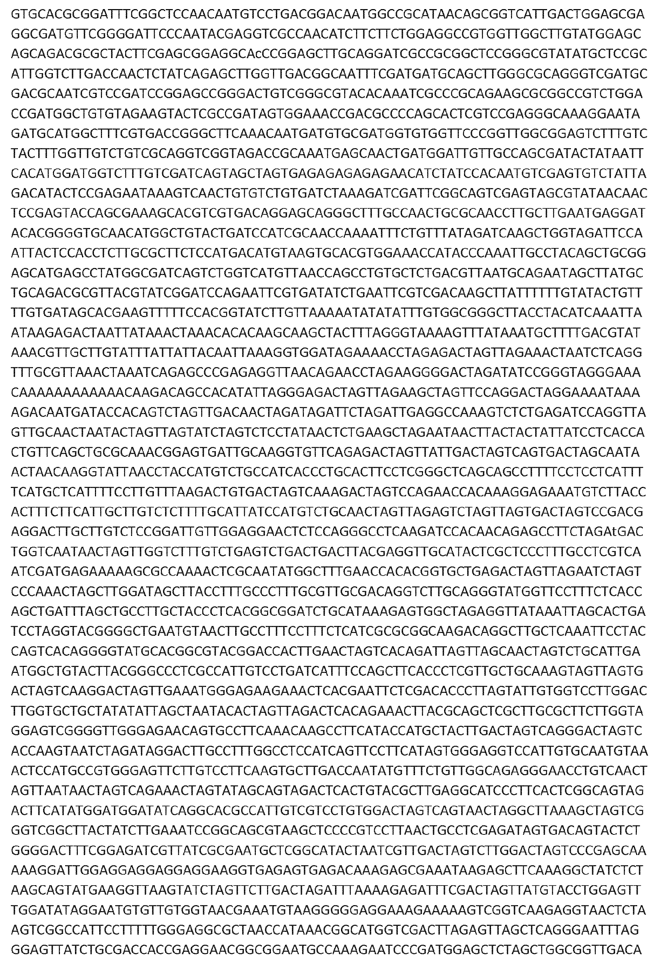

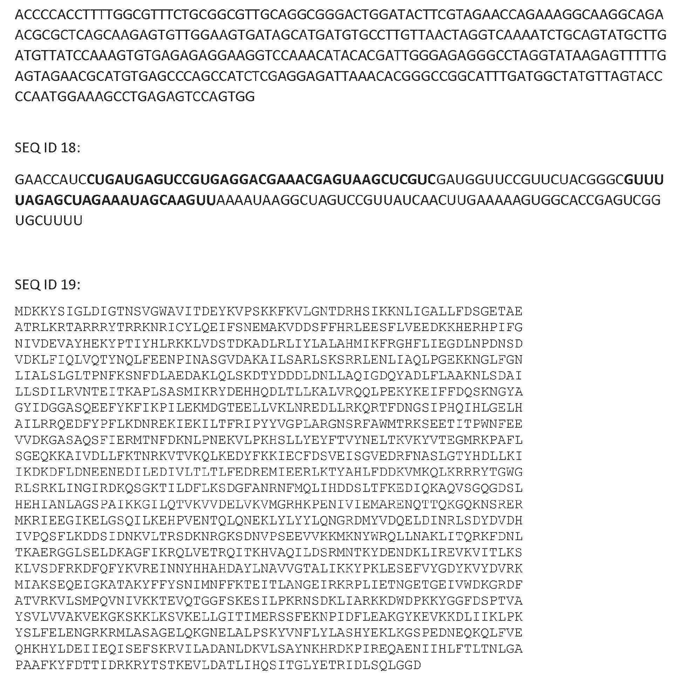

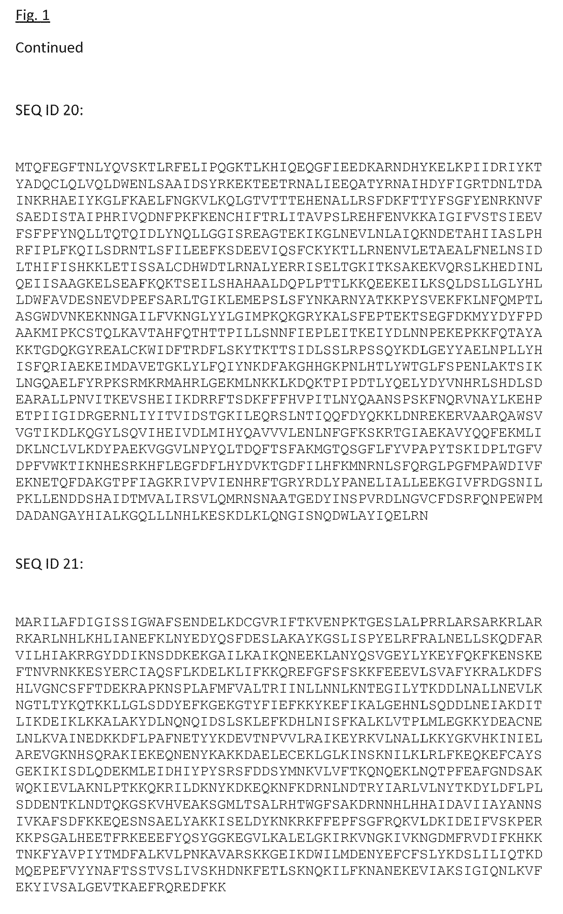

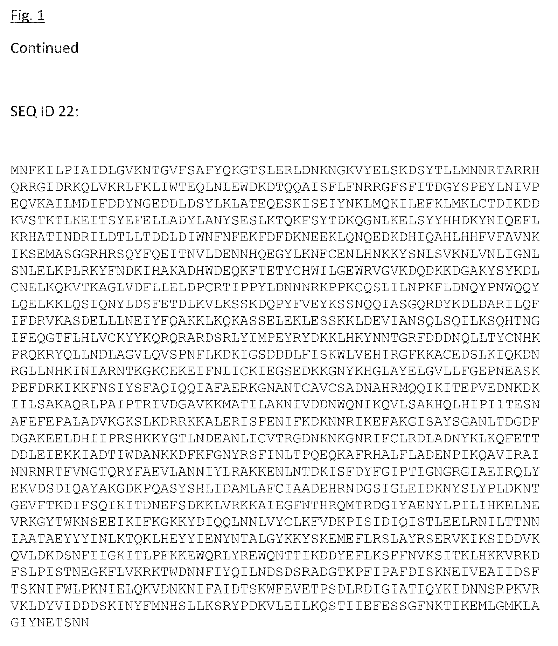

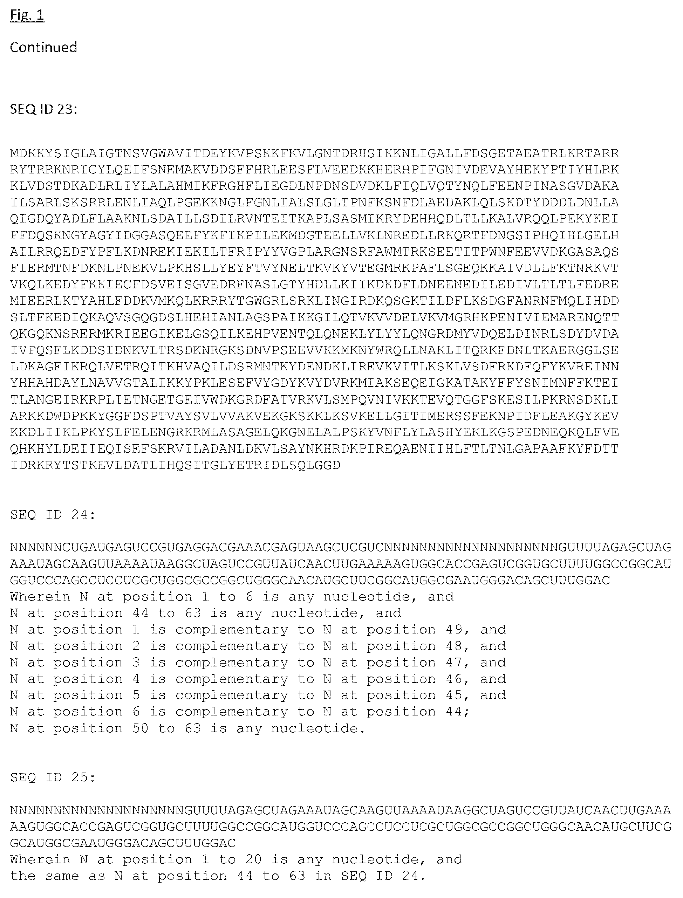

Figure 1: Sequence information

SEQ ID 1: RNA Sequence of the HH ribozyme constant region "backbone"
SEQ ID 2: RNA Sequence of an alternative HH ribozyme constant region "backbone")
SEQ ID 3: RNA Sequence of an alternative HH ribozyme constant region "backbone")
SEQ ID 4: RNA Sequence of an alternative HH ribozyme constant region "backbone")
SEQ ID 5: RNA Sequence of the HH ribozyme constant region "backbone"
SEQ ID 6: RNA Sequence of a thermosensitive pre-gRNA, with a HH fused to the gRNA targeting TS
SEQ ID 7: RNA Sequence of the general structure of a thermosensitive pre-gRNA, with a HH fused to the gRNA
SEQ ID 8: RNA Sequence of the general structure of a thermosensitive pre-gRNA, with a HH fused to the gRNA
SEQ ID 9: RNA Sequence of the general structure of a thermosensitive pre-gRNA, with a HH fused to the gRNA, which has a modified stem loop III.
SEQ ID 10: RNA Sequence of the HDV ribozyme
SEQ ID 11: DNA Sequence of the target sequence (TS) in the agdA gene 214233 (JGI assembly v 3.0 (June 30, 2008)) with PAM
SEQ ID 12: DNA Sequence of the core HH ribozyme
SEQ ID 13: DNA Sequence of primer check_fw for amplification of the agdA TS locus
SEQ ID 14: DNA Sequence of primer check_rev for amplification of the agdA TS locus
SEQ ID 15: DNA Sequence of the target sequence (TS) in the agdA gene 214233 (JGI assembly v 3.0 (June 30, 2008)) without PAM
SEQ ID 16: DNA sequence, coding sequence of the Cas9 protein containing an c-terminal SV40 nuclear localization sequence

SEQ ID 17: DNA sequence of the circular plasmid MST623 containing an expression cassette for the thermosensitive gRNA and a constitutive Cas9 expression in filamentous fungi

SEQ ID 18: RNA sequence of a temperature sensitive gRNA, which is inactive at temperatures below 30°C, targeting the gene kynurenine hydroxylase-white ($kh^w$) of *Anopheles stephensi*

SEQ ID 19: Protein sequence, CRISPR-associated endonuclease Cas9 /Csn1, *Streptococcus pyogenes serotype M1;* UniProt: Q99ZW2

SEQ ID 20: Protein sequence, RISPR-associated endonuclease Cpf1, *Acidaminococcus sp. (strain BV3L6*),

SEQ ID 21: Protein sequence, CRISPR-associated endonuclease Cas9, Campylobacter jejuni subsp. jejuni serotype O:2 (strain NCTC 11168); UniProt: QOP897

SEQ ID 22: Protein sequence, CRISPR-associated endonuclease Cas9, *Francisella tularensis subsp. novicida (strain U112);* UniProt: AOQ5Y3

SEQ ID 23: Protein sequence, CRISPR-associated nuclease deficient endonuclease Cas9, GenBank: AKA60242.1

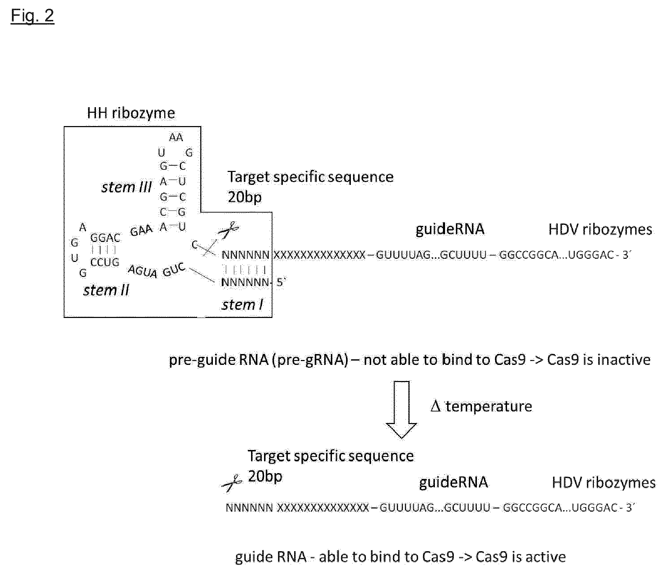

SEQ ID 24: RNA Sequence of a thermosensitive pre-gRNA, with a HH fused to the gRNA targeting TS and a HDV ribozyme fused to the 3'end. A graphical representation of the sequence is shown in Fig. 2 in the upper part.

SEQ ID 25: RNA Sequence of a thermosensitive gRNA, which released the 5' HH ribozyme with a HDV ribozyme still fused to the 3'end. A graphical representation of the sequence is shown in Fig. 2 in the lower part.

Figure 2: Structure of the pre-guideRNA and temperature activation mechanism to form the active gRNA which can be recognized by Cas9

The sequence of the upper part is identified as SEQ ID 24.

The sequence of the lower part is identified as SEQ ID 25.

## DETAILED DESCRIPTION OF THE INVENTION

Specific terms as used throughout the specification have the following meaning.

The term "cell line" as used herein refers to an established clone of a particular cell type that has acquired the ability to proliferate over a prolonged period of time. The term "host cell line" refers to a cell line as used for expressing an endogenous or recombinant gene or products of a metabolic pathway to produce polypeptides or cell metabolites mediated by such polypeptides. A "production host cell line" or "production cell line" is commonly understood to be a cell line ready-to-use for cultivation in a bioreactor to obtain the product of a production process, such as a POI. The term "eukaryotic host" or "eukaryotic cell line" shall mean any eukaryotic cell or organism, which may be cultivated to produce a POI or a host cell metabolite.

It is well understood that the eukaryotic cell lines as specifically described herein are somatic cell lines, thus, the scope of the present invention does not encompass human beings or techniques directly related to human germline manipulation or human cloning.

Isolated clones or elements of the CRISPR/Cas system used in the host cells are artificial products, "man-made" or synthetic products. In particular, such clones or elements are not naturally-occurring, and are therefore not considered as a result of "law of nature". The mutant host cell as described herein is specifically understood as an artificial cell, which is non-naturally occurring, thus, is differentiated from a wild-type host cell.

The host cell engineered as described herein, thereby obtaining the mutant host cell, specifically is provided as a host cell line, and preferably as a production host cell line, which further comprises a gene encoding a POI, in particular a heterologous POI, and the respective expression system for POI production. Specifically, the cell line is a eukaryotic host cell line.

Yet, certain embodiments refer to a host cell in a transgenic animal, and host cells as used for transgenic animal production, in particular transgenic mammals, however, excluding human beings.

Whereas the host cell as described herein is specifically characterized by an expression system expressing the GOI, the mutant host cell produced by the method as described herein is specifically characterized by an altered expression system and the altered expression of the GOI, thereby regulating the expression of a gene encoding a POI, and the POI production. Specifically, the mutant host cell is characterized by an altered GOI expression system that is not natively associated with the coding sequence of the POI.

Mutant cell lines may be recombinant cell lines employing recombination means and methods to obtain a recombinant DNA, thus obtained by recombinantly engineering the cell genome. Such recombinant engineering typically employs artificial constructs like plasmids or oligonucleotides or RNA/ DNA or respective fragments, as tools to produce a recombined DNA. Specific mutants may be obtained by mutating a (chromosomal) region, thereby obtaining a genomic mutation at a specific locus of the chromosome. A mutant recombinant DNA may specifically be produced by either random or targeted recombination. Exemplary mutated cells comprise at least one genetic element exogenous to the cell that is integrated into the cell genome. In some aspects, the exogenous genetic element can be integrated at a random location in the cell genome. In other aspects, the genetic element is integrated at a specific site in the genome. For example, the genetic element may be integrated at a specific position such as to provide a change relative to the endogenous sequence.

Specific exemplary mutated production cells as described herein comprise MOI to change the expression of a GOI in support of a POI production. It is understood that mutant cell lines may be provided as a product ready-to-use for cultivation, e.g. for research, industrial or analytical use.

The term "cell culture" or "cultivation", also termed "fermentation", with respect to a host cell line is meant the maintenance of cells in an artificial, e.g., an *in vitro* environment, under conditions favoring growth, differentiation or continued viability, in an active or quiescent state, of the cells, specifically in a controlled bioreactor according to methods known in the industry.

When cultivating a cell culture, the cell culture is brought into contact with suitable cell culture media in a culture vessel or with substrate under conditions suitable to support cultivation of the cell culture. Cell culture media provide the nutrients necessary to maintain and grow cells in a controlled, artificial and *in vitro* environment. Characteristics and compositions of the cell culture media vary depending on the particular cellular requirements. Important parameters include osmolality, pH, and nutrient formulations. Feeding of nutrients may be done in a continuous or discontinuous mode according to methods known in the art.

Whereas a batch process is a cultivation mode in which all the nutrients necessary for cultivation of the cells are contained in the initial culture medium, without additional supply of further nutrients during fermentation, in a fed-batch process, after a batch phase, a feeding phase takes place in which one or more nutrients are supplied to the culture by feeding. The purpose of nutrient feeding is to increase the amount of biomass in order to increase the amount of recombinant protein as well.

In certain embodiments, the method as described herein is employed in a fed-batch process. Specifically, the host cell culture is first cultured at the first working temperature in a growth phase medium and transitioned to a production phase medium applying the second working temperature in order to produce a desired recombinant POI.

In another embodiment, host cells are first processed by the method steps a) and b), thereby obtaining the engineered host cell ready for POI production, followed by cultivation in batch, fed-batch, or continuous mode, e.g. a chemostat. A continuous fermentation process is characterized by a defined, constant and continuous rate of feeding of fresh culture medium into the bioreactor, whereby culture broth is at the same time removed from the bioreactor at the same defined, constant and continuous removal rate. By keeping culture medium, feeding rate and removal rate at the same constant level, the cultivation parameters and conditions in the bioreactor remain constant.

The suitable cultivation techniques may encompass cultivation in a bioreactor starting with a batch phase, followed by a short exponential fed batch phase at high specific growth rate, further followed by a fed batch phase at a low specific growth rate. Another suitable cultivation technique may encompass a batch phase followed by a continuous cultivation phase at a low dilution rate.

A preferred embodiment includes a batch culture to provide biomass followed by a fed-batch culture for high yields POI production.

It is preferred to cultivate the host cell line as described herein in a bioreactor under growth conditions to obtain a cell density of at least 1 g/L cell dry weight, more preferably at least 10 g/L cell dry weight, preferably at least 20 g/L cell dry weight. It is advantageous to provide for such yields of biomass production on a pilot or industrial scale.

The POI production method as described herein specifically allows for the fermentation process on a pilot or industrial scale. The industrial process scale would preferably employ volumina of at least 10 L, specifically at least 50 L, preferably at least 1 $m^3$, preferably at least 10 $m^3$, most preferably at least 100 $m^3$.

Production conditions in industrial scale are preferred, which refer to e.g. fed batch cultivation in reactor volumes of 100 L to 10 $m^3$ or larger, employing typical process times of several days, or continuous processes in fermenter volumes of approximately 50 - 1000 L or larger, with dilution rates of approximately 0.02 - 0.15 h-1

A stable cell culture is specifically understood to refer to a cell culture maintaining the genetic properties, specifically keeping the POI production level high, e.g. at least at a μg level, even after about 20 generations of cultivation, preferably at least 30 generations, more preferably at least 40 generations, most preferred of at least 50 generations. Specifically, a stable recombinant host cell line is provided which is considered a great advantage when used for industrial scale production.

The cell culture as described herein is particularly advantageous for methods on an industrial manufacturing scale, e.g. with respect to both the volume and the technical system, in combination with a cultivation mode that is based on feeding of nutrients, in particular a fed-batch or batch process, or a continuous or semi-continuous process (e.g. chemostat).

The term "expression" or "expression system" or "expression cassette" refers to nucleic acid molecules containing a desired coding sequence and control sequences in operable linkage, so that hosts transformed or transfected with these sequences are capable of producing the encoded proteins or host cell metabolites. Expression may be transient or may be stable. In order to effect transformation, the expression system may be included in a vector; however, the relevant DNA may also be integrated into the host chromosome. Expression may refer to secreted or non-secreted expression products, including polypeptides or metabolites.

"Expression constructs" or "vectors" used herein are defined as DNA sequences that are required for the transcription of cloned recombinant nucleotide sequences, i.e. of recombinant genes and the translation of their mRNA in a suitable host organism. A vector as used herein specifically includes autonomously replicating nucleotide sequences as well as genome integrating nucleotide sequences.

Expression vectors, herein also referred to as "plasmids" usually comprise an origin for autonomous replication in the host cells, selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The promoter for controlling transcription can be any promoter for any RNA polymerase. If transcription occurs *ex vivo,* one typically uses bacteriophage-derived T7, T3, and SP6 RNA polymerases in conjunction with their cognate promoters. A DNA template for transcription may be obtained by cloning a nucleic acid and introducing it into an appropriate vector for transcription. The DNA may be obtained by reverse transcription of RNA.

As described herein, the RNA specifically used in the RNA-guided system may be provided by *in vitro* transcription wherein RNA is *in vitro* synthesized in a cell-free system, preferably using appropriate cell extracts or chemical synthesis, or by *in vivo* transcription wherein RNA is *in vivo* synthesized in a cell-based system. Preferably, an expression plasmid is applied for the generation of transcripts obtained by transcription of an appropriate DNA template, which plasmids are herein specifically understood as cloning vectors. Specifically an expression plasmid employed for the purpose of the invention may be used for expression of the pre-gRNA, or any of its components which assemble to produce the pre-gRNA in the host cell; in particular for the expression of the gRNA, or any of the tracrRNA and the crRNA components of a gRNA. Specifically, the respective nucleotide sequences may be provided by one or more expression plasmids which are co-transfected.

RNA expression systems commonly used for delivery of RNA molecules to the cell may be employed. According to a specific embodiment, the endonuclease is co-expressed together with a tracrRNA and/or crRNA and/or gRNA designed to target a specific coding or non-coding sequence, e.g. an endogenous host cell gene to impair or knock out the function of a gene encloding the POI. A suitable DNA may be used in an expression construct to express the tracrRNA and/or crRNA and/or gRNA or the functional pair of the tracrRNA or gRNA or the constant part of the gRNA and the RNA-guided endonuclease. Therefore, there is further provided such DNA which is a template DNA, e.g. comprising the sequence encoding the tracrRNA and/or crRNA and/or gRNA and/or the constant part of the gRNA, and optionally a DNA encoding the RNA-guided endonuclease, specifically operably linked to regulatory sequences to express such molecules *in vivo* or *in vitro.*

The RNA(s) may be synthesized ex vivo, e.g. in vitro transcribed RNA or synthetic RNA, and delivered to, e.g. (co-)transfected into, a cell by suitable means.

Transfection of RNA or the DNA encoding such RNA may be accomplished by a variety of means known to the art including, e.g., electroporation, microinjection, liposome fusion, lipofection.

According to a specific aspect, transformed or transfected cells transiently express the inserted DNA or RNA for limited periods of time. For instance, the foreign DNA or RNA persists in the nucleus of the cell for several days. Transfection may as well be stable to produce a stable transfectant, e.g. introducing and optionally integrating foreign DNA or RNA into the transfected cell. Likewise, the endonuclease may be produced by a cell transformed by a DNA encoding the endonuclease, in particular a codon-optimized DNA, or produced separate from the cell, and delivered to the cell by suitable means, including electroporation.

The term "RNA" as used herein encompasses double-stranded RNA, single-stranded RNA, isolated RNA such as partially or completely purified RNA, essentially pure RNA, synthetic RNA, and recombinantly generated RNA, such as modified RNA which is functionally the same or similar, but differs from naturally-occurring RNA by addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

The terms "gRNA", "pre-gRNA", "tracrRNA", and "crRNA" are understood in the following way.

A guide RNA (gRNA, also termed chimeric guide RNA) is a chimeric RNA molecule comprising the tracrRNA, which - together with the constant part of the crRNA - specifically determines the structure of the gRNA necessary to provide a co-substrate to a matching RNA-guided endonuclease, also termed chimeric guide RNA scaffold, which is understood as a constant RNA sequence forming a functional pair with an endonuclease guided by the gRNA. The crRNA comprises a constant part capable of interacting with or linking to the tracrRNA, and a variable part (also termed oligo RNA) which is composed of a short oligonucleotide sequence which is complementary to a DNA target site in the human genome. The constant part of the crRNA is typically located at the 3' part of the molecule, whereas the variable part is typically located at the 5' end of the molecule. The tracrRNA and the crRNA may directly associate though hybridizing parts, or joined with a linker sequence.

gRNA forms a co-substrate to direct RNA-guided endonuclease activity to the genomic target site where the gRNA (through its crRNA component) hybridizes with the target. Thus, the crRNA is understood as containing the part encoding the genome editing information in the form of complementary sequences (allowing GU as well as GC base pairs), and the RNA-guided DNA endonuclease is understood as a nuclease cleaving target DNA at a specific site. For example, a Cas endonuclease may assemble with the chimeric gRNA in a host cell and can induce the formation of a DNA breaks, e.g. a double strand DNA break at a site complementary to the gRNA sequence in genomic DNA. This cleavage activity requires both Cas9 and the complementary binding of the guide RNA through the variable crRNA part.

The term "cellular repair mechanism" as used herein is specifically understood as mechanisms to detect and repair the various types of damage that can occur to DNA. A specific DNA damage is single-strand or double-strand breaks, which may be highly deleterious possibly leading to loss or rearrangement of genomic sequences. Double-strand breaks are repaired through non-homologous end joining (NHEJ) or homologous recombination (HR). In NHEJ, additional errors can be introduced during this process leading to specific mutations proximal to the DNA break. Therefore, NHEJ is considered inherently mutagenic as it relies on chance pairings, called microhomologies, between the single-stranded tails of the two DNA fragments to be joined. HR is a repair process that uses a DNA template for correction. It is more precise than NHEJ, yet less efficient. If a suitable exogenous DNA template is provided to the cells, HR offers the possibility to engineer mutations in specific genes of interest.

Therefore the gRNA as described herein is typically a non-coding RNA, specifically hybridizing with a DNA target site and directing the RNA-guided endonuclease to the DNA target site, to induce a DNA break within the region of hybridization. This system provides for invaluable tools for host cell genome engineering at the cellular level by a CRISPR/Cas system to achieve RNA-guided genome engineering in the cells.

The present description refers to the pre-gRNA, which is an inactive co-substrate of the Cas9 endonuclease. Such pre-gRNA can be activated by cleavage at a predetermined cleavage site to produce the gRNA within a host cell.

The pre-gRNA as described herein is specifically characterized by containing one or more additional ribosomal components which mask the crRNA sequence such that is not hybridizing to the target site, or which otherwise prohibit the co-substrate activity thereby rendering the Cas9 endonuclease inactive. As additional ribosomal element the HH ribozyme is particularly used. Specific embodiments of a pre-gRNA comprise the gRNA flanked by the nucleotide sequence encoding the ribozyme on one end, preferably on the 5'end, or on both sides, as an extension of the gRNA sequence. In certain embodiments, a HH ribozyme is at the 5' end of the gRNA. In certain embodiments, a HH ribozyme is at the 3' end of the gRNA. In certain embodiments, a HDV ribozyme is at the 5' end of gRNA. In some embodiments, a HDV ribozyme is at the 3' end of the gRNA. In a certain embodiment, a HH ribozyme is at the 5' end and a HDV ribozyme is at the 3' end of gRNA.

A specific embodiment refers to the HH ribozyme directly linked to the crRNA component of the gRNA, which produces the gRNA with well-defined ends upon cleavage. No additional nucleotide which would extend the crgRNA upon, e.g. no additional guanosine nucleotide upstream the genomic targeting site, is needed. In a specific example, the HH ribozyme is linked to the gRNA as a 5-terminal part of the pre-gRNA, by fusing a short (e.g. 3-10 nt) reverse complementary sequence to the 5' end of the HH ribozyme, which hybridizes to the 5' end of the gRNA, which is part of the variable region of the crRNA adjacent to the 3' end of the HH ribozyme.

Any of the ribozymes as used in the pre-gRNA specifically comprises a ribonuclease recognition site, to support the cleavage between the gRNA and the rest of the ribozyme, thereby releasing the active gRNA. Thus, the pre-gRNA specifically comprises the gRNA which is flanked by one or two ribonuclease recognition sites of a ribozyme, on one or on both ends. A genetic element is said to be "flanked" by another genetic element when located immediately adjacent to each other.

The HH ribozyme is specifically understood as follows.

The HH ribozyme is a small naturally-occurring ribozyme, i.e. a catalytic RNA motif or molecule capable of endonucleolytic (self-) cleavage (Long and Uhlenbeck (1993) Faseb J. 7: 25-30. It is composed of a highly conserved catalytic core of eleven nucleotides (nts), which are flanked by three helices which are base-paired stems, called stem I, stem II and stem III, surrounding the conserved core of nucleotides, loop regions are found adjacent to the stem region. In HH ribozymes of type I the loop region adjacent to stem I is open, whereas the loop regions of stem II and stem III are forming closed loops (hairpin loops). The loop region of stem I and II form essential tertiary interactions for fast self-scission under physiological conditions. It belongs to the family of small endonucleolytic ribozymes that have sizes in the range of from 50 to 150 nucleotides (nt). Other members of this family are hepatitis delta virus (HDV) ribozymes, the Varkud satellite (VS), and the hairpin ribozymes. An exemplary structure or sequence of a HH ribozyme without the open stem I (i.e. backbone) can be found in SEQ ID 5.

The HH ribozyme catalyzes the scission of its own phosphodiester backbone by means of a transesterification reaction that proceeds under inversion of the configuration.

HH ribozymes as used herein can be naturally-occurring, e.g. obtained from any of the following species: *Schistosoma mansoni, Selaginella moellendorfii, Desulfotomaculum reducens, Nemastostella vectensis, Xenopus tropicalis.* Further examples can be found in the following publication Hammann, C., et al. 2012. Rna 18, 871-885. Alternatively, artificial functional variants of any wild-type ribozyme may be used. Exemplary variants may specifically differ in any of the hairpin stem or loop sequence, or length. Specifically, any of the stems may be extended or truncated to obtain a varying hairpin length and thermostability.

Specifically, the hairpin stem I can be extended to increase the second working temperature. Therefore, the hairpin stem I may consist of at least 7 bp, or at least 8 bp, or at least 9 bp, or at least 10 bp, e.g. up to 12 bp or 11 bp.

Specifically, the hairpin stem I can be truncated to lower the second working temperature. Therefore, the hairpin stem I may consist of at least 3 bp and up to 5 bp, or up to 4 bp, or 3 bp.

Exemplary HH ribozyme sequences are provided in Figure 2.

The HH hairpin as described herein specifically comprises a "hairpin stem" part which is a structured section of the RNA with intrastrand pairing, i.e. two stem areas of the RNA sequence that have internal complementarity which results in it folding into a hairpin. The region between the two stem areas is forming a loop, herein also referred to as "hairpin loop". A thermosensitive RNA hairpin as used for the purpose of the invention as an internal structure element of the HH ribozyme is herein also referred to as HH hairpin. Such HH hairpin is specifically engineered as a thermosensitive element, which has the hairpin structure at a first working temperature, and which diverges (by splitting the paired stem areas) to obtain the linear conformational structure at the elevated (second) working temperature. By such change of the hairpin structure the HH ribozyme is activated with the consequence of self-cleaving at the cleavage site adjacent to the gRNA of the pre-gRNA, thereby re-establishing the crRNA sequence which is complementary to the genomic target site and recognizes such target site within the host cell genome.

Any of the naturally occurring HH ribozymes can be used as a parent HH ribozyme to engineer and produce a mutated HH ribozyme which is characterized by one or more stems of different composition or length. Such modifications provide for a change of thermosensitivity, because it takes higher or lower energy to induce the conformational change of the HH hairpin. Specifically, the stem I and/or the stem III regions of a HH ribozyme as used herein may be modified. Specific ways to modify the HH hairpin are described in the examples section herein.

Specifically, a stem region of the hammerhead ribozyme can be modified by the selection of a TS, which contains:

a) More or equal than 4 G or C residues in first 6 bp of the 20bp sequence
b) Or by an elongation of the stem I region (>6bp) so that the melting temperature >= 20°C
c) The sequence of the gRNA starts with NNNCUC, wherein N is any of A, G, C, or U.

The melting temperature (Tm) of the stem I is thus calculated by assigning 2°C to each A-U pair and 4°C to each G-C pair and forming the sum out of each residue, which contributes to the stem.

Equation: Calculation melting temperature

$$Tm\ [°C] = 4[°C]^{*}\ \#(GC) + 2[°C]^{*}\ \#(AU)$$

#(GC) number of GC pairs in sequence
#(AU) number of AU pairs in sequence

Therefore, the hairpin structure specifically determines the thermosensitive characteristics of the HH ribozyme. Such pre-gRNA as described herein can be used in a CRISPR/Cas system as a thermo-inducible element of protein production. The thermoinduction of gRNA release and activity typically is not reversible. Upon activating the pre-gRNA at the second working temperature, the host cell can be cultivated at a third working temperature, which is independent on any prior working temperature, thus, which may be different from any of the first or second temperatures.

Certain pre-gRNA further comprise a HDV ribozyme which is characterized bya intricate nested double pseudoknot structure consisting of five paired helical segments, P1, P2, P3, P4, and P1.1 (Ferre-D'Amare, Nature, 395 (1998), pp. 567-574).

Exemplary HDV ribozyme sequences are provided in Figure 1.

The CRISPR/Cas system typically comprises a set of matching RNA-guided endonuclease and tracrRNA or gRNA or constant part of gRNA, which is herein understood as a functional pair, which may be used with one or more variable parts, i.e. with one or more crRNA or crRNA variable parts, e.g. a 20b, 22b, 24b or 26b RNA-type oligonucleotide, to target one or more predetermined, random or different genomic target sites of the host cell. For example, a set of Cas endonuclease, e.g. type II, and a matching tracrRNA is used for interference of the crRNA (the oligonucleotide conjugated to the 5' end of the tracrRNA, e.g. employing a linker) with the target nucleic acid sequence through its variable crRNA oligo sequence. Specifically, the Type-II Cas9 protein is used, which is targeted to DNA. Targeting occurs upon hybridization of the crRNA to the complementary target site.

Exemplary sequences of pre-gRNA and Cas endonucleases are shown in Figure 1. Functional variants of the endonuclease, tracrRNA or the gRNA are feasible. In particular, gRNA variants may comprise a variable 3' end, e.g. within the region of the 20, or 15, or 10, or 6 terminal bases, such as a truncation, elongation and/or one or more point mutations of any of the bases in the 3' terminal RNA sequence.

Functional variants of the RNA-guided endonuclease or Cas endonuclease are specifically those of the same type or subtype as obtained from bacterial sources or derived from the amino acid sequences of bacterial origin, including artificial or recombinant enzymes comprising the same or mutated sequences, e.g. comprising one or more mutations and a specific sequence identity to the wild-type sequence. Specifically, the variants are considered functional if the Cas endonuclease is transcriptionally active. Specific functional variants of the Cas endonuclease comprise a codon-optimized sequence, for improved expression in a host cell. Further specific functional variants are Cas endonucleases which have "nickase" activity. Specific Cas9 nickases are derived from the Cas9 of S. *pyogenes* and comprise an amino acid mutation at position D10A or H840A resulting in the inactivation of the catalytic activity of one nuclease domain and converting Cas9 to a "nickase" enzyme that makes single-stranded breaks at the target site. A mutation in RuvC-like (RuvCl) domain or in the HNH domain of the S. *pyogenes* Cas9 (D10A or H840A)) renders it to a nickase, which only can cleave a single strand of the DNA.

Examples of wild-type (wt) enzymes are those obtained from bacterial sources. Such wt enzymes may serve as parent sequence to obtain analogs from other species e.g. other bacterial species of the same genus or family as the parent endonuclease, or mutated wt sequences comprising one or more point mutations, such as to obtain functional variants comprising mutations that do not interfere and even improve the endonuclease activity.

As a co-substrate to the Cas endonuclease, the pre-gRNA comprising the wt tracrRNA or gRNA, in particular the constant part of the gRNA or tracrRNA may be used. Alternatively, functional variants of the tracrRNA or gRNA (in particular the constant part of gRNA) may be used, e.g. which are obtained by mutagenesis of the wt sequences used as parent sequences.

The functionally active variant of an RNA, such as a gRNA or a component of gRNA, e.g. the tracrRNA as described herein, is specifically understood to encompass a nucleotide sequence which forms a functional co-substrate to the matching RNA-guided endonuclease, and/ or any of the functionally active variants, including truncated versions or fragments, mutants or hybrid nucleic acid sequences of a wild-type RNA. Functional variants of the RNA molecules as described herein may e.g. be obtained by one or more mutations in the nucleotide sequence of a parent (wild-type) RNA, wherein the mutated RNA is still functional and hybridizes under stringent conditions to a strand complementary to the parent RNA.

It is understood that the term "constant" with respect to a RNA sequence or a part of an RNA sequence, as used herein shall refer to the sequence of the RNA which is determined by the sequence of bacterial origin of a specific species, independent on the variability of the oligonucleotide (being part of the crRNA) which hybridizes with a target DNA. Such constant RNA molecule or part of a gRNA is typically of the same or similar structure for all cells of a specific species, and provides for interaction with the RNA-guided endonuclease of the same species thereby forming a functional pair, independent on type or origin of the genomic target site. It is well understood that such constant molecules or parts of the molecules may still vary from species to species, or be used as a parent molecule to produce mutants, which may be used as functional variants.

The "variable" part of the gRNA or the crRNA as described herein is understood as the part that hybridizes with a specific part of a target DNA, thus is complementary to any specific site. Since the genomic target sites may be located throughout the host cell genome, a plurality of oligonucleotides may be used for hybridizing the crRNA or gRNA with the target site. Therefore, this part is considered to be variable, characterized by the sequence which has an affinity to bind the specific hybridization target.

The term "variant" as used herein shall refer to any sequence with a specific sequence identity or homology to a comparable parent sequence. A variant is specifically any sequence derived from a parent sequence e.g., by size variation, such as (terminal or non-terminal, such as "interstitional" i.e. with deletions or insertions within the nucleotide sequence) elongation, or fragmentation, mutation, hybridization (including combination of sequences).

The "functionally active variant" or "functional variant" of a nucleotide or amino acid sequence as used herein specifically means a mutant sequence, e.g. resulting from modification of a parent sequence by insertion, deletion or substitution of one or more nucleotides or amino acids within the sequence or at either or both of the distal ends of the sequence, and which modification does not affect (in particular impair) the activity of this sequence.

Specifically, the functionally active variant of any of the molecules or components of the CRISPR/Cas system has substantially the same activity as the non-modified (naturally-occurring or wild-type) parent molecule and is selected from the group consisting of

- homologs with at least about 60% nucleotide sequence identity, preferably at least 70%, at least 80%, or at least 90% degree of homology or sequence identity to the parent sequence; and/or

- homologs obtainable by modifying the parent sequence, or the sequence of a size variant used as a template to provide for mutations, e.g. by insertion, deletion or substitution of one or more nucleotides within the sequence or at either or both of the distal ends of the sequence;
- sequence variants derived from a parent or wild-type sequence as described herein by extension and/or fragmentation of the parent sequence, e.g. +/- 50% or +/-25%, or +/-10% of the length; or
- analogs derived from species other than *Streptococcus pyogenes, Streptococcus thermophiles, Neisseria Meningitis or Treponema Denticola,* or the Cpf1 enzyme originating from *Acidaminococcus sp.* or *Lachnospiraceae bacterium.*

The functionally active variants as described herein are also understood to encompass hybrids or chimeras of two or more parent sequences, e.g. resulting from combination of sequences that qualify as parent sequence with functional activity.

Suitable variants have "substantially the same activity", which term is herein specifically understood to refer to the activity as indicated by substantially the same or improved efficacy of directed DNA break and/or mutagenesis, e.g. +/- 50% or +/- 25%, or +/-10%, as determined by the rate of successful DNA break and/or recombination.

The term "homolog" or "homology" indicates that two or more nucleotide or amino acid sequences have the same or conserved pairs at a corresponding position, to a certain degree, up to a degree close to 100%. A homologous sequence of a functionally active variant typically has at least about 60% nucleotide or amino acid sequence identity, preferably at least about 70% identity, more preferably at least about 80% identity, more preferably at least about 90% identity, more preferably at least about 95% identity, more preferably at least about 98% or 99% identity. The term "homologous" may also include analogous sequences.

"Percent (%) identity" with respect to the nucleotide or amino acid sequence is defined as the percentage of nucleotides in a candidate DNA sequence that is identical with the nucleotides in the DNA sequence or the amino acids in a peptide/ polypeptide/ protein sequence, after aligning the sequence and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for measuring alignment, including any algorithms needed to achieve maximal alignment over the full length of the sequences being compared.

A functionally active variant of a parent sequence as described herein may specifically be obtained through mutagenesis methods. The term "mutagenesis" as described herein shall refer to a method of providing mutants of a sequence, e.g. through insertion, deletion and/or substitution of one or more nucleotides or amino acids, so to obtain variants thereof. Mutagenesis may be through random, semi-random or site directed mutation. Typically large randomized gene libraries are produced with a high gene diversity, which may be selected according to a specifically desired genotype or phenotype.

Preferably, the functionally active tracrRNA comprises or consists of a nucleotide sequence of at least 50 bases, specifically at least 60 bases, typically up to 90 or 100 bases. According to a specific example, the truncated tracrRNA is typically about 60 bases long, preferably 60-70 bases, e.g. 66 bases long, the full-length tracrRNA is typically 90 bases long. Some of the preferred functionally active variants of the tracrRNA as described herein are size variants or specifically fragments of a tracrRNA including truncated versions, preferably those including the 3' part of the tracrRNA molecule, e.g. including a truncated 5' part of a nucleotide sequence. For example a nucleotide sequence derived from one of exemplary tracrRNA nucleotide sequences which has a specific length and insertions or a deletion of the 5' terminal region, e.g. an elongation or truncation of the nucleotide sequence at the 5' end, so to obtain a specific length with a range from the 3' end to a varying 5' end, such as with a length of the nucleotide sequence of at least 50 bases, preferably at least 60 bases. The elongated size variant preferably comprises additional one or more nucleotide(s) at the 5' end of the tracrRNA sequence.

Preferably, the functionally active crRNA comprises or consists of a nucleotide sequence of at least 25 bases, specifically at least 30 bases, typically up to 70 or 80 or 90 or 100 bases. According to a specific example, the truncated crRNA is typically about 30 bases long, preferably 30-40 bases, e.g. 32 bases long, the full-length crRNA is typically 50-60 bases long, e.g. 55 bases. Some of the preferred functionally active variants of the crRNA as described herein are size variants or specifically fragments of a crRNA including truncated versions, preferably those including the 5' part of the crRNA molecule, e.g. including a truncated 3' part of a nucleotide sequence. For example a nucleotide sequence derived from one of exemplary crRNA nucleotide sequences which has a specific length and insertions or a deletion of the 3' terminal region, e.g. an elongation or truncation of the nucleotide sequence at the 3' end, so to obtain a specific length with a range from the 5' end to a varying 3' end, such as with a length of the nucleotide sequence of at least 25 bases, preferably at least 30 bases. The elongated size variant preferably comprises additional one or more nucleotide(s) at the 3' end of the crRNA sequence.

The functionally active tracrRNA variants may still include a region of complementarity to interact with the constant part of the crRNA. On the other hand, the functionally active crRNA variants may still include a region of complementarity to interact with the trcrRNA. Typically, the 3' part of the crRNA or a functional variant of the crRNA is interacting with the 5' part of the tracrRNA (with or without a linker) through a region of complementarity. Thus, it is preferred that functional variants of the tracrRNA and the crRNA still comprise a region of complementarity which is at least 5 bp, preferably at least 10 bp, specifically located in the 5' part of the tracrRNA and in the 3' part of the crRNA.

A functionally active variant of a crRNA, in particular the variable part of the crRNA, or an oligonucleotide as described for the purpose of the present invention need not be 100% complementary to its target sequence to be specifically hybridizable. An oligonucleotide is specifically hybridizable when binding of the oligonucleotide to the target DNA molecule interferes with the normal function of the target DNA, and there is a sufficient degree of complementarity to avoid non-specific binding of the oligonucleotide to non-target sequences under conditions where specific binding is desired, for example under physiological conditions in the case of in vivo assays or systems. Such binding is referred to as specific hybridization, e.g. hybridization under stringent conditions.

Preferably, the functionally active RNA-guided endonuclease comprises or consists of an amino acid sequence of 500 to 3000 amino acids, preferably at least 1000 amino acids. Some of the preferred functionally active variants of the endonuclease as described herein are size variants or specifically fragments of a parent enzyme, in particular where the functionally active variants still comprise the active site of the enzyme including a RuvCI domain (containing a catalytic Asp residue) and an HNH domain (containing a catalytic His residue). Functionally active Cas endonuclease variants as described herein are specifically characterized by exhibiting substantially the same endonuclease activity as the non-modified (wt) Cas endonuclease (+/-10%, or +/- 5%), or even a higher endonuclease activity.

The term "GOI" as used herein is understood as a "gene of interest" which regulates the expression of a gene encoding a POI. Thus, changing the structure (sequence) or function of GOI would change the host cell's capability of producing the POI. A MOI as described herein typically comprises one or more several (e.g. at least 1, 2, or 3; and/or up to 10 or 15) point mutations at the GOI which upregulates or downregulates the GOI expression. Such MOI is typically located within the GOI or at least within the GOI expression system, e.g. the GOI ORF. As typical for the CRISPR/Cas system, the MOI is located in close proximity to a PAM sequence where the DNA break induced by the Cas endonuclease and the DNA repair takes place. Exemplary GOI are listed in Table 1:

Table 1: List of exemplary GOI

| **Function/Annotation** | **Name** | **Systematic GeneID** | **Organism** |
|---|---|---|---|
| alpha-glucosidase | agdA | An04g06920 | Aspergillus niger |
| Orotidine-5'-phosphate decarboxylase | pyrG | An12g03570 | Aspergillus niger |
| Actin structural protein | actA | An15g00560 | Aspergillus niger |
| Pyruvate kinase | pkiA | An07g08990 | Aspergillus niger |
| 6-phosphofructo-1-kinase | pfkA | An18g01670 | Aspergillus niger |
| Essential chromatin-associated protein | MCM10 | YI L150C | Saccharomyces cerevisiae |
| Essential nuclear envelope integral membrane protein | BRL1 | YHR036W | Saccharomyces cerevisiae |

A "MOI" as understood herein, typically has a mutagenesis pattern of a CRISPR/Cas system which is characterized by a small number of mutations, e.g. 1-x point mutations, which can include one or more of any of small (random) inserts or deletions of one or more nucleotides as desirable, e.g. to produce frameshift mutations. In particular, such deletions or insertions or frameshift mutations provide for knockout mutations, which are understood to encompass any mutation within a gene sequence or regulatory sequence directing the function of a gene, e.g. leading to a different gene expression as assessed at the protein level or a different phenotype, e.g. leading to a significant loss of the function of a gene (partial knock-out) or a complete knock-out of the gene. The significant functional loss of a gene specifically provides for a gene expression level or gene function of less than 10%, preferably less than 5%, or no detectable gene expression or function as compared to the parent cell without the knockout mutation. Specific mutations lead to a different gene expression. Also, exons or genes or chromosomal parts including a series of genes may be exchanged and marker sites introduced, e.g. restriction sites, or tags.

The term "genomic target site" or "target site" as used herein shall refer to a genetic sequence of interest which is any locus or nucleic acid sequence endogenous to a cell, such as, for example a gene or a non-coding sequence within or adjacent to a GOI, in which it is desirable to modify by targeted mutagenesis and/or targeted homologous recombination. The genetic target site can be within the coding sequence of a gene, within transcribed non-coding sequence such as, for example, promoter or leader sequences, or introns, or within non-transcribed sequence, either upstream or downstream of a coding sequence.

The DNA target site is typically characterized by a protospacer associated motif (PAM), which is a short DNA recognition site located adjacent to the target site in the human DNA sequence and which defines the site of RNA hybridization and the DNA break. Typically, the RNA hybridization is such that the crRNA hybridizes with the DNA sequence upstream the PAM motif, e.g. the DNA sequence joined to the 5' end of the motif. The DNA break is then catalyzed within the region of hybridization, e.g. a DNA break proximal to the PAM motif, in most cases in close proximity to the 5' end of the motif, such as within 10 positions, or within/ at 5 positions or within/ at 3 positions upstream the PAM motif. Following the DNA break, the cellular repair mechanism provides for rejoining the DNA ends with or without incorporating mutations, typically proximal to the DNA break, e.g. in close proximity to the 5' end or 3' end of the DNA break, such as within 20 positions, or within 10 positions, or within 5 positions or within 3 positions upstream or downstream the DNA break.

A specific genomic target site of interest is particularly predetermined and selected at a position of the host cell chromosomal genome where a DNA cleavage (single stranded or double stranded DNA break) and optionally recombination and/or mutation is desirable to regulate protein expression, in particular the POI expression, and where a PAM motif is present or has been introduced.

Specifically the genomic target site as described herein is in close proximity to a PAM motif, in particular in close proximity to the 5' end of a PAM motif, such as within 10 positions, or within/ at 5 positions or within/ at 3 positions upstream the PAM motif. Following the DNA break, the cellular repair mechanism provides for rejoining the DNA ends with or without incorporating mutations, typically proximal to the DNA break, e.g. in close proximity to the 5' end or 3' end of the DNA break, such as within 20 positions, or within 10 positions, or within 5 positions or within 3 positions upstream or downstream the DNA break.

Therefore, a specific genomic target site is suitably predetermined at the GOI, which means that the target site is located in the genome in close proximity or within a GOI which is subject to mutation by the present method. Typically the target site is within the GOI, or within 20 positions, or within 10 positions, or within 5 positions or within 3 positions upstream or downstream the GOI.

As used herein, the term "hybridization" or "hybridizing" is intended to mean the process during which two nucleic acid sequences anneal to one another with stable and specific hydrogen bonds so as to form a double strand under appropriate conditions. The hybridization between two complementary sequences or sufficiently complementary sequences depends on the operating conditions that are used, and in particular the stringency. The stringency may be understood to denote the degree of homology; the higher the stringency, the higher percent homology between the sequences. The stringency may be defined in particular by the base composition of the two nucleic sequences, and/or by the degree of mismatching between these two nucleic sequences. By varying the conditions, e.g. salt concentration and temperature, a given nucleic acid sequence may be allowed to hybridize only with its exact complement (high stringency) or with any somewhat related sequences (low stringency). Increasing the temperature or decreasing the salt concentration may tend to increase the selectivity of a hybridization reaction.

As used herein, the phrase "hybridizing under stringent hybridizing conditions" is preferably understood to refer to hybridizing under conditions of certain stringency. In a preferred embodiment the "stringent hybridizing conditions" are conditions where the two complementary sequences or sufficiently complementary sequences hybridize within the host cell, thus, under physiological conditions, in particular under conditions which are physiological to the host cell.

Sequences of a certain complementarity like the part of the gRNA complementary to the target site, or the complementary sequences which form the thermosensitive stem I of the HH ribozyme, are particularly hybridizing within the host cell, i.e. *in vivo* hybridizing. Therefore, the homology of the two nucleic acid sequences is specifically at least 70%, preferably at least 80%, preferably at least 90%, i.e. the double strand obtained during this hybridization comprises preferably at least 70%, preferably at least 80%, preferably at least 90% or 100% A-T bonds and C-G bonds.

The stringent or physiological conditions and the respective stringency can be determined by those skilled in the art, e.g. as described in Sambrook et al. (Molecular Cloning: A Laboratory Manual, Cold Spring Harbor, 1989).

The term "isolated" or "isolation" as used herein with respect to a nucleic acid, e.g. an isolated pre-gRNA; gRNA, an isolated constant part of the gRNA, an isolated tracrRNA or crRNA, or an isolated protein, e.g. an isolated RNA-guided endonuclease, or an isolated functional pair, such as an isolated pair or complex of a gRNA or a tracrRNA associated or bound to the RNA-guided endonuclease, shall refer to such compound that has been sufficiently separated from the environment with which it would naturally be associated, so as to exist in "substantially pure" form. "Isolated" does not necessarily mean the exclusion of artificial or synthetic mixtures with other compounds or materials, or the presence of impurities that do not interfere with the fundamental activity, and that may be present, for example, due to incomplete purification. In particular, isolated nucleic acid molecules as described herein are also meant to include those chemically synthesized.

Nucleic acids as described herein are specifically provided as "isolated nucleic acid" or as an "isolated nucleic acid sequence". This term, when applied to RNA or DNA, refers to a molecule that is separated from sequences with which it is immediately contiguous in the naturally-occurring organism. For example, an "isolated nucleic acid" may comprise a DNA molecule inserted into a vector, such as a plasmid, to express the respective gRNA encoded by such DNA. An "isolated nucleic acid" (either DNA or RNA) may further represent a molecule produced directly by biological or synthetic means and separated from other components present during its production.

An isolated RNA-guided endonuclease is typically provided as a molecule isolated from a natural source, e.g. a bacterial cell culture, or provided as a recombinant molecule obtained from a recombinant host cell culture, or provided as artificial product obtained by a suitable method of synthesis. Such isolation typically involves suitable methods of purification, e.g. to obtained a purity of at least 80%, preferably at least 90% or at least 95%, up to 100% (w/w).

The term "protein of interest (POI)" as used herein refers to a polypeptide or a protein that is produced by means of recombinant technology in a host cell, i.e. a recombinant POI. More specifically, the protein may either be a polypeptide not naturally-occurring in the host cell, i.e. a heterologous protein, or else may be native to the host cell, i.e. a homologous protein to the host cell, but is produced, for example, by transformation with a self-replicating vector containing the nucleic acid sequence encoding the POI, or upon integration by recombinant techniques of one or more copies of the nucleic acid sequence encoding the POI into the genome of the host cell, or by recombinant modification of one or more regulatory sequences controlling the expression of the gene encoding the POI, e.g. of the promoter sequence. In some cases the term POI as used herein also refers to any metabolite product by the host cell as mediated by the recombinantly expressed protein.

The POI can be any eukaryotic, prokaryotic or synthetic polypeptide. It can be a secreted protein or an intracellular protein. The present invention also provides for the recombinant production of functional homologs, functional equivalent variants, derivatives and biologically active fragments of naturally occurring proteins. Functional homologs are preferably identical with or correspond to and have the functional characteristics of a sequence.

A POI referred to herein may be a product homologous to the eukaryotic host cell or heterologous, preferably for therapeutic, prophylactic, diagnostic, analytic or industrial use.

The POI is preferably a heterologous recombinant polypeptide or protein, produced in a eukaryotic cell, preferably a yeast cell, preferably as secreted proteins. Examples of preferably produced proteins are immunoglobulins, immunoglobulin fragments, aprotinin, tissue factor pathway inhibitor or other protease inhibitors, and insulin or insulin precursors, insulin analogues, growth hormones, interleukins, tissue plasminogen activator, transforming growth factor a or b, glucagon, glucagon-like peptide 1 (GLP-1), glucagon-like peptide 2 (GLP-2), GRPP, Factor VII, Factor VIII, Factor XIII, platelet-derived growth factor1, serum albumin, enzymes, such as lipases or proteases, or a functional homolog, functional equivalent variant, derivative and biologically active fragment with a similar function as the native protein. The POI may be structurally similar to the native protein and may be derived from the native protein by addition of one or more amino acids to either or both the C- and N-terminal end or the side-chain of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

A POI can also be selected from substrates, enzymes, inhibitors or cofactors that provide for biochemical reactions in the host cell, with the aim to obtain the product of said biochemical reaction or a cascade of several reactions, e.g. to obtain a metabolite of the host cell. Exemplary products can be vitamins, such as riboflavin, organic acids, and alcohols, which can be obtained with increased yields following the expression of a recombinant protein or a POI as described herein.

The term "heterologous" as used herein with respect to a nucleotide or amino acid sequence or protein, refers to a compound which is either foreign, i.e. "exogenous", such as not found in nature, to a given host cell; or that is naturally found in a given host cell, e.g., is "endogenous", however, in the context of a heterologous construct, e.g. employing a heterologous nucleic acid. The heterologous nucleotide sequence as found endogenously may also be produced in an unnatural, e.g. greater than expected or greater than naturally found, amount in the cell. The heterologous nucleotide sequence, or a nucleic acid comprising the heterologous nucleotide sequence, possibly differs in sequence from the endogenous nucleotide sequence but encodes the same protein as found endogenously. Specifically, heterologous nucleotide sequences are those not found in the same relationship to a host cell in nature. Any recombinant or artificial nucleotide sequence engineered to transform a particular host cell is understood to be heterologous to the host cell. An example of a heterologous polynucleotide is a nucleotide sequence not natively associated with the gene to be expressed, e.g. a heterologous gene encoding the POI which expression is

- positively regulated by the altered GOI expression, i.e. with an increased yield of POI production when the GOI is altered by the method as described herein; and/or
- negatively regulated by the parent (non-altered) GOI expression, i.e. with a reduced yield of POI production when the GOI is not altered by the method as described herein.

A specific example of a heterologous compound is a POI encoding gene or polynucleotide, to which endogenous, naturally-occurring genetic or regulatory elements of the host cell is not normally operably linked.

It is understood that the method disclosed herein may include cultivating a recombinant host cell line under conditions permitting the expression of the POI, preferably in the secreted form or else as intracellular product. A recombinantly produced POI or a host cell metabolite can then be isolated from the cell culture medium and further purified by techniques well known to a person skilled in the art.

The POI produced as described herein typically can be isolated and purified using state of the art techniques, including the increase of the concentration of the desired POI and/or the decrease of the concentration of at least one impurity.

If the POI is secreted from the cells, it can be isolated and purified from the culture medium using state of the art techniques. Secretion of the recombinant expression products from the host cells is generally advantageous for reasons that include facilitating the purification process, since the products are recovered from the culture supernatant rather than from the complex mixture of proteins that results when yeast cells are disrupted to release intracellular proteins.

The cultured transformant cells may also be ruptured sonically or mechanically, enzymatically or chemically to obtain a cell extract containing the desired POI, from which the POI is isolated and purified.

As isolation and purification methods for obtaining a recombinant polypeptide or protein product, methods, such as methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be used.

The highly purified product is essentially free from contaminating proteins, and preferably has a purity of at least 90%, more preferred at least 95%, or even at least 98%, up to 100%. The purified products may be obtained by purification of the cell culture supernatant or else from cellular debris.

As isolation and purification methods the following standard methods are preferred: Cell disruption (if the POI is obtained intracellularly), cell (debris) separation and wash by Microfiltration or Tangential Flow Filter (TFF) or centrifugation, POI purification by precipitation or heat treatment, POI activation by enzymatic digest, POI purification by chromatography, such as ion exchange (IEX), hydrophobic interaction chromatography (HIC), Affinity chromatography, size exclusion (SEC) or HPLC Chromatography, POI precipitation of concentration and washing by ultrafiltration steps.

The POI may specifically be recovered from the cell culture in the purified form, e.g. substantially pure. The isolated and purified POI can be identified by conventional methods such as Western blot, HPLC, activity assay, or ELISA.

The term "substantially pure" or "purified" as used herein shall refer to a preparation comprising at least 50% (w/w), preferably at least 60%, 70%, 80%, 90% or 95% of a compound, such as a nucleic acid molecule or a POI. Purity is measured by methods appropriate for the compound (e.g. chromatographic methods, polyacrylamide gel electrophoresis, HPLC analysis, and the like).

The term "recombinant" as used herein shall mean "being prepared by or the result of genetic engineering". Thus, a "recombinant microorganism" comprises at least one "recombinant nucleic acid". A recombinant microorganism specifically comprises an expression vector or cloning vector, or it has been genetically engineered to contain a recombinant nucleic acid sequence. A "recombinant protein" is produced by expressing a respective recombinant nucleic acid in a host.

In general, the recombinant nucleic acids or organisms as referred to herein may be produced by recombination techniques well known to a person skilled in the art. In accordance with the present invention there may be employed conventional molecular biology, microbiology, and recombinant DNA techniques within the skill of the art. Such techniques are explained fully in the literature. See, e.g., Maniatis, Fritsch & Sambrook, "Molecular Cloning: A Laboratory Manual, Cold Spring Harbor, (1982).

Expression vectors may include but are not limited to cloning vectors, modified cloning vectors and specifically designed plasmids. The preferred expression vector as used herein may be any expression vector suitable for expression of a recombinant gene in a host cell and is selected depending on the host organism. The recombinant expression vector may be any vector which is capable of replicating in or integrating into the genome of the host organisms, also called host vector.

For recombinant POI production, appropriate expression vectors typically comprise regulatory sequences suitable for expressing DNA encoding a POI in a eukaryotic host cell. Examples of regulatory sequences include promoter, operators, enhancers, ribosomal binding sites, and sequences that control transcription and translation initiation and termination. The regulatory sequences are typically operably linked to the DNA sequence to be expressed. To allow expression of a recombinant nucleotide sequence in a host cell, the expression vector specifically provides a promoter adjacent to the 5' end of the coding sequence, e.g. upstream from the gene encoding the POI or a signal peptide gene enabling secretion of the POI. The transcription is thereby regulated and initiated by this promoter sequence.

The POI can be produced by cultivating the recombinant host cell line transformed with the expression vector in an appropriate medium, isolating the expressed product or metabolite from the culture, and optionally purifying it by a suitable method.

Transformants as described herein can be obtained, e.g. by introducing an expression vector or plasmid into the host cell and selecting transformants which express the POI or the host cell metabolite with high yields. Host cells are treated to enable them to incorporate foreign DNA by methods conventionally used for transformation of eukaryotic cells. Preferred methods of transformation for the uptake of the recombinant DNA fragment by the eukaryotic host cells include chemical transformation, electroporation or transformation by protoplastation.

There are several different preferred approaches for the production of the POI as described herein. Substances may be expressed, processed and optionally secreted by transforming a eukaryotic host cell with an expression vector harboring recombinant DNA encoding a relevant protein and at least one of the regulatory elements as described above, preparing a culture of the transformed cell, growing the culture, inducing transcription and POI production, and recovering the product of the fermentation process. The host cell as described herein is specifcially tested for its expression capacity or yield upon activating the pre-gRNA which initiates the production phase, e.g. as an in process control. Therefore, the following tests may be used: ELISA, activity assay, HPLC, or other suitable tests.

The foregoing description will be more fully understood with reference to the following examples. Such examples are, however, merely representative of methods of practicing one or more embodiments of the present invention and should not be read as limiting the scope of invention.

## EXAMPLES

### Example 1: Plasmid preparation containing a thermosensitive pre-gRNA element for the expression in *Aspergillus niger* targeting a specific locus in the genome

As a GOI the *A. niger* gene 214233 (JGI assembly v 3.0 (June 30, 2008)) or An04g06920 was selected. A 23 bp sequence (SEQ ID 11) was selected in the coding sequence of the gene which contained a PAM site on its 3'end having the sequence 5'- NGG and is named target sequence (TS). The first 6 bp from the 5'end of the TS (sequence: TGGCTC) contained 4 G or C residues and only 2 A or T residues.

In order to construct the pre-guide RNA, the reverse complementary sequence of these 6 bp (sequence: GAGCCA) was fused on the 5'end of a hammerhead ribozyme (SEQ ID 12). On the 3'end of the HH ribozyme the first 20 bp (TS without PAM, SEQ ID 15) of the TS were fused followed by the core gRNA sequence and the HDV ribozyme. This sequence constitutes thermosensitive pre-gRNA of the target locus 214233. In order to produce the RNA *in vivo,* a DNA plasmid is constructed, which serves as a transcriptional template. The plasmid contains the pre-gRNA, which is flanked by the fungal pmbfA promoter *(A. niger)* (Blumhoff M, et al. 2013. Appl. Microbiol. Biotechnol. 97: 259-67.) and the trpC terminator *(A. nidulans).* The Cas9 coding sequence was obtained from plasmid #43802 from addgene (SEQ ID 16) The Cas9 gene is under control of the fungal coxA promoter *(A. niger)* (Blumhoff M, et al. 2013. Appl. Microbiol. Biotechnol. 97: 259-67.) and is terminated by the cyc terminator of S *cerevisiae.* The expression plasmids were constructed following a modular cloning system (Engler et al., 2009; Werner et al., 2012). Single DNA fragments were amplified by PCR removing internal Bbsl and Bsal with mutated primers. Bbsl restriction sites were fused to the ends of each fragment by means of PCR using extended primers. Multiple DNA fragments were assembled in a one-pot reaction in presence of a type IIS restriction enzyme (Bbsl for level1 or Bsal for level2, New England Biolabs), T4 ligase (New England Biolabs) and T4 ligase buffer. The assembly was incubated allowing 45 cycles of restriction and ligation (respectively, 37 °C, 1 min and 16 °C, 2.5 min). Obtained plasmids were cloned into E. coli DH10B and isolated according to standard methods (Sambrook, J., Russel, D., 2001. Molecular cloning: a laboratory manual, 3 rd. ed. Coldd Spring Harbor Laboratory Press, New York.).

*Escherichia coli* DH10B was used as host for DNA manipulation. *E. coli* transformants were grown on LB (lysogeny broth, Sambrook and Russel, 2001) supplemented with 50 μg/ml kanamycine, 50 μg/mL ampicillin or 100 μg/ml hygromycin B.

The final plasmid containing the pre-guide RNA for the DNA TS 214233 was named MST623 and was selected on LB plates supplemented with hygromycin B. The respective plasmid sequence is given in file (SEQ ID 17).

It is also possible to obtain the before mentioned DNA sequence by partial or total gene synthesis which is commercially provided.

### Example 2: Generation of conditional gene deletion strains in *A. niger*

*Aspergillus niger* JB1 was used as parental strain. JB1 is an industrial derivative of the strain ATCC 1015 obtained by classical UV mutagenesis and selection for high citric acid secretion (Pelechová, J. et al. 1990 Folia Microbiol. (Praha). 35, 138-42.). Protoplast transformation of *A. niger* was performed according to the previously described method (Arentshorst et al., 2012). Briefly, protoplasts are generated by incubation in the presence of lysing enzymes (Sigma Aldrich, Lysing enzymes form *Trichoderma harzianum,* L1412) in an osmotically stabilized buffer. Protoplasts are thereafter washed and separated by centrifugation at 2000 RCF, 4°C. The transformation of DNA into the protoplasts is achieved in the presence of PEG6000. In total 1-10μg plasmid DNA are needed for the transformation using about 10^6 protoplasts. Selection of transformants was achieved on osmotically stabilized MMS plates supplemented with 150 μg/mL hygromycin B (MMSH). All transformants were purified by single colony isolation on the selection medium at least twice.

Transformed protoplasts were plated on MMSH (minimal medium saccharose hygromycin) plates and incubated on <25 °C, 30°C or 37°C. In addition the control pMST621 was transformed and transformed protoplasts were plated on MMSHU (minimal medium saccharose hygromycin uridin) plates at 30°C. Individual transformants were obtained and purified twice on MMH (minimal medium hygromycin) plates. The successful CRISPR deletion was verified by PCR after DNA extraction (Arentshorst et al., 2012). The transformants were cultivated in liquid malt extract agar (3%) at <25 °C, 30°C or 37°C C in an orbital shaker at 150rpm. Mycelia was harvested after 24 hour. DNA was extracted from the transformants (Arentshorst et al., 2012) and the sequence of the target locus was amplified by PCR using the primer check_fw (SEQ ID 13 and check_rev (SEQ ID 14) The occurrence of insertions or deletions on the target locus was checked by sequencing. The obtained PCR product was sent for sequencing at a commercial sequencing service (Microsynth AG).

In Table 2 frequency of finding a gene deletion on the respective target locus in individual transformants is shown. At temperatures around 25°C no mutations can be found using the temperature inducible pre-guide RNA. At 30°C about 50% of the analyzed transformants carry a MOI at the respective target site. The gene deletion frequency at 37°C reaches up to 100%. However, at this elevated temperature the optimal growth temperature of the fungal cell exceeded and less transformants are obtained.

Table 2 Gene deletion efficiency at different temperatures using a temperature inducible gene deletion system. Percentage of successful gene deletions verified by sequencing of the respective gene locus. The obtained MOI were small insertions or deletions adjacent to the PAM site of the target locus.

| Temperature | MOI frequency in transformed strains | MOI frequency in transformed strains % |
|---|---|---|
| <25°C | 0 from 8 tested | 0% |
| 30°C | 8 from 15 tested | 53% |
| 37 | 1 from 1 tested | 100 / |

Example 3: Conditional gene deletions in *Aspergillus niger* deletion after induction of a stable transformant

As described in Example 1, *A. niger* transformant strains were obtained after transformation with plasmid pMST623. Eight strains which were kept at 25°C had no MOI on the respective target locus. Three of these strains were selected and conidia were obtained on MMH plates. Conidia of the fungus were plated on fresh MMH plates and the plates were separately incubated on 25°C, 30°C or 37°C for at least 5 days or until the conidia formation was visible.

The strains obtained after this temperature induction step were purified in two consecutive steps on MMH plates and were further incubated on 25°C, 30°C or 37°C. The transformants were cultivated in liquid malt extract agar (3%) at <25 °C, 30°C or 37°C in an orbital shaker at 150rpm. Mycelia was harvested after 24 hour. DNA was extracted from the transformants (Arentshorst et al., 2012) and the sequence of the target locus was amplified by PCR using the primer check_fw (SEQ ID 13) and check_rev (SEQ ID 14). The occurrence of insertions or deletions on the target locus was checked by sequencing. The obtained PCR product was sent for sequencing at a commercial sequencing service (Microsynth AG).

No mutations were found after an induction temperature of <25°C. At 30°C two strains carried a MOI and at 37°C all three strains carried a MOI (Table 3). This result shows that a strain which is cultivated at temperatures below 25°C is stable in respect to the GOI, but can be mutated by shifting the temperature to values >30°C. This strain is thus referred to as a (temperature inducible) conditional deletion strain.

Table 3: Gene deletion efficiency at different temperatures using a temperature inducible gene deletion system. Percentage of successful gene deletions verified by sequencing of the respective gene locus. The obtained MOI were small insertions or deletions adjacent to the PAM site of the target locus.

| Temperature | MOI frequency in temperature induced strains | MIC frequency in temperature induced strains % |
|---|---|---|
| <25°C | 0 from 3 tested | 0% |
| 30°C | 2 from 3 tested | ~66% |
| 37 | 3 from 3 tested | 100 % |

Example 4: Finding other DNA targeting site which are temperature sensitive by modifying the stem I region of the hammerhead ribozyme

To create more gRNA, which are temperature sensitive, the stem loop I region of the hammerhead ribozyme is altered. This can be achieved by the selection of a TS, which contains:

a) More or equal than 4 G or C residues in first 6 bp of the 20bp sequence
b) Or by an elongation of the stem I region (>6bp) so that the melting temperature >= 20°C
c) The sequence of the gRNA starts with NNNCUC, wherein N is any of A, G, C, or U.

The melting temperature (Tm) of the stem I is thus calculated by assigning 2°C to each A-U pair and 4°C to each G-C pair and forming the sum out of each residue, which contributes to the stem loop, as shown below.

Equation 1: Calculation melting temperature

$$Tm\ [°C] = 4[°C] * \#(GC) + 2[°C] * \#(AU)$$

#(GC) number of GC pairs in sequence
#(AU) number of AU pairs in sequence

The calculated melting temperature resembles the melting temperature of the stem I of the hammerhead ribozyme. The respective working temperatures (first working temperature and second working temperature) may differ from the melting temperature because it is dependent on the microbial host and the respective applied salt concentration. Based on the calculated melting temperature, the working temperatures with inactive pre-gRNA and for activating the gRNA can be determined in the appropriate host cell. The first working temperature according to example 3, where the pre-gRNA is not cleaved, is 20°C and the second working temperature, which activates the pre-gRNA by self-cleaving, is 37°C.

Example 5: Using a modified four U stem/loop III for a hammerhead ribozyme to create a temperature sensitive pro-guide RNA

Another option to create a thermosensitive pre-gRNA is presented. This design allows to targeted virtual any 20 bp region with the gRNA/Cas9 complex and is thus sequence independent. A hammerhead ribozyme is created which has a modified stem/loop III region. It is replaced by the Stem/ loop III fourU, which is known to be temperature sensitive. The sequence of the constant part of the hammerhead backbone is depicted in SEQ ID 4. The sequence of the hammerhead ribozyme fused to the gRNA has a sequence as depicted in SEQ ID 9, whereas the positions marked with N a variable (wherein N is any of A, G, C, or U) and are only defined by the targeting locus. The hammerhead ribozyme in this setup is inactive at temperatures > 35°C and active at temperatures below 30°C (Saragliadis, 2013, RNAbiology, 10, 1010-1016).

Example 6: Activation of gene drives with a temperature inducible gRNA

CRISPR Cas9 enables the development of gene drives which have the capability to genetically modify entire populations of a species like the mosquito *Anopheles stephensi,* a vector of malaria (Gantz et al. 2015 Proc. Natl. Acad. Sci. 201521077. doi:10.1073/pnas.1521077112). In order to control the spread of a gene drive it might be beneficial to link it to external parameters like ambient temperature. A temperature inducible gRNA allows to control the spreading of a gene drive depending on the ambient temperature of *Anopheles stephensi.* Only at temperatures above 30°C the respective gene drive is propagated throughout the population. The gRNA sequence in Gantz et al. needs to be replaced by SEQ ID 18 in order to obtain a functional temperature sensitive gRNA, which is inactive at temperatures below 30°C.

Example 7: Decoupling of the biomass formation phase and the production phase

The temperature inducible element is used to decouple the biomass formation phase in a fermentation from the phase were only the product is formed. This product can be itaconic acid and the respective organism is *E. coli* (expressing *cadA* ATEG_09971 from *A. terreus* under a constitutive promoter). As a TS for the temperature inducible CRISPR element a gene is selected, which is essential for biomass formation. This gene is isocitrate dehydrogenase (icd). The cells are transformed with a plasmid encoding a temperature sensitive pre-guide RNA targeting icd. Furthermore, a Λ Red recombineering system and donor template DNA to repair the introduce a desired frameshift mutation in icd gene is transformed to *E. coli* (Li, Y et al. 2015, Metab. Eng. 31, 13-2. The transformants are cultivated at 25°C to produce biomass until a desired optical density is reached. Then the temperature is shifted to 37°C inducing formation of mature gRNA and the gene deletion of icd. This will interrupt the circular action of the TCA cycle, leading to a reduction of the biomass formation rate and to an increased production of itaconic acid.

Example 8: Testing the efficiency of a temperature inducible CRISPR/Cas9 system in *Pichia pastoris* with flow cytometry

The temperature inducible CRISPR/Cas9 system as described in example 1 was introduced into *Pichia pastoris.* In order to have a reporter construct a green fluorescene protein (sGFP) was created containing the TS in its coding sequence. For this purpose the sGFP coding sequence was designed by replacing the sGFP start codon by the 23 bp of the TS sequence (SEQ ID 11) and adding an additional base pair A at the 5'end of the sequence. The respective codon sequence was assembled by a golden gate cloning procedure with a pGAP promoter of *Pichia pastoris* and a CYC1 terminator of S. *cerevisiae.* The respective cassette was integrated in single copy at the GAP locus in the *P. pastoris* genome. Transformation of *P. pastoris* by electroporation was performed following the standard protocol described in the Pichia manuals (Invitrogen). The respective strain was further transformed with a plasmid containing a pre-gRNA (Seq ID: 6) expressed under the control of a pGAP promoter controlling the temperature after transformation at 20 °C.

The obtained strain was grown at 20°C, 30°C and 37°C and the green fluorescence per cell was measured by flow cytometry.

References:

Arentshorst, M., Ram, A.F.J., Meyer, V., 2012. Using non-homologous end-joining-deficient strains for functional gene analyses in filamentous fungi. Methods Mol. Biol. 835, 133-50. doi:10.1007/978-1-61779-501-5_9

Blumhoff, M.L., Steiger, M.G., Marx, H., Mattanovich, D., Sauer, M., 2013. Six novel constitutive promoters for metabolic engineering of Aspergillus niger. Appl. Microbiol. Biotechnol. 97, 259-267. doi:10.1007/s00253-012-4207-9

Engler, C., Gruetzner, R., Kandzia, R., Marillonnet, S., 2009. Golden gate shuffling: a one-pot DNA shuffling method based on type IIs restriction enzymes. PLoS One 4, e5553. doi:10.1371/journal.pone.0005553

Gao, Y., Zhao, Y., 2014. Self-processing of ribozyme-flanked RNAs into guide RNAs in vitro and in vivo for CRISPR-mediated genome editing. J. Integr. Plant Biol. 56, 343-349. doi:10.1111/jipb.12152

Jennifer A. DOUDNA, Martin Jinek, Emmanuelle Charpentier, Krzysztof Chylinski, James Harrison Doudna Cate, Wendell Lim, Lei Qi, W.«., n.d. Methods and compositions for rna-directed target dna modification and for rna-directed modulation of transcription. US20140068797.

Nodvig, C.S., Nielsen, J.B., Kogle, M.E., Mortensen, U.H., 2015. A CRISPR-Cas9 System for Genetic Engineering of Filamentous Fungi. PLoS One 10, e0133085. doi:10.1371/journal.pone.0133085

Sambrook, J., Russel, D., 2001. Molecular cloning: a laboratory manual, 3 rd. ed. Coldd Spring Harbor Laboratory Press, New York.

Saragliadis, A., Krajewski, S.S., Rehm, C., Narberhaus, F., Hartig, J.S., 2013. Thermozymes: Synthetic RNA thermometers based on ribozyme activity. RNA Biol. 10, 1010-6. doi:10.4161/rna.24482

Werner, S., Engler, C., Weber, E., Gruetzner, R., Marillonnet, S., 2012. Fast track assembly of multigene constructs using golden gate cloning and the MoClo system. Bioeng. Bugs 3, 38-43. doi:10.4161/bbug.3.1.18223

Zetsche, B., Gootenberg, J.S., Abudayyeh, O.O., Slaymaker, I.M., Makarova, K.S., Essletzbichler, P., Volz, S.E., Joung, J., van der Oost, J., Regev, A., Koonin, E. V., Zhang, F., 2015. Cpf1 Is a Single RNA-Guided Endonuclease of a Class 2 CRISPR-Cas System. Cell 163, 759-771. doi:10.1016/j.cell.2015.09.038

## Claims

**1.** A method of producing a mutant host cell with altered expression of a gene of interest (GOI) by a temperature-inducible CRISPR/Cas system, which comprises:

a) at a first working temperature, introducing into a host cell a CRISPR/Cas system employing

i) an inactive pre-guide RNA (pre-gRNA) which is a ribozyme-extended guide RNA comprising a guide RNA (gRNA) that recognizes a predetermined target site at the GOI, terminally extended by at least one self-processing Hammerhead (HH) ribozyme **characterized by** the presence of a thermosensitive hairpin and a cleavage site adjacent to the gRNA; and
ii) a RNA-guided Cas endonuclease which recognizes the target site upon hybridizing with the gRNA;

b) increasing the first working temperature to a second working temperature, thereby changing the structure of the thermosensitive hairpin and activating the pre-gRNA by self-catalyzed cleavage at the cleavage site to obtain the gRNA, which induces binding of the endonuclease to the target site, thereby altering expression of the GOI.

**2.** The method of claim 1, wherein the endonuclease catalyzes a DNA break at the target site upon hybridizing with the gRNA and the GOI expression is altered by cellular repair mechanisms induced by a DNA break, thereby introducing a mutation of interest (MOI) at the GOI, preferably a MOI comprising at least one frameshift mutation, insertion, substitution, and/or deletion of one or more nucleotides impairing the open reading frame (ORF) of the GOI.

**3.** The method of claim 1 or 2, wherein said HH ribozyme comprises

a) a HH ribozyme constant region;
b) a variable 3'-terminal nucleotide sequence of at least 3 nucleotides; and
c) a complementary 5'-terminal sequence hybridizing to the 3'-terminal nucleotide sequence, thereby obtaining a thermosensitive stem I of at least 3 complementary base pairs,

preferably, wherein the 3'-terminal nucleotide sequence of the HH ribozyme forms the 5'-terminus of the gRNA upon cleaving the pre-gRNA; and the 5'-terminal nucleotide sequence of the HH ribozyme is hybridizing thereto.

**4.** The method of claim 3, wherein the HH ribozyme constant region comprises a nucleotide sequence identified as SEQ ID 5, preferably any of the nucleotide sequences identified as SEQ ID 1, SEQ ID 2, SEQ ID 3, or SEQ ID 4, or a functional variant of any of the foregoing with at least 70 % sequence identity.

**5.** The method of any of claims 1 to 4, wherein the pre-gRNA comprises any of the nucleotide sequences identified as SEQ ID 6, SEQ ID 7, SEQ ID 8, or SEQ ID 9, or a functional variant of any of the foregoing with at least 70 % sequence identity.

**6.** The method of any of claims 1 to 8, wherein the pre-gRNA further comprises a hepatitis delta virus type (HDV) ribozyme as a further terminal extension of the gRNA sequence opposite to the extension by the HH ribozyme, preferably wherein the HDV ribozyme comprises the nucleotide sequence identified as SEQ ID 10, or a functional variant thereof with at least 70 % sequence identity.

**7.** The method of any of claims 1 to 10, wherein the first working temperature is any below 30 °C, and the second working temperature is any above 30°C.

**8.** The method of any of claims 1 to 7, wherein the host cell is cultivated in a fed-batch culture, wherein the host cell is first cultivated at the first working temperature during the growth phase, followed by increasing the first working temperature to the second working temperature thereby activating the pre-gRNA and altering the expression of the GOI in the production phase.

**9.** The method of any of claims 1 to 8, wherein the host cell is any of a bacterial, filamentous fungi, yeast, mammalian, or avian host cell.

**10.** The method of any of claims 1 to 9, wherein the endonuclease is selected from the group consisting of Cas9 enzymes originating from any of *Streptococcus pyogenes, Streptococcus thermophiles, Neisseria Meningitis* or *Treponema Denticola,* or the Cpf1 enzyme originating from *Acidaminococcus sp.* or *Lachnospiraceae bacterium,* and functional variants of any of the foregoing, including Cas9 nickases or artificial enzymes.

**11.** The method of any of claims 1 to 10, wherein the GOI is selected from the group consisting of genes encoding regulatory proteins of the host cell, preferably metabolic enzymes, or proteins involved in cell cycle arrest, preferably any of the regulatory proteins listed in Table 1, or analogues thereof naturally-occurring in the host cell.

**12.** A method of producing a protein of interest (POI) in a host cell transformed with the temperature-inducible CRISPR/Cas system as defined in claim 1, wherein the host cell comprises a gene encoding the POI regulated by the GOI expression product, comprising

a) growing the host cell under growth conditions at the first working temperature thereby obtaining a cell culture;
b) elevating said first working temperature in the cell culture to the second working temperature, thereby altering expression of the GOI and initiating the production of the POI; and
c) isolating the POI from the cell culture,

preferably wherein the POI is a heterologous protein, preferably selected from therapeutic proteins, including antibodies or fragments thereof, enzymes and peptides, protein antibiotics, toxin fusion proteins, carbohydrate - protein conjugates, structural proteins, regulatory proteins, vaccines and vaccine like proteins or particles, process enzymes, growth factors, hormones and cytokines, or a metabolite of a POI.

**13.** A host cell transformed with the temperature-inducible CRISPR/Cas system as defined in claim 1, wherein the host cell comprises a gene encoding a heterologous POI regulated by the GOI expression product.

**14.** A thermosensitive HH ribozyme, which comprises or consists of

a) a HH ribozyme constant region, preferably consisting of the nt sequence identified as SEQ ID 5; which is terminally extended by
b) at least a variable 3'-terminal nucleotide sequence of at least 3 nucleotides; and
c) a complementary 5'-terminal nucleotide sequence hybridizing to the 3'-terminal nucleotide sequence, thereby obtaining a thermosensitive stem I,

wherein the stem I is **characterized by** at least any of

i. the hybridizing part of the variable 3'-terminal nucleotide sequence of b) consists of at least 6 nucleotides and comprises at least four G or C residues;
ii. the hybridizing part of the variable 3'-terminal nucleotide sequence of b) comprises or consists of at least 6 nucleotides having the sequence NNNCUC, wherein N is any of A, G, C, or U; or
iii. a length of at least 7 bp, or less than 6 bp.

**15.** A pre-gRNA, which comprises the thermosensitive HH ribozyme of claim 14.

Fig. 1

SEQ ID1: CUGAUGAGUCCGUGAGGACGAAACGAGUAAGCUCGUC

SEQ ID 2: CUGAUGAGGCCGAAAGGCCGAAAACCGUUAAGACGGUC

SEQ ID 3: CUGAUGAGGCCGAAAACCGUUGAACUUUUGAAUAGUGAUUCAGGAGGUUAAAAUUGCUC

SEQ ID4:

CUGAUGAGUCCGUGAGGACGAAAACCGUUGAACUUUUGAAUAGUGAUUCAGGAGGUUAAAAUUGCUC

SEQ ID 5: CUGAUGARNNNNNNNNNNYGAAANNNNNNNNNNNNUC

Wherein R at position 8 is either nucleotide A or G, and
Y at position 19 is either nucleotide C or T, and
R at position 8 is complementary to Y at position 19;
N at position 9 to 18 is any nucleotide, and
N at position 9 is complementary to N at position 18, and
N at position 10 is complementary to N at position 17, and
N at position 11 is complementary to N at position 16;
N at position 12 to 15 is any nucleotide, optionally extended by up to 10 nucleotides;
N at position 24 to 35 is any nucleotide, and
N at position 24 is complementary to N at position 35, and
N at position 25 is complementary to N at position 34, and
N at position 26 is complementary to N at position 33, and
N at position 27 is complementary to N at position 32;
N at position 28 to 31 is any nucleotide, optionally extended by up to 10 nucleotides.

SEQ ID 6:
GAGCCACUGAUGAGUCCGUGAGGACGAAACGAGUAAGCUCGUCUGGCUCGCUCCAUAAGCCAGGUUUUAG
AGCUAGAAAUAGCAAGUUAAAAUAAGGCUAGUCCGUUAUCAACUUGAAAAAGUGGCACCGAGUCGGUGCU
UUU

SEQ ID 7:
GAGNNNCUGAUGAGUCCGUGAGGACGAAACGAGUAAGCUCGUCNNNCTCNNNNNNNNNNNNNNGUUUU
AGAGCUAGAAAUAGCAAGUUAAAAUAAGGCUAGUCCGUUAUCAACUUGAAAAAGUGGCACCGAGUCGGUG
CUUUU

Wherein N at position 4 to 6 is any nucleotide, and
N at position 44 to 46 is any nucleotide, and
N at position 4 is complementary to N at position 46, and
N at position 5 is complementary to N at position 45, and
N at position 6 is complementary to N at position 44;
N at position 50 to 63 is any nucleotide.

Fig. 1

Continued

SEQ ID 8:
NNNNNNCUGAUGAGUCCGUGAGGACGAAACGAGUAAGCUCGUCNNNNNNNNNNNNNNNNNNNNGUUU
UAGAGCUAGAAAUAGCAAGUUAAAAUAAGGCUAGUCCGUUAUCAACUUGAAAAAGUGGCACCGAGUCGGU
GCUUUU

Wherein N at position 1 to 6 is any nucleotide, and
N at position 44 to 63 is any nucleotide, and
N at position 1 is complementary to N at position 49, and
N at position 2 is complementary to N at position 48, and
N at position 3 is complementary to N at position 47, and
N at position 4 is complementary to N at position 46, and
N at position 5 is complementary to N at position 45, and
N at position 6 is complementary to N at position 44;
N at position 50 to 63 is any nucleotide.

SEQ ID 9:
NNNNNNCUGAUGAGUCCGUGAGGACGAAAACCGUUGAACUUUUGAAUAGUGAUUCAGGAGGUUAAAAUU
GCUCNNNNNNNNNNNNNNNNNNNNGUUUUAGAGCUAGAAAUAGCAAGUUAAAAUAAGGCUAGUCCGUU
AUCAACUUGAAAAAGUGGCACCGAGUCGGUGCUUUU

Wherein N at position 1 to 6 is any nucleotide, and
N at position 74 to 93 is any nucleotide, and
N at position 1 is complementary to N at position 79, and
N at position 2 is complementary to N at position 78, and
N at position 3 is complementary to N at position 77, and
N at position 4 is complementary to N at position 76, and
N at position 5 is complementary to N at position 75, and
N at position 6 is complementary to N at position 74.

SEQ ID 10:
GGCCGGCAUGGUCCCAGCCUCCUCGCUGGCGCCGGCUGGGCAACAUGCUUCGGCAUGGCGAAUGGGACAG
CUUUGGAC

SEQ ID 11: TGGCTCGCTCCATAAGCCAGAGG

SEQ ID 12: CTGATGAGTCCGTGAGGACGAAACGAGTAAGCTCGTC

SEQ ID 13: CCATTCTCATCAAGGATTACGC

SEQ ID 14: GAATTGTGCTGCACTTTTGAAG

SEQ ID 15: TGGCTCGCTCCATAAGCCAG

Fig. 1

Continued

SEQ ID 16 (2 pages):
ATGGACAAGAAGTACTCCATTGGGCTCGATATCGGCACAAACAGCGTCGGTTGGGCCGTCATTACGGACGAGTA
CAAGGTGCCGAGCAAAAAATTCAAAGTTCTGGGCAATACCGATCGCCACAGCATAAAGAAGAACCTCATTGGCG
CCCTCCTGTTCGACTCCGGGGAGACGGCCGAAGCCACGCGGCTCAAAAGAACAGCACGGCGCAGATATACCCGC
AGAAAGAATCGGATCTGCTACCTGCAGGAGATCTTTAGTAATGAGATGGCTAAGGTGGATGACTCTTTCTTCCAT
AGGCTGGAGGAGTCCTTTTTGGTGGAGGAGGATAAAAAGCACGAGCGCCACCCAATCTTTGGCAATATCGTGGA
CGAGGTGGCGTACCATGAAAAGTACCCAACCATATATCATCTGAGGAAGAAGCTTGTAGACAGTACTGATAAGG
CTGACTTGCGGTTGATCTATCTCGCGCTGGCGCATATGATCAAATTTCGGGGACACTTCCTCATCGAGGGGGACC
TGAACCCAGACAACAGCGATGTCGACAAACTCTTTATCCAACTGGTTCAGACTTACAATCAGCTTTTCGAAGAGA
ACCCGATCAACGCATCCGGAGTTGACGCCAAAGCAATCCTGAGCGCTAGGCTGTCCAAATCCCGGCGGCTCGAA
AACCTCATCGCACAGCTCCCTGGGGAGAAGAAGAACGGCCTGTTTGGTAATCTTATCGCCCTGTCACTCGGGCTG
ACCCCCAACTTTAAATCTAACTTCGACCTGGCCGAAGATGCCAAGCTTCAACTGAGCAAAGACACCTACGATGAT
GATCTCGACAATCTGCTGGCCCAGATCGGCGACCAGTACGCAGACCTTTTTTTGGCGGCAAAGAACCTGTCAGAC
GCCATTCTGCTGAGTGATATTCTGCGAGTGAACACGGAGATCACCAAAGCTCCGCTGAGCGCTAGTATGATCAA
GCGCTATGATGAGCACCACCAAGACTTGACTTTGCTGAAGGCCCTTGTCAGACAGCAACTGCCTGAGAAGTACA
AGGAAATTTTCTTCGATCAGTCTAAAAATGGCTACGCCGGATACATTGACGGCGGAGCAAGCCAGGAGGAATTT
TACAAATTTATTAAGCCCATCTTGGAAAAAATGGACGGCACCGAGGAGCTGCTGGTAAAGCTTAACAGAGAAGA
TCTGTTGCGCAAACAGCGCACTTTCGACAATGGAAGCATCCCCCACCAGATTCACCTGGGCGAACTGCACGCTAT
CCTCAGGCGGCAAGAGGATTTCTACCCCTTTTTGAAAGATAACAGGGAAAAGATTGAGAAAATCCTCACATTTCG
GATACCCTACTATGTAGGCCCCCTCGCCCGGGGAAATTCCAGATTCGCGTGGATGACTCGCAAATCAGAAGAGA
CtATCACTCCCTGGAACTTCGAGGAAGTCGTGGATAAGGGGGCCTCTGCCCAGTCCTTCATCGAAAGGATGACTA
ACTTTGATAAAAATCTGCCTAACGAAAAGGTGCTTCCTAAACACTCTCTGCTGTACGAGTACTTCACAGTTTATAA
CGAGCTCACCAAGGTCAAATACGTCACAGAAGGGATGAGAAAGCCAGCATTCCTGTCTGGAGAGCAGAAGAAA
GCTATCGTGGACCTCCTCTTCAAGACGAACCGGAAAGTTACCGTGAAACAGCTCAAAGAgGACTATTTCAAAAAG
ATTGAATGTTTCGACTCTGTTGAAATCAGCGGAGTGGAGGATCGCTTCAACGCATCCCTGGGAACGTATCACGAT
CTCCTGAAAATCATTAAAGACAAGGACTTCCTGGACAATGAGGAGAACGAGGACATTCTTGAGGACATTGTCCT
CACCCTTACGTTGTTTGAAGATAGGGAGATGATTGAAGAACGCTTGAAAACTTACGCTCATCTCTTCGACGACAA
AGTCATGAAACAGCTCAAGAGGCGCCGATATACAGGATGGGGGCGGCTGTCAAGAAAACTGATCAATGGGATC
CGAGACAAGCAGAGTGGAAAGACAATCCTGGATTTTCTTAAGTCCGATGGATTTGCCAACCGGAACTTCATGCA
GTTGATCCATGATGACTCTCTCACCTTTAAGGAGGACATCCAGAAAGCACAAGTTTCTGGCCAGGGGGACAGcCT
TCACGAGCACATCGCTAATCTTGCAGGTAGCCCAGCTATCAAAAAGGGAATACTGCAGACCGTTAAGGTCGTGG
ATGAACTCGTCAAAGTAATGGGAAGGCATAAGCCCGAGAATATCGTTATCGAGATGGCCCGAGAGAACCAAACT
ACCCAGAAGGGACAGAAGAACAGTAGGGAAAGGATGAAGAGGATTGAAGAGGGTATAAAAGAACTGGGGTCC
CAAATCCTTAAGGAACACCCAGTTGAAAACACCCAGCTTCAGAATGAGAAGCTCTACCTGTACTACCTGCAGAAC
GGCAGGGACATGTACGTGGATCAGGAACTGGACATCAATCGGCTCTCCGACTACGACGTGGATCATATCGTGCC
CCAGTCTTTTCTCAAAGATGATTCTATTGATAATAAAGTGTTGACAAGATCCGATAAAAATAGAGGGAAGAGTGA
TAACGTCCCCTCAGAAGAAGTTGTCAAGAAAATGAAAAATTATTGGCGGCAGCTGCTGAACGCCAAACTGATCA
CACAACGGAAGTTCGATAATCTGACTAAGGCTGAACGAGGTGGCCTGTCTGAGTTGGATAAAGCCGGCTTCATC
AAAAGGCAGCTTGTTGAGACACGCCAGATCACCAAGCACGTGGCCCAAATTCTCGATTCACGCATGAACACCAA
GTACGATGAAAATGACAAACTGATTCGAGAGGTGAAAGTTATTACTCTGAAGTCTAAGCTcGTCTCAGATTTCAG
AAAGGACTTTCAGTTTTATAAGGTGAGAGAGATCAACAATTACCACCATGCGCATGATGCCTACCTGAATGCAGT
GGTAGGCACTGCACTTATCAAAAAATATCCCAAGCTTGAATCTGAATTTGTTTACGGAGACTATAAAGTGTACGA
TGTTAGGAAAATGATCGCAAAGTCTGAGCAGGAAATAGGCAAGGCCACCGCTAAGTACTTCTTTTACAGCAATA

TTATGAATTTTTTCAAGACCGAGATTACACTGGCCAATGGAGAGATTCGGAAGCGACCACTTATCGAAACAAACG
GAGAAACAGGAGAAATCGTGTGGGACAAGGGTAGGGATTTCGCGACAGTCCGGAAGGTCCTGTCCATGCCGCA
GGTGAACATCGTTAAAAAGACCGAAGTACAGACCGGAGGCTTCTCCAAGGAAAGTATCCTCCCGAAAAGGAACA
GCGACAAGCTGATCGCACGCAAAAAAGATTGGGACCCCAAGAAATACGGCGGATTCGATTCTCCTACAGTCGCT
TACAGTGTACTGGTTGTGGCCAAAGTGGAGAAAGGGAAGTCTAAAAAACTCAAAAGCGTCAAGGAACTGCTGG
GCATCACAATCATGGAGCGATCAAGCTTCGAAAAAAACCCCATCGACTTTCTCGAGGCGAAAGGATATAAAGAG
GTCAAAAAAGACCTCATCATTAAGCTTCCCAAGTACTCTCTCTTTGAGCTTGAAAACGGCCGGAAACGAATGCTC
GCTAGTGCGGGCGAGCTGCAGAAAGGTAACGAGCTGGCACTGCCCTCTAAATACGTTAATTTCTTGTATCTGGCC
AGCCACTATGAAAAGCTCAAAGGGTCcCCCGAAGATAATGAGCAGAAGCAGCTGTTCGTGGAACAACACAAACA
CTACCTTGATGAGATCATCGAGCAAATAAGCGAATTCTCCAAAAGAGTGATCCTCGCCGACGCTAACCTCGATAA
GGTGCTTTCTGCTTACAATAAGCACAGGGATAAGCCCATCAGGGAGCAGGCAGAAAACATTATCCACTTGTTTAC
TCTGACCAACTTGGGCGCGCCTGCAGCCTTCAAGTACTTCGACACCACCATAGACAGAAAGCGGTACACCTCTAC
AAAGGAGGTCCTGGACGCCACACTGATTCATCAGTCAATTACGGGGCTCTATGAAACAAGAATCGACCTCTCTCA
GCTCGGTGGAGACAGCAGGGCTGACCCCAAGAAGAAGAGGAAGGTGTGA

Fig. 1

Continued

SEQ ID 17 (5 pages):

CGCTGTGAGCAAAAGGCCAGCAAAAGGCCAGGAACCGTAAAAAGGCCGCGTTGCTGGCGTTTTTCCATAGGCTC
CGCCCCCCTGACGAGCATCACAAAAATCGACGCTCAAGTCAGAGGTGGCGAAACCCGACAGGACTATAAAGATA
CCAGGCGTTTCCCCCTGGAAGCTCCCTCGTGCGCTCTCCTGTTCCGACCCTGCCGCTTACCGGATACCTGTCCGCC
TTTCTCCCTTCGGGAAGCGTGGCGCTTTCTCAATGCTCACGCTGTAGGTATCTCAGTTCGGTGTAGGTCGTTCGCT
CCAAGCTGGGCTGTGTGCACGAACCCCCCGTTCAGCCCGACCGCTGCGCCTTATCCGGTAACTATCGTCTTGAGT
CCAACCCGGTAAGACACGACTTATCGCCACTGGCAGCAGCCACTGGTAACAGGATTAGCAGAGCGAGGTATGTA
GGCGGTGCTACAGAGTTCTTGAAGTGGTGGCCTAACTACGGCTACACTAGAAGGACAGTATTTGGTATCTGCGC
TCTGCTGAAGCCAGTTACCTTCGGAAAAAGAGTTGGTAGCTCTTGATCCGGCAAACAAACCACCGCTGGTAGCG
GTGGTTTTTTTGTTTGCAAGCAGCAGATTACGCGCAGAAAAAAAGGATCTCAAGAAGATCCTTTGATCTTTTCTAC
GGGGTGGAGcgcaaaaaaccccgcccctgacagggcggggtttttcgcGATCGGAGGTACAGTGGCCATGAAATCCAATCAT
TTCCTTCTGGCCGCCCTCGGGCAAGAGATAGTGCCGCAGAGGACTCTCACAGCATCTACATCTGCGACCGCAACA
GCCACCAAGCGAGGCGCACATGAGCTTGTCCTCCTCCCATGCCAAAGTTTGGCCCTCTTCGTTTCTGTGATGCTGA
AGGAAGTCAAACTCGTCGATGATAGGACCAGATGGTTTGTCAAGGGTCAACGCTTTCCATGCCTTCTGGCACCG
GTAGTAATGCTCTTCTGCAAGGGAGACTTGACGTTTCGGATCCGCGGGCCCCGGGACATGCTGGAAGGGATTTT
CTGGCTCAATACCACGTCTGTATTTGACCCTTTCCAGACAGTTAATCCGCTGCAGGAGGGCGAACTGTAGCTCCTC
GTTCTCCTTGTAGCGCTTGATCCAGTCTTTTTGGATGTTGCACTTGCTTGGCCTATGCTTCTCATATAATCTTGCCCT
GTCATAGAGACGACGTCTGAGATTGTAGCGTTCGTCTTTGATCACCCGGAGCCAGATAGGCCTGAGTATATCTGA
CATTAGATCAAAGGGTCTGTGGATAGTCTCCTTCAGCATCAGCGACGCATGTGACTCGCATGTCGGAGAGAGCTT
GTGGGTGGTCATCTTTGATGGCGTCCTCTGCTTTCCCTTGATTTTCGTTGATTGTTTTTCGAAAGTTAAGTCTGGAA
GTCAAGAGAATCCTTCTGCCAGACATTATATTTACGTATACTGACGTAGTAGAAACAGCGTCAGGATGAGGACAT
GGTGTGTGCTGGACCACGGAATCATAGTTCATCAGTATATTGGGTTGGACAAATAACGCTGAGCATGTATATGTC
TTTACACACTATAAAAGCCAGCGAACGCCAATAAAATAGGGCATATTGATGTGAAAATATGACACCAGTTAAAA
GCAGTGTATTGATTTTATCTCTCTTCACCTCGGACCTATACTACCGTATACAAGACTCAACTTACTTCCAGATATAG
TAATATACACCCTATGGACGAACCAGCACAATAATTACAGCCAAACAACACCACCCAAATGGCATATTCCTAATC
AGCACTAAGCACAAATACCACTGTCATCACAGCATAATCAATAAGAATCCCAGACAACCGACTCACTCTGACTCA
CCTTACACAAACCCCCAAGCAAAGCGCAGCCCAGAACCTCAGCCAACAATCGGGCAACGTACGGGGAAAGATTG
GCCGATCCATGATGTCAGCAGCCCTAACCCAAAGCGGACTAGCGCATACCGCCCCTCTGACTCCGCCATCCCAGG
GCTCGAGAAGCTTCCGTGGCGTCGATATAAATTCAGCGGGCCTTGAACATCCCTCCTTACGACACACCTCACGCG
ATCGATTTTGACACTCACACACCGCCACCCTCACATCCTCCACCCACACCACACCCCTTAATCAACCCACCATCACC
GCTAGAACGTCTATCTCATCACCGACTTCTCATCCATCTTCAAACATGGAGCCACTGATGAGTCCGTGAGGACGA
AACGAGTAAGCTCGTCTGGCTCGCTCCATAAGCCAGgttttagagctagaaatagcaagttaaaataaggctagtccgttatcaact
tgaaaaagtggcaccgagtcggtgcttttGGCCGGCATGGTCCCAGCCTCCTCGCTGGCGCCGGCTGGGCAACATGCTTCG
GCATGGCGAATGGGACAGCTTTGGACTGCTTGATCCACTTAACGTTACTGAAATCATCAAACAGCTTGACGAATC
TGGATATAAGATCGTTGGTGTCGATGTCAGCTCCGGAGTTGAGACAAATGGTGTTCAGGATCTCGATAAGATAC
GTTCATTTGTCCAAGCAGCAAAGAGTGCCTTCTAGTGATTTAATAGCTCCATGTCAACAAGAATAAAACGCGTTTC
GGGTTTACCTCTTCCAGATACAGCTCATCTGCAATGCATTAATGCATTGGACCTCGCAACCCTAGTACGCCCTTCA
GGCTCCGGCGAAGCAGAAGAATAGCTTAGCAGAGTCTATTTTCATTTTCGGGAGACGAGATCAAGCAGATCAAC
GGTCGTCAAGAGTCCTACGAGACTGAGGAATCCGCTCTTGGCTCCACGCGACTATATATTTGTCTCTAATTGTACT
TTGACATGCTCCTCTTCTTTACTCTGATAGCTTGACTATGAAAATTCCGTCACCAGCCCCTGGGTTCGCAAAGATA
ATTGCACTGTTTCTTCCTTGAACTCTCAAGCCTACAGGACACACATTCATCGTAGGTATAAACCTCGAAAATCATT
CCTACTAAGATGGGTATACAATAGTAACCATGCATGGTTGCCTAGTGAATGCTCCGTAACACCCAATACGCCGGC
CGAAACTTTTTTACAACTCTCCTATGAGTCGTTTACCCAGAATGCACAGGTACACTTGTTTAGAGGTAATCCTTCTT

TCTAGAAGTCCTCGTGTACTGTGTAAGCGCCCACTCCACATCTCCACTCGCTCCGGGGAGAACCGAAGAGGATG
GGAACCCCGACCATGGCCTGCGGCCGCAGCTATCAGTCCTCTCAGCTGACGCCCCTCACGAACCCTCGCACTCTC
CCGAAATGCTCTTCGGTGTCCATGGCGCATCATCTCAGCAGCATCCCATGTCAAACCATGGCTTTGGGCCCACGG
ACTCTGTGGCCCTGCCGCAACATCACCATCACCATCGACTCCCGCCCCATGCAGCGCTGCGCGCCCCAGGGCGTC
ATGGAGTGAATGTGGAATCTCCTCCTTTGCCCTCGGGCCCGCCGAGTGTGGTGGGCCAGCCTGGAATGCCTGAT
CCAGCCCCCAGGCCTCGAGGACCAAAACTGAAGTTTACTCCCGAAGAGGACGCTCTACTGGTGGAGTTGAAGGA
AAACAAGAACTTGACGTGGAAGCAAATTGCAGACTTCTTCCCGGGCCGAACGAGCGGTACCTTGCAAGTCCGAT
ACTGCACCAAGCTGAAGGCTAAGGATGTAGCTTGGAGTGACGAAATGGTTCGATTTGCTCCTGATGTATTTCTAC
GCCTGTCTCACACATGCTAATGAAGGGAATAGGTACAAAGGCTGCAGCGGGCAATGCACGAGTACGAGAACGA
TCGGTGGCGCATCATTGCAGGGAAGGTTGGAAATGGCTTCACCCCAGCTGCTTGCCGCGAGAAAGCCATGCAGC
TCCATGAGTAAAAGCGTTGGGGAATTTTCATATTTATATCTACTGTCGCCAGATTCGGCCCTGCTTGGACCCTCTG
ATCTCCTTACTCTCCATATTGGTTCAAATGTCGGGTCACCGATAGGGCTGGTGGTGCAGGCTTGTTGTAGGCACG
GGAGGATGATCAGCATAACTCTGAGTCACTATAGGGACGGGTTGATGTAAGGTATTAAGTGATGTATGATAATT
CATTTTAGCCCGGGGGAACATATGGCGCCGGCATTTGTTCGTTCGCAATGAACCGACACTAGCGTCCGCTCTCGC
AGTTTAGCACCGGCTGATCCCGGGCTGAACGCGGCCATTGCTCGGCCGGGGCATGTGTTCCTTATCTACGGCAG
ACCGCAGATGACCACTGGAGCAGATTATAGACCCTAAGCCCTAAGCCGGACACCCAATCGAGTAGGTCTGCGGA
CCAGGTCACTGCGGGCAGCCGGAGAAGCTCCGCAACCAATCAATCCCCGGCGCTGACTAAGGGCAGGCGACCA
CGGGCCGAAGCGGCTTCAAACTCACCTCAACCTCCAAACTCCCTCATCTCCAAACGTCCTTGCCTTGTCTGCCGTC
ATTGCAACCCACCCACCAGGACACATGGACAAGAAGTACTCCATTGGGCTCGATATCGGCACAAACAGCGTCGG
TTGGGCCGTCATTACGGACGAGTACAAGGTGCCGAGCAAAAAATTCAAAGTTCTGGGCAATACCGATCGCCACA
GCATAAAGAAGAACCTCATTGGCGCCCTCCTGTTCGACTCCGGGGAGACGGCCGAAGCCACGCGGCTCAAAAGA
ACAGCACGGCGCAGATATACCCGCAGAAAGAATCGGATCTGCTACCTGCAGGAGATCTTTAGTAATGAGATGGC
TAAGGTGGATGACTCTTTCTTCCATAGGCTGGAGGAGTCCTTTTTGGTGGAGGAGGATAAAAAGCACGAGCGCC
ACCCAATCTTTGGCAATATCGTGGACGAGGTGGCGTACCATGAAAAGTACCCAACCATATATCATCTGAGGAAG
AAGCTTGTAGACAGTACTGATAAGGCTGACTTGCGGTTGATCTATCTCGCGCTGGCGCATATGATCAAATTTCGG
GGACACTTCCTCATCGAGGGGGACCTGAACCCAGACAACAGCGATGTCGACAAACTCTTTATCCAACTGGTTCAG
ACTTACAATCAGCTTTTCGAAGAGAACCCGATCAACGCATCCGGAGTTGACGCCAAAGCAATCCTGAGCGCTAG
GCTGTCCAAATCCCGGCGGCTCGAAAACCTCATCGCACAGCTCCCTGGGGAGAAGAAGAACGGCCTGTTTGGTA
ATCTTATCGCCCTGTCACTCGGGCTGACCCCCAACTTTAAATCTAACTTCGACCTGGCCGAAGATGCCAAGCTTCA
ACTGAGCAAAGACACCTACGATGATGATCTCGACAATCTGCTGGCCCAGATCGGCGACCAGTACGCAGACCTTTT
TTTGGCGGCAAAGAACCTGTCAGACGCCATTCTGCTGAGTGATATTCTGCGAGTGAACACGGAGATCACCAAAG
CTCCGCTGAGCGCTAGTATGATCAAGCGCTATGATGAGCACCACCAAGACTTGACTTTGCTGAAGGCCCTTGTCA
GACAGCAACTGCCTGAGAAGTACAAGGAAATTTTCTTCGATCAGTCTAAAAATGGCTACGCCGGATACATTGAC
GGCGGAGCAAGCCAGGAGGAATTTTACAAATTTATTAAGCCCATCTTGGAAAAAATGGACGGCACCGAGGAGCT
GCTGGTAAAGCTTAACAGAGAAGATCTGTTGCGCAAACAGCGCACTTTCGACAATGGAAGCATCCCCCACCAGA
TTCACCTGGGCGAACTGCACGCTATCCTCAGGCGGCAAGAGGATTTCTACCCCTTTTTGAAAGATAACAGGGAAA
AGATTGAGAAAATCCTCACATTTCGGATACCCTACTATGTAGGCCCCCTCGCCCGGGGAAATTCCAGATTCGCGT
GGATGACTCGCAAATCAGAAGAGACtATCACTCCCTGGAACTTCGAGGAAGTCGTGGATAAGGGGGCCTCTGCC
CAGTCCTTCATCGAAAGGATGACTAACTTTGATAAAAATCTGCCTAACGAAAAGGTGCTTCCTAAACACTCTCTGC
TGTACGAGTACTTCACAGTTTATAACGAGCTCACCAAGGTCAAATACGTCACAGAAGGGATGAGAAAGCCAGCA
TTCCTGTCTGGAGAGCAGAAGAAAGCTATCGTGGACCTCCTCTTCAAGACGAACCGGAAAGTTACCGTGAAACA
GCTCAAAGAgGACTATTTCAAAAAGATTGAATGTTTCGACTCTGTTGAAATCAGCGGAGTGGAGGATCGCTTCAA
CGCATCCCTGGGAACGTATCACGATCTCCTGAAAATCATTAAAGACAAGGACTTCCTGGACAATGAGGAGAACG
AGGACATTCTTGAGGACATTGTCCTCACCCTTACGTTGTTTGAAGATAGGGAGATGATTGAAGAACGCTTGAAAA
CTTACGCTCATCTCTTCGACGACAAAGTCATGAAACAGCTCAAGAGGCGCCGATATACAGGATGGGGGCGGCTG
TCAAGAAAACTGATCAATGGGATCCGAGACAAGCAGAGTGGAAAGACAATCCTGGATTTTCTTAAGTCCGATGG
ATTTGCCAACCGGAACTTCATGCAGTTGATCCATGATGACTCTCTCACCTTTAAGGAGGACATCCAGAAAGCACA
AGTTTCTGGCCAGGGGGACAGcCTTCACGAGCACATCGCTAATCTTGCAGGTAGCCCAGCTATCAAAAAGGGAA

TACTGCAGACCGTTAAGGTCGTGGATGAACTCGTCAAAGTAATGGGAAGGCATAAGCCCGAGAATATCGTTATC
GAGATGGCCCGAGAGAACCAAACTACCCAGAAGGGACAGAAGAACAGTAGGGAAAGGATGAAGAGGATTGAA
GAGGGTATAAAAGAACTGGGGTCCCAAATCCTTAAGGAACACCCAGTTGAAAACACCCAGCTTCAGAATGAGAA
GCTCTACCTGTACTACCTGCAGAACGGCAGGGACATGTACGTGGATCAGGAACTGGACATCAATCGGCTCTCCG
ACTACGACGTGGATCATATCGTGCCCCAGTCTTTTCTCAAAGATGATTCTATTGATAATAAAGTGTTGACAAGATC
CGATAAAAATAGAGGGAAGAGTGATAACGTCCCCTCAGAAGAAGTTGTCAAGAAAATGAAAAATTATTGGCGG
CAGCTGCTGAACGCCAAACTGATCACACAACGGAAGTTCGATAATCTGACTAAGGCTGAACGAGGTGGCCTGTC
TGAGTTGGATAAAGCCGGCTTCATCAAAAGGCAGCTTGTTGAGACACGCCAGATCACCAAGCACGTGGCCCAAA
TTCTCGATTCACGCATGAACACCAAGTACGATGAAAATGACAAACTGATTCGAGAGGTGAAAGTTATTACTCTGA
AGTCTAAGCTcGTCTCAGATTTCAGAAAGGACTTTCAGTTTTATAAGGTGAGAGAGATCAACAATTACCACCATG
CGCATGATGCCTACCTGAATGCAGTGGTAGGCACTGCACTTATCAAAAAATATCCCAAGCTTGAATCTGAATTTG
TTTACGGAGACTATAAAGTGTACGATGTTAGGAAAATGATCGCAAAGTCTGAGCAGGAAATAGGCAAGGCCACC
GCTAAGTACTTCTTTTACAGCAATATTATGAATTTTTTCAAGACCGAGATTACACTGGCCAATGGAGAGATTCGGA
AGCGACCACTTATCGAAACAAACGGAGAAACAGGAGAAATCGTGTGGGACAAGGGTAGGGATTTCGCGACAGT
CCGGAAGGTCCTGTCCATGCCGCAGGTGAACATCGTTAAAAAGACCGAAGTACAGACCGGAGGCTTCTCCAAGG
AAAGTATCCTCCCGAAAAGGAACAGCGACAAGCTGATCGCACGCAAAAAAGATTGGGACCCCAAGAAATACGG
CGGATTCGATTCTCCTACAGTCGCTTACAGTGTACTGGTTGTGGCCAAAGTGGAGAAAGGGAAGTCTAAAAAAC
TCAAAAGCGTCAAGGAACTGCTGGGCATCACAATCATGGAGCGATCAAGCTTCGAAAAAAACCCCATCGACTTTC
TCGAGGCGAAAGGATATAAAGAGGTCAAAAAAGACCTCATCATTAAGCTTCCCAAGTACTCTCTCTTTGAGCTTG
AAAACGGCCGGAAACGAATGCTCGCTAGTGCGGGCGAGCTGCAGAAAGGTAACGAGCTGGCACTGCCCTCTAA
ATACGTTAATTTCTTGTATCTGGCCAGCCACTATGAAAAGCTCAAAGGGTCcCCCGAAGATAATGAGCAGAAGCA
GCTGTTCGTGGAACAACACAAACACTACCTTGATGAGATCATCGAGCAAATAAGCGAATTCTCCAAAAGAGTGAT
CCTCGCCGACGCTAACCTCGATAAGGTGCTTTCTGCTTACAATAAGCACAGGGATAAGCCCATCAGGGAGCAGG
CAGAAAACATTATCCACTTGTTTACTCTGACCAACTTGGGCGCGCCTGCAGCCTTCAAGTACTTCGACACCACCAT
AGACAGAAAGCGGTACACCTCTACAAAGGAGGTCCTGGACGCCACACTGATTCATCAGTCAATTACGGGGCTCT
ATGAAACAAGAATCGACCTCTCTCAGCTCGGTGGAGACAGCAGGGCTGACCCCAAGAAGAAGAGGAAGGTGTG
AGCTTCGTCCGACGGCGGCCCACGGGTCCCAGGCCTCGGAGATCCGTCCCCCTTTTCCTTTGTCGATATCATGTA
ATTAGTTATGTCACGCTTACATTCACGCCCTCCCCCCACATCCGCTCTAACCGAAAAGGAAGGAGTTAGACAACCT
GAAGTCTAGGTCCCTATTTATTTTTTTATAGTTATGTTAGTATTAAGAACGTTATTTATATTTCAAATTTTTCTTTTTT
TTCTGTACAGACGCGTGTACGCATGTAACATTATACTGAAAACCTTGCTTGAGAAGGTTTTGGGACGCTCGAAGG
CTTTAATTTGCAAGCTGGCGCTAATTCATGAATAACGGTGAGACTAGCGGCCGGTCCCCTTATCCcAGCTGTtCCA
CGTTGGCCTGCCCCTCAGTTAGCGCTCAACTCAATGCCCCTCACTGGCGAGGCGAGGGCAAGGATGGAGGGGCA
GCATCGCCTGAGTTGGAGCAAAGCGGCCGCCATGGGAGCAGCGAACCAACGGAGGGATGCCGTGCTTTGTCGT
GGCTGCTGTGGCCAATCCGGGCCCTTGGTTGGCTCACAGAGCGTTGCTGTGAGTCCATGAGCTATTATTGCTAGG
TACAGTATAGAGAGAGGAGAGAGAGAGAGAGAGAGAGAGAGGGGAAAAAAGGTGAGGTTGAAGTGAGAAAAAA
AAAAAAAAAAATCCAACCACTGACGGCTGCCGGCTCTGCCACCCCCCTCCCTCCACCCCAGACCACCTGCACACTC
AGCGCGCAGCATCACCTAATCTTGGCTCGCCTTCCCGCAGCTCAGGTTGTTTTTTTTTTCTCTCTCCCTCGTCGAAG
CCGCCCTTGTTCCCTTATTTATTTCCCTCTCCATCCTTGTCTGCCTTTGGTCCATCTGCCCCTTTGTCTGCATCTCTTT
TGCACGCATCGCCTTATCGTCGTCTCTTTTTTCACTCACGGGAGCTTGACGATGACCTGACTCGTGAGCCTCACCT
GCTGATTTCTCTCCCCCCCTCCCGACCGGCTTGACTTTTGTTTCTCCTCCAGTACCTTATCGCGAAGCCGGAAGAAC
CTCTTAACCTCTAGATGAAAAAGCCTGAACTCACCGCGACGTCTGTCGAGAAGTTcCTGATCGAAAAGTTCGACA
GCGTCTCCGACCTGATGCAGCTCTCGGAGGGCGAAGAATCTCGTGCTTTCAGCTTCGATGTAGGAGGGCGTGGA
TATGTCCTGCGGGTAAATAGCTGCGCCGATGGTTTCTACAAAGATCGTTATGTTTATCGGCACTTTGCATCGGCC
GCGCTCCCGATTCCGGAAGTGCTTGACATTGGGGAATTCAGCGAGAGCCTGACCTATTGCATCTCCCGCCGTGCA
CAGGGTGTCACGTTGCAAGACCTGCCTGAAACCGAACTGCCCGCTGTTCTGCAGCCGGTCGCGGAGGCCATGGA
TGCGATCGCTGCGGCCGATCTcAGCCAGACGAGCGGGTTCGGCCCATTCGGACCGCAAGGAATCGGTCAATACA
CTACATGGCGTGATTTCATATGCGCGATTGCTGATCCCCATGTGTATCACTGGCAAACTGTGATGGACGACACCG
TCAGTGCGTCCGTCGCGCAGGCTCTCGATGAGCTGATGCTTTGGGCCGAGGACTGCCCCGAAGTCCGGCACCTC

GTGCACGCGGATTTCGGCTCCAACAATGTCCTGACGGACAATGGCCGCATAACAGCGGTCATTGACTGGAGCGA
GGCGATGTTCGGGGATTCCCAATACGAGGTCGCCAACATCTTCTTCTGGAGGCCGTGGTTGGCTTGTATGGAGC
AGCAGACGCGCTACTTCGAGCGGAGGCAcCCGGAGCTTGCAGGATCGCCGCGGCTCCGGGCGTATATGCTCCGC
ATTGGTCTTGACCAACTCTATCAGAGCTTGGTTGACGGCAATTTCGATGATGCAGCTTGGGCGCAGGGTCGATGC
GACGCAATCGTCCGATCCGGAGCCGGGACTGTCGGGCGTACACAAATCGCCCGCAGAAGCGCGGCCGTCTGGA
CCGATGGCTGTGTAGAAGTACTCGCCGATAGTGGAAACCGACGCCCCAGCACTCGTCCGAGGGCAAAGGAATA
GATGCATGGCTTTCGTGACCGGGCTTCAAACAATGATGTGCGATGGTGTGGTTCCCGGTTGGCGGAGTCTTTGTC
TACTTTGGTTGTCTGTCGCAGGTCGGTAGACCGCAAATGAGCAACTGATGGATTGTTGCCAGCGATACTATAATT
CACATGGATGGTCTTTGTCGATCAGTAGCTAGTGAGAGAGAGAGAACATCTATCCACAATGTCGAGTGTCTATTA
GACATACTCCGAGAATAAAGTCAACTGTGTCTGTGATCTAAAGATCGATTCGGCAGTCGAGTAGCGTATAACAAC
TCCGAGTACCAGCGAAAGCACGTCGTGACAGGAGCAGGGCTTTGCCAACTGCGCAACCTTGCTTGAATGAGGAT
ACACGGGGTGCAACATGGCTGTACTGATCCATCGCAACCAAAATTTCTGTTTATAGATCAAGCTGGTAGATTCCA
ATTACTCCACCTCTTGCGCTTCTCCATGACATGTAAGTGCACGTGGAAACCATACCCAAATTGCCTACAGCTGCGG
AGCATGAGCCTATGGCGATCAGTCTGGTCATGTTAACCAGCCTGTGCTCTGACGTTAATGCAGAATAGCTTATGC
TGCAGACGCGTTACGTATCGGATCCAGAATTCGTGATATCTGAATTCGTCGACAAGCTTATTTTTTGTATACTGTT
TTGTGATAGCACGAAGTTTTTCCACGGTATCTTGTTAAAAATATATATTTGTGGCGGGCTTACCTACATCAAATTA
ATAAGAGACTAATTATAAACTAAACACACAAGCAAGCTACTTTAGGGTAAAAGTTTATAAATGCTTTTGACGTAT
AAACGTTGCTTGTATTTATTATTACAATTAAAGGTGGATAGAAAACCTAGAGACTAGTTAGAAACTAATCTCAGG
TTTGCGTTAAACTAAATCAGAGCCCGAGAGGTTAACAGAACCTAGAAGGGGACTAGATATCCGGGTAGGGAAA
CAAAAAAAAAAAACAAGACAGCCACATATTAGGGAGACTAGTTAGAAGCTAGTTCCAGGACTAGGAAAATAAA
AGACAATGATACCACAGTCTAGTTGACAACTAGATAGATTCTAGATTGAGGCCAAAGTCTCTGAGATCCAGGTTA
GTTGCAACTAATACTAGTTAGTATCTAGTCTCCTATAACTCTGAAGCTAGAATAACTTACTACTATTATCCTCACCA
CTGTTCAGCTGCGCAAACGGAGTGATTGCAAGGTGTTCAGAGACTAGTTATTGACTAGTCAGTGACTAGCAATA
ACTAACAAGGTATTAACCTACCATGTCTGCCATCACCCTGCACTTCCTCGGGCTCAGCAGCCTTTTCCTCCTCATTT
TCATGCTCATTTTCCTTGTTTAAGACTGTGACTAGTCAAAGACTAGTCCAGAACCACAAAGGAGAAATGTCTTACC
ACTTTCTTCATTGCTTGTCTCTTTTGCATTATCCATGTCTGCAACTAGTTAGAGTCTAGTTAGTGACTAGTCCGACG
AGGACTTGCTTGTCTCCGGATTGTTGGAGGAACTCTCCAGGGCCTCAAGATCCACAACAGAGCCTTCTAGAtGAC
TGGTCAATAACTAGTTGGTCTTTGTCTGAGTCTGACTGACTTACGAGGTTGCATACTCGCTCCCTTTGCCTCGTCA
ATCGATGAGAAAAAGCGCCAAAACTCGCAATATGGCTTTGAACCACACGGTGCTGAGACTAGTTAGAATCTAGT
CCCAAACTAGCTTGGATAGCTTACCTTTGCCCTTTGCGTTGCGACAGGTCTTGCAGGGTATGGTTCCTTTCTCACC
AGCTGATTTAGCTGCCTTGCTACCCTCACGGCGGATCTGCATAAAGAGTGGCTAGAGGTTATAAATTAGCACTGA
TCCTAGGTACGGGGCTGAATGTAACTTGCCTTTCCTTTCTCATCGCGCGGCAAGACAGGCTTGCTCAAATTCCTAC
CAGTCACAGGGGTATGCACGGCGTACGGACCACTTGAACTAGTCACAGATTAGTTAGCAACTAGTCTGCATTGA
ATGGCTGTACTTACGGGCCCTCGCCATTGTCCTGATCATTTCCAGCTTCACCCTCGTTGCTGCAAAGTAGTTAGTG
ACTAGTCAAGGACTAGTTGAAATGGGAGAAGAAACTCACGAATTCTCGACACCCTTAGTATTGTGGTCCTTGGAC
TTGGTGCTGCTATATATTAGCTAATACACTAGTTAGACTCACAGAAACTTACGCAGCTCGCTTGCGCTTCTTGGTA
GGAGTCGGGGTTGGGAGAACAGTGCCTTCAAACAAGCCTTCATACCATGCTACTTGACTAGTCAGGGACTAGTC
ACCAAGTAATCTAGATAGGACTTGCCTTTGGCCTCCATCAGTTCCTTCATAGTGGGAGGTCCATTGTGCAATGTAA
ACTCCATGCCGTGGGAGTTCTTGTCCTTCAAGTGCTTGACCAATATGTTTCTGTTGGCAGAGGGAACCTGTCAACT
AGTTAATAACTAGTCAGAAACTAGTATAGCAGTAGACTCACTGTACGCTTGAGGCATCCCTTCACTCGGCAGTAG
ACTTCATATGGATGGATATCAGGCACGCCATTGTCGTCCTGTGGACTAGTCAGTAACTAGGCTTAAAGCTAGTCG
GGTCGGCTTACTATCTTGAAATCCGGCAGCGTAAGCTCCCCGTCCTTAACTGCCTCGAGATAGTGACAGTACTCT
GGGGACTTTCGGAGATCGTTATCGCGAATGCTCGGCATACTAATCGTTGACTAGTCTTGGACTAGTCCCGAGCAA
AAAGGATTGGAGGAGGAGGAGGAAGGTGAGAGTGAGACAAAGAGCGAAATAAGAGCTTCAAAGGCTATCTCT
AAGCAGTATGAAGGTTAAGTATCTAGTTCTTGACTAGATTTAAAAGAGATTTCGACTAGTTATGTACCTGGAGTT
TGGATATAGGAATGTGTTGTGGTAACGAAATGTAAGGGGGAGGAAAGAAAAAGTCGGTCAAGAGGTAACTCTA
AGTCGGCCATTCCTTTTTGGGAGGCGCTAACCATAAACGGCATGGTCGACTTAGAGTTAGCTCAGGGAATTTAG
GGAGTTATCTGCGACCACCGAGGAACGGCGGAATGCCAAAGAATCCCGATGGAGCTCTAGCTGGCGGTTGACA

ACCCCACCTTTTGGCGTTTCTGCGGCGTTGCAGGCGGGACTGGATACTTCGTAGAACCAGAAAGGCAAGGCAGA
ACGCGCTCAGCAAGAGTGTTGGAAGTGATAGCATGATGTGCCTTGTTAACTAGGTCAAAATCTGCAGTATGCTTG
ATGTTATCCAAAGTGTGAGAGAGGAAGGTCCAAACATACACGATTGGGAGAGGGCCTAGGTATAAGAGTTTTTG
AGTAGAACGCATGTGAGCCCAGCCATCTCGAGGAGATTAAACACGGGCCGGCATTTGATGGCTATGTTAGTACC
CCAATGGAAAGCCTGAGAGTCCAGTGG

SEQ ID 18:

GAACCAUC**CUGAUGAGUCCGUGAGGACGAAACGAGUAAGCUCGUC**GAUGGUUCCGUUCUACGGGC**GUUU**
**UAGAGCUAGAAAUAGCAAGUU**AAAAUAAGGCUAGUCCGUUAUCAACUUGAAAAAGUGGCACCGAGUCGG
UGCUUUU

SEQ ID 19:

MDKKYSIGLDIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIFG
NIVDEVAYHEKYPTIYHLRKKLVDSTDKADLRLIYLALAHMIKFRGHFLIEGDLNPDNSD
VDKLFIQLVQTYNQLFEENPINASGVDAKAILSARLSKSRRLENLIAQLPGEKKNGLFGN
LIALSLGLTPNFKSNFDLAEDAKLQLSKDTYDDDLDNLLAQIGDQYADLFLAAKNLSDAI
LLSDILRVNTEITKAPLSASMIKRYDEHHQDLTLLKALVRQQLPEKYKEIFFDQSKNGYA
GYIDGGASQEEFYKFIKPILEKMDGTEELLVKLNREDLLRKQRTFDNGSIPHQIHLGELH
AILRRQEDFYPFLKDNREKIEKILTFRIPYYVGPLARGNSRFAWMTRKSEETITPWNFEE
VVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKI
IKDKDFLDNEENEDILEDIVLTLTLFEDREMIEERLKTYAHLFDDKVMKQLKRRRYTGWG
RLSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSRER
MKRIEEGIKELGSQILKEHPVENTQLQNEKLYLYYLQNGRDMYVDQELDINRLSDYDVDH
IVPQSFLKDDSIDNKVLTRSDKNRGKSDNVPSEEVVKKMKNYWRQLLNAKLITQRKFDNL
TKAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYDVRK
MIAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSPTVA
YSVLVVAKVEKGKSKKLKSVKELLGITIMERSSFEKNPIDFLEAKGYKEVKKDLIIKLPK
YSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGA
PAAFKYFDTTIDRKRYTSTKEVLDATLIHQSITGLYETRIDLSQLGGD

Fig. 1

Continued

SEQ ID 20:

MTQFEGFTNLYQVSKTLRFELIPQGKTLKHIQEQGFIEEDKARNDHYKELKPIIDRIYKT
YADQCLQLVQLDWENLSAAIDSYRKEKTEETRNALIEEQATYRNAIHDYFIGRTDNLTDA
INKRHAEIYKGLFKAELFNGKVLKQLGTVTTTEHENALLRSFDKFTTYFSGFYENRKNVF
SAEDISTAIPHRIVQDNFPKFKENCHIFTRLITAVPSLREHFENVKKAIGIFVSTSIEEV
FSFPFYNQLLTQTQIDLYNQLLGGISREAGTEKIKGLNEVLNLAIQKNDETAHIIASLPH
RFIPLFKQILSDRNTLSFILEEFKSDEEVIQSFCKYKTLLRNENVLETAEALFNELNSID
LTHIFISHKKLETISSALCDHWDTLRNALYERRISELTGKITKSAKEKVQRSLKHEDINL
QEIISAAGKELSEAFKQKTSEILSHAHAALDQPLPTTLKKQEEKEILKSQLDSLLGLYHL
LDWFAVDESNEVDPEFSARLTGIKLEMEPSLSFYNKARNYATKKPYSVEKFKLNFQMPTL
ASGWDVNKEKNNGAILFVKNGLYYLGIMPKQKGRYKALSFEPTEKTSEGFDKMYYDYFPD
AAKMIPKCSTQLKAVTAHFQTHTTPILLSNNFIEPLEITKEIYDLNNPEKEPKKFQTAYA
KKTGDQKGYREALCKWIDFTRDFLSKYTKTTSIDLSSLRPSSQYKDLGEYYAELNPLLYH
ISFQRIAEKEIMDAVETGKLYLFQIYNKDFAKGHHGKPNLHTLYWTGLFSPENLAKTSIK
LNGQAELFYRPKSRMKRMAHRLGEKMLNKKLKDQKTPIPDTLYQELYDYVNHRLSHDLSD
EARALLPNVITKEVSHEIIKDRRFTSDKFFFHVPITLNYQAANSPSKFNQRVNAYLKEHP
ETPIIGIDRGERNLIYITVIDSTGKILEQRSLNTIQQFDYQKKLDNREKERVAARQAWSV
VGTIKDLKQGYLSQVIHEIVDLMIHYQAVVVLENLNFGFKSKRTGIAEKAVYQQFEKMLI
DKLNCLVLKDYPAEKVGGVLNPYQLTDQFTSFAKMGTQSGFLFYVPAPYTSKIDPLTGFV
DPFVWKTIKNHESRKHFLEGFDFLHYDVKTGDFILHFKMNRNLSFQRGLPGFMPAWDIVF
EKNETQFDAKGTPFIAGKRIVPVIENHRFTGRYRDLYPANELIALLEEKGIVFRDGSNIL
PKLLENDDSHAIDTMVALIRSVLQMRNSNAATGEDYINSPVRDLNGVCFDSRFQNPEWPM
DADANGAYHIALKGQLLLNHLKESKDLKLQNGISNQDWLAYIQELRN

SEQ ID 21:

MARILAFDIGISSIGWAFSENDELKDCGVRIFTKVENPKTGESLALPRRLARSARKRLAR
RKARLNHLKHLIANEFKLNYEDYQSFDESLAKAYKGSLISPYELRFRALNELLSKQDFAR
VILHIAKRRGYDDIKNSDDKEKGAILKAIKQNEEKLANYQSVGEYLYKEYFQKFKENSKE
FTNVRNKKESYERCIAQSFLKDELKLIFKKQREFGFSFSKKFEEEVLSVAFYKRALKDFS
HLVGNCSFFTDEKRAPKNSPLAFMFVALTRIINLLNNLKNTEGILYTKDDLNALLNEVLK
NGTLTYKQTKKLLGLSDDYEFKGEKGTYFIEFKKYKEFIKALGEHNLSQDDLNEIAKDIT
LIKDEIKLKKALAKYDLNQNQIDSLSKLEFKDHLNISFKALKLVTPLMLEGKKYDEACNE
LNLKVAINEDKKDFLPAFNETYYKDEVTNPVVLRAIKEYRKVLNALLKKYGKVHKINIEL
AREVGKNHSQRAKIEKEQNENYKAKKDAELECEKLGLKINSKNILKLRLFKEQKEFCAYS
GEKIKISDLQDEKMLEIDHIYPYSRSFDDSYMNKVLVFTKQNQEKLNQTPFEAFGNDSAK
WQKIEVLAKNLPTKKQKRILDKNYKDKEQKNFKDRNLNDTRYIARLVLNYTKDYLDFLPL
SDDENTKLNDTQKGSKVHVEAKSGMLTSALRHTWGFSAKDRNNHLHHAIDAVIIAYANNS
IVKAFSDFKKEQESNSAELYAKKISELDYKNKRKFFEPFSGFRQKVLDKIDEIFVSKPER
KKPSGALHEETFRKEEEFYQSYGGKEGVLKALELGKIRKVNGKIVKNGDMFRVDIFKHKK
TNKFYAVPIYTMDFALKVLPNKAVARSKKGEIKDWILMDENYEFCFSLYKDSLILIQTKD
MQEPEFVYYNAFTSSTVSLIVSKHDNKFETLSKNQKILFKNANEKEVIAKSIGIQNLKVF
EKYIVSALGEVTKAEFRQREDFKK

Fig. 1

Continued

SEQ ID 22:

MNFKILPIAIDLGVKNTGVFSAFYQKGTSLERLDNKNGKVYELSKDSYTLLMNNRTARRH
QRRGIDRKQLVKRLFKLIWTEQLNLEWDKDTQQAISFLFNRRGFSFITDGYSPEYLNIVP
EQVKAILMDIFDDYNGEDDLDSYLKLATEQESKISEIYNKLMQKILEFKLMKLCTDIKDD
KVSTKTLKEITSYEFELLADYLANYSESLKTQKFSYTDKQGNLKELSYYHHDKYNIQEFL
KRHATINDRILDTLLTDDLDIWNFNFEKFDFDKNEEKLQNQEDKDHIQAHLHHFVFAVNK
IKSEMASGGRHRSQYFQEITNVLDENNHQEGYLKNFCENLHNKKYSNLSVKNLVNLIGNL
SNLELKPLRKYFNDKIHAKADHWDEQKFTETYCHWILGEWRVGVKDQDKKDGAKYSYKDL
CNELKQKVTKAGLVDFLLELDPCRTIPPYLDNNNRKPPKCQSLILNPKFLDNQYPNWQQY
LQELKKLQSIQNYLDSFETDLKVLKSSKDQPYFVEYKSSNQQIASGQRDYKDLDARILQF
IFDRVKASDELLLNEIYFQAKKLKQKASSELEKLESSKKLDEVIANSQLSQILKSQHTNG
IFEQGTFLHLVCKYYKQRQRARDSRLYIMPEYRYDKKLHKYNNTGRFDDDNQLLTYCNHK
PRQKRYQLLNDLAGVLQVSPNFLKDKIGSDDDLFISKWLVEHIRGFKKACEDSLKIQKDN
RGLLNHKINIARNTKGKCEKEIFNLICKIEGSEDKKGNYKHGLAYELGVLLFGEPNEASK
PEFDRKIKKFNSIYSFAQIQQIAFAERKGNANTCAVCSADNAHRMQQIKITEPVEDNKDK
IILSAKAQRLPAIPTRIVDGAVKKMATILAKNIVDDNWQNIKQVLSAKHQLHIPIITESN
AFEFEPALADVKGKSLKDRRKKALERISPENIFKDKNNRIKEFAKGISAYSGANLTDGDF
DGAKEELDHIIPRSHKKYGTLNDEANLICVTRGDNKNKGNRIFCLRDLADNYKLKQFETT
DDLEIEKKIADTIWDANKKDFKFGNYRSFINLTPQEQKAFRHALFLADENPIKQAVIRAI
NNRNRTFVNGTQRYFAEVLANNIYLRAKKENLNTDKISFDYFGIPTIGNGRGIAEIRQLY
EKVDSDIQAYAKGDKPQASYSHLIDAMLAFCIAADEHRNDGSIGLEIDKNYSLYPLDKNT
GEVFTKDIFSQIKITDNEFSDKKLVRKKAIEGFNTHRQMTRDGIYAENYLPILIHKELNE
VRKGYTWKNSEEIKIFKGKKYDIQQLNNLVYCLKFVDKPISIDIQISTLEELRNILTTNN
IAATAEYYYINLKTQKLHEYYIENYNTALGYKKYSKEMEFLRSLAYRSERVKIKSIDDVK
QVLDKDSNFIIGKITLPFKKEWQRLYREWQNTTIKDDYEFLKSFFNVKSITKLHKKVRKD
FSLPISTNEGKFLVKRKTWDNNFIYQILNDSDSRADGTKPFIPAFDISKNEIVEAIIDSF
TSKNIFWLPKNIELQKVDNKNIFAIDTSKWFEVETPSDLRDIGIATIQYKIDNNSRPKVR
VKLDYVIDDDSKINYFMNHSLLKSRYPDKVLEILKQSTIIEFESSGFNKTIKEMLGMKLA
GIYNETSNN

Fig. 1

Continued

SEQ ID 23:

MDKKYSIGLAIGTNSVGWAVITDEYKVPSKKFKVLGNTDRHSIKKNLIGALLFDSGETAEATRLKRTARR
RYTRRKNRICYLQEIFSNEMAKVDDSFFHRLEESFLVEEDKKHERHPIFGNIVDEVAYHEKYPTIYHLRK
KLVDSTDKADLRLIYLALAHMIKFRGHFLIEGDLNPDNSDVDKLFIQLVQTYNQLFEENPINASGVDAKA
ILSARLSKSRRLENLIAQLPGEKKNGLFGNLIALSLGLTPNFKSNFDLAEDAKLQLSKDTYDDDLDNLLA
QIGDQYADLFLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSIPHQIHLGELH
AILRRQEDFYPFLKDNREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQKKAIVDLLFKTNRKVT
VKQLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQLIHDD
SLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDA
IVPQSFLKDDSIDNKVLTRSDKNRGKSDNVPSEEVVKKMKNYWRQLLNAKLITQRKFDNLTKAERGGLSE
LDKAGFIKRQLVETRQITKHVAQILDSRMNTKYDENDKLIREVKVITLKSKLVSDFRKDFQFYKVREINN
YHHAHDAYLNAVVGTALIKKYPKLESEFVYGDYKVYDVRKMIAKSEQEIGKATAKYFFYSNIMNFFKTEI
TLANGEIRKRPLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPIDFLEAKGYKEV
KKDLIIKLPKYSLFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAPAAFKYFDTT
IDRKRYTSTKEVLDATLIHQSITGLYETRIDLSQLGGD

SEQ ID 24:

NNNNNNCUGAUGAGUCCGUGAGGACGAAACGAGUAAGCUCGUCNNNNNNNNNNNNNNNNNNNNGUUUUAGAGCUAG
AAAUAGCAAGUUAAAAUAAGGCUAGUCCGUUAUCAACUUGAAAAAGUGGCACCGAGUCGGUGCUUUUGGCCGGCAU
GGUCCCAGCCUCCUCGCUGGCGCCGGCUGGGCAACAUGCUUCGGCAUGGCGAAUGGGACAGCUUUGGAC
Wherein N at position 1 to 6 is any nucleotide, and
N at position 44 to 63 is any nucleotide, and
N at position 1 is complementary to N at position 49, and
N at position 2 is complementary to N at position 48, and
N at position 3 is complementary to N at position 47, and
N at position 4 is complementary to N at position 46, and
N at position 5 is complementary to N at position 45, and
N at position 6 is complementary to N at position 44;
N at position 50 to 63 is any nucleotide.

SEQ ID 25:

NNNNNNNNNNNNNNNNNNNNGUUUUAGAGCUAGAAAUAGCAAGUUAAAAUAAGGCUAGUCCGUUAUCAACUUGAAA
AAGUGGCACCGAGUCGGUGCUUUUGGCCGGCAUGGUCCCAGCCUCCUCGCUGGCGCCGGCUGGGCAACAUGCUUCG
GCAUGGCGAAUGGGACAGCUUUGGAC
Wherein N at position 1 to 20 is any nucleotide, and
the same as N at position 44 to 63 in SEQ ID 24.

Fig. 2

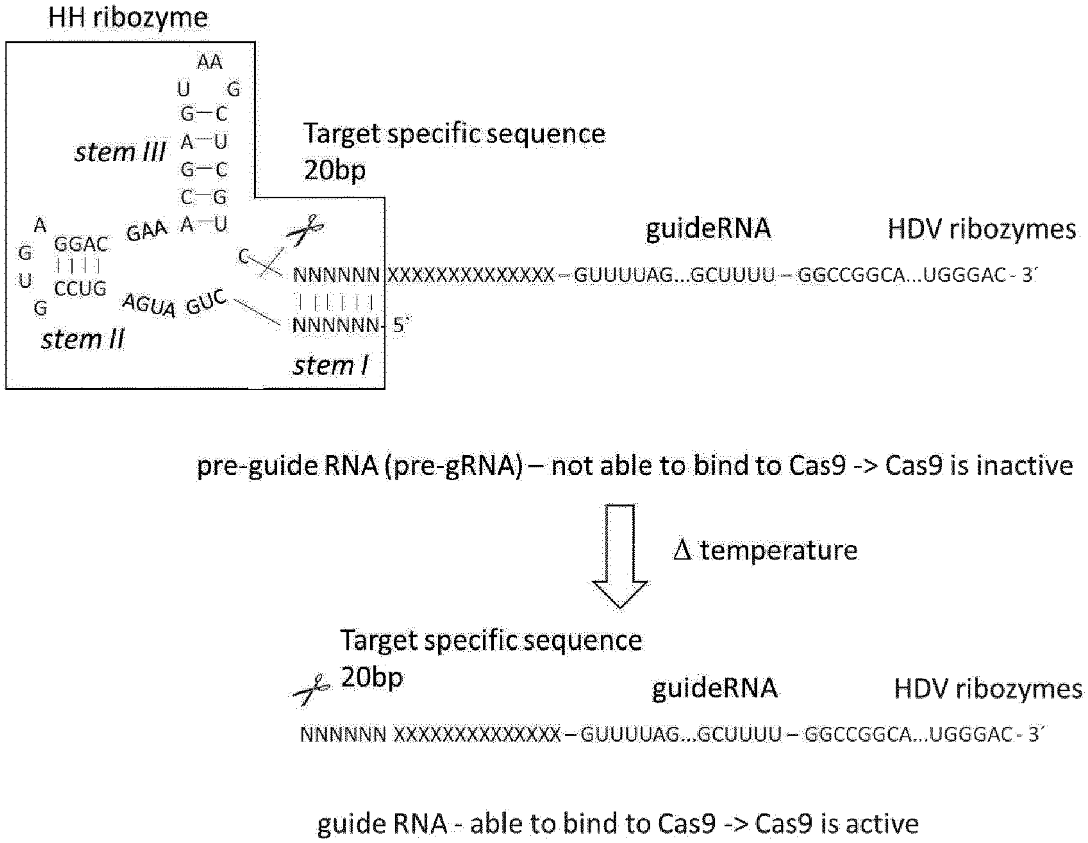
HH ribozyme
stem III
stem II
stem I
Target specific sequence 20bp
guideRNA
HDV ribozymes
NNNNNN XXXXXXXXXXXXXX – GUUUUAG...GCUUUU – GGCCGGCA...UGGGAC - 3´
NNNNNN- 5`
pre-guide RNA (pre-gRNA) – not able to bind to Cas9 -> Cas9 is inactive
Δ temperature
Target specific sequence 20bp
guideRNA
HDV ribozymes
NNNNNN XXXXXXXXXXXXXX – GUUUUAG...GCUUUU – GGCCGGCA...UGGGAC - 3´
guide RNA - able to bind to Cas9 -> Cas9 is active

Europäisches Patentamt
European Patent Office
Office européen des brevets

# EUROPEAN SEARCH REPORT

Application Number
EP 16 15 2731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ATHANASIOS SARAGLIADIS ET AL: "Thermozymes", RNA BIOLOGY, vol. 10, no. 6, 1 June 2013 (2013-06-01), pages 1009-1016, XP055283108, US ISSN: 1547-6286, DOI: 10.4161/rna.24482 ----- | 1-13,15 | INV. C12N15/11 |
| A | YIN LINLIN ET AL: "Multiplex Conditional Mutagenesis Using Transgenic Expression of Cas9 and sgRNAs", GENETICS, GENETICS SOCIETY OF AMERICA, AUSTIN, TX, US, vol. 200, no. 2, 1 June 2015 (2015-06-01), pages 431-441, XP009189703, ISSN: 0016-6731 * page 437 * ----- | 1-13,15 | |
| A | SHEN ZHONGFU ET AL: "Conditional Knockouts Generated by Engineered CRISPR-Cas9 Endonuclease Reveal the Roles of Coronin inC. elegansNeural Development", DEVELOPMENTAL CELL, CELL PRESS, US, vol. 30, no. 5, 21 August 2014 (2014-08-21), pages 625-636, XP029052192, ISSN: 1534-5807, DOI: 10.1016/J.DEVCEL.2014.07.017 * figure 1 * ----- | 1-13,15 | TECHNICAL FIELDS SEARCHED (IPC) C12N |
| A | SHIN YOSHIOKA ET AL: "Development of a mono-promoter-driven CRISPR/Cas9 system in mammalian cells", SCIENTIFIC REPORTS, vol. 5, 16 December 2015 (2015-12-16), page 18341, XP055282633, DOI: 10.1038/srep18341 ----- | 1-13,15 | |

The present search report has been drawn up for all claims

1

| Place of search | Date of completion of the search | Examiner |
|---|---|---|
| The Hague | 4 July 2016 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 16 15 2731

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-13, 15

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 16 15 2731

The Search Division considers that the present European patent application does not comply with the requirements of unity of invention and relates to several inventions or groups of inventions, namely:

1. claims: 1-13, 15

   A method of producing a mutant host cell with altered expression of a gene of interest, using a temperature-inducible CRPSPR/Cas system comprising an inactive pre-guide RNA which comprises a guide RNA terminally extended by at least one self-processing hammerhead ribozyme having a thermosensitive hairpin; methods and reagents derived therefrom

   ---

2. claim: 14

   A thermosensitive hammerhead ribozyme which comprises a hammerhead region extended by a 3'-terminal sequence of at least 3 nucleotides, a complementary 5'-terminal sequence hybridizing to th 3'-terminal sequence, thereby forming a thermosensitive stem.

   ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015195621 A1
- WO 2015153940 A1
- US 20140068797 A


### Non-patent literature cited in the description


- **SARAGLIADIS et al.** *RNA Biology,* 2013, vol. 106, 1009-1016
- **JINEK M et al.** *Science,* 2012, vol. 337, 816-21
- **SYSTEM CC et al.** *Cell,* 2015, vol. 163, 1-13
- **RAN F ANN et al.** *Nature Protocols,* 2013, vol. 8 (11), 2281-2308
- **GAO Y et al.** *J. Integr. Plant Biol.,* 2014, vol. 56, 343-9
- **ARAKAWA H et al.** *BMC Biotechnol.,* 2001, vol. 1 (7
- **LONG ; UHLENBECK.** *Faseb J.,* 1993, vol. 7, 25-30
- **HAMMANN, C. et al.** *Rna,* 2012, vol. 18, 871-885
- **FERRE-D'AMARE.** *Nature,* 1998, vol. 395, 567-574
- **SAMBROOK et al.** Molecular Cloning: A Laboratory Manual. Cold Spring Harbor, 1989
- **MANIATIS ; FRITSCH ; SAMBROOK.** Molecular Cloning: A Laboratory Manual. Cold Spring Harbor, 1982
- **BLUMHOFF M et al.** *Appl. Microbiol. Biotechnol.,* 2013, vol. 97, 259-67
- **SAMBROOK, J. ; RUSSEL, D.** Molecular cloning: a laboratory manual. Coldd Spring Harbor Laboratory Press, 2001
- **PELECHOVÁ, J. et al.** *Folia Microbiol. (Praha),* 1990, vol. 35, 138-42
- **SARAGLIADIS.** *RNAbiology,* 2013, vol. 10, 1010-1016
- **GANTZ et al.** *Proc. Natl. Acad. Sci.,* 2015
- **LI, Y et al.** *Metab. Eng.,* 2015, vol. 31, 13-2
- **ARENTSHORST, M. ; RAM, A.F.J. ; MEYER, V.** Using non-homologous end-joining-deficient strains for functional gene analyses in filamentous fungi. *Methods Mol. Biol.,* 2012, vol. 835, 133-50
- **BLUMHOFF, M.L. ; STEIGER, M.G. ; MARX, H. ; MATTANOVICH, D. ; SAUER, M.** Six novel constitutive promoters for metabolic engineering of Aspergillus niger. *Appl. Microbiol. Biotechnol.,* 2013, vol. 97, 259-267
- **ENGLER, C. ; GRUETZNER, R. ; KANDZIA, R. ; MARILLONNET, S.** Golden gate shuffling: a one-pot DNA shuffling method based on type IIs restriction enzymes. *PLoS One,* 2009, vol. 4, e5553
- **GAO, Y. ; ZHAO, Y.** Self-processing of ribozyme-flanked RNAs into guide RNAs in vitro and in vivo for CRISPR-mediated genome editing. *J. Integr. Plant Biol.,* 2014, vol. 56, 343-349
- **NODVIG, C.S. ; NIELSEN, J.B. ; KOGLE, M.E. ; MORTENSEN, U.H.** A CRISPR-Cas9 System for Genetic Engineering of Filamentous Fungi. *PLoS One,* 2015, vol. 10, e0133085
- **SARAGLIADIS, A. ; KRAJEWSKI, S.S. ; REHM, C. ; NARBERHAUS, F. ; HARTIG, J.S. ; RNA BIOL.** *Thermozymes: Synthetic RNA thermometers based on ribozyme activity,* 2013, vol. 10, 1010-6
- **WERNER, S. ; ENGLER, C. ; WEBER, E. ; GRUETZNER, R. ; MARILLONNET, S.** Fast track assembly of multigene constructs using golden gate cloning and the MoClo system. *Bioeng. Bugs,* 2012, vol. 3, 38-43
- **ZETSCHE, B. ; GOOTENBERG, J.S. ; ABUDAYYEH, O.O. ; SLAYMAKER, I.M. ; MAKAROVA, K.S. ; ESSLETZBICHLER, P. ; VOLZ, S.E. ; JOUNG, J. ; VAN DER OOST, J. ; REGEV, A.** Cpf1 Is a Single RNA-Guided Endonuclease of a Class 2 CRISPR-Cas System. *Cell,* 2015, vol. 163, 759-771